# EUROPÄISCHE PATENTSCHRIFT

(11) **EP 1 799 690 B1**
(45) Veröffentlichungstag und Bekanntmachung des Hinweises auf die Patenterteilung: **16.04.2008**
(21) Anmeldenummer: 05792037.3
(22) Anmeldetag: 05.10.2005
(51) Int. Cl.: C07D 513/04, C07D 417/06, C07D 277/82, A61K 31/429, A61P 35/00

(54) **THIAZOLYL-DIHYDRO-INDAZOLE**
THIAZOLYL-DIHYDRO INDAZOLES
THIAZOLYL-DIHYDRO-INDAZOLES

(30) Priorität: 07.10.2004 DE 102004048877; 09.02.2005 DE 102005005813; 05.08.2005 EP 05107230
(43) Veröffentlichungstag der Anmeldung: 27.06.2007
(73) Patentinhaber: Boehringer Ingelheim International GmbH, 55216 Ingelheim am Rhein (DE); Boehringer Ingelheim Pharma GmbH & Co. KG, 55216 Ingelheim am Rhein (DE)
(72) Erfinder: BETZEMEIER, Bodo, A-1140 Wien (AT); BRANDL, Trixi, CH 4056 Basel (CH); BREITFELDER, Steffen, 88433 Assmannshardt (DE); BRUECKNER, Ralph, A-1130 Wien (AT); GERSTBERGER, Thomas, A-1230 Wien (AT); GMACHL, Michael, A-1140 Wien (AT); GRAUERT, Matthias, 88400 Biberach (DE); HILBERG, Frank, A-1050 Wien (AT); HOENKE, Christoph, 55218 Ingelheim (DE); HOFFMANN, Matthias, 88441 Mittelbiberach (DE); IMPAGNATIELLO, Maria, A-1030 Wien (AT); KESSLER, Dirk, A-1120 Wien (AT); KLEIN, Christian, A-1070 Wien (AT); KRIST, Bernd, 86450 Altenmuenster (DE); MAIER, Udo, 89250 Senden (DE); MCCONNELL, Darryl, A-1200 Wien (AT); REITHER, Charlotte, A-1230 Wien (AT); SCHEUERER,Stefan, 88447 Warthausen (DE); SCHOOP, Andreas, A-1150 Wien (AT); SCHWEIFER, Norbert, A-1210 Wien (AT); SIMON, Oliver, A-1120 Wien (AT); STEEGMAIER, Martin, 72762 Reutlingen (DE); STEURER, Steffen, A-1190 Wien (AT); WAIZENEGGER, Irene, A-1030 Wien (AT); WEYER-CZERNILOFSKY, Ulrike, A-2500 Baden (AT); ZOEPHEL, Andreas, A-1130 Wien (AT)
(74) Vertreter: Hammann, Heinz
(86) Internationale Anmeldenummer: PCT/EP2005/055021
(87) Internationale Veröffentlichungsnummer: WO 2006/040281

(56) Entgegenhaltungen:
- WO-A-03/035618
- US-A1- 2002 151 544
- DATABASE BEILSTEIN BEILSTEIN INSTITUTE FOR ORGANIC CHEMISTRY, FRANKFURT-MAIN, DE; XP002359147 Database accession no. BRN: 791545 & GAZZETTA CHIMICA ITALIANA, Bd. 103, 1973, Seiten 755-770,

## Beschreibung

Die vorliegende Erfindung betrifft neue Thiazolyl-dihydro-indazole der allgemeinen Formel (1) 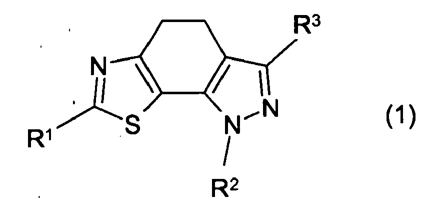 wobei die Reste R¹ bis R³ die in den Ansprüchen und der Beschreibung genannten Bedeutungen haben, deren Isomere, Verfahren zur Herstellung dieser Thiazolyl-dihydro-indazole sowie deren Verwendung als Arzneimittel.

### Hintergrund der Erfindung

Die Phosphorylierung von Proteinen und Lipiden ist ein wichtiger zellulärer Regulationsmechanismus, der bei multiplen biologischen Vorgängen, wie z.B. Zellproliferation, Differenzierung, Apoptose, Metabolismus, Inflammation, Immunreaktionen und Angiogenese eine Rolle spielt. Im humanen Genom sind über 500 Kinasen kodiert. Tyrosin-Protein-Kinasen werden im allgemeinen durch Wachstumsfaktoren oder andere mitogene Signale stimuliert und phosphorylieren Proteine, die schnelle Signalübertragungen initiieren. Serin/Threonin-Protein-Kinasen phosphorylieren meist Proteine, die intrazelluläre Signale vernetzen und amplifizieren. Lipid-Kinasen sind ebenfalls wichtige Schaltstellen intrazellulärer Signalwege, die diverse biologische Vorgänge verknüpfen.

Eine Reihe von Protein-Kinasen haben sich bereits als geeignete Zielmoleküle für die therapeutische Intervention in verschiedenen Indikationen, z.B. Krebs, entzündlichen und Autoimmunerkrankungen erwiesen. Da ein hoher Prozentsatz der bisher identifizierten, an der Krebsentstehung beteiligten Gene für Kinasen kodiert, stellen diese Enzyme speziell für die Krebstherapie attraktive Zielmoleküle dar.

Phosphatidylinositol-3-Kinasen (PI3-Kinasen) sind ein Subfamilie der Lipid-Kinasen, die die Übertragung eines Phosphatrestes auf die 3'-Position des Inositolrings von Phosphoinositiden katalysieren. Sie spielen eine wichtige Rolle bei zahlreichen zellulären Vorgängen wie z.B. Zellwachstums- und Differenzierungsvorgängen, der Steuerung von cytoskeletalen Veränderungen und der Regulation intrazellulärer Transportvorgänge. Aufgrund ihrer *in vitro-*Spezifität für bestimmte Phosphoinositid-Substrate können die PI3-Kinasen in verschiedene Klassen eingeteilt werden.

Von den Mitgliedern der Klasse I PI3-Kinasen werden die PI3-Kinase α, β und δ (Klasse IA) hauptsächlich durch Rezeptor-Tyrosin-Kinasen (RTKs) oder lösliche Tyrosinkinasen aktiviert. Die PI3-Kinase γ (Klasse IB) hingegen wird hauptsächlich durch Gβγ-Untereinheiten aktiviert, die aus heterotrimeren G-Proteinen nach Aktivierung von heptahelikalen Rezeptoren freigesetzt werden. Durch diese unterschiedliche Kopplung an Zelloberflächen-Rezeptoren in Kombination mit einer mehr oder weniger restriktiven Expression ergeben sich zwangsläufig sehr unterschiedliche Aufgaben und Funktionen der 4 Klasse I PI3-Kinasen im intakten Organismus.

Viele unabhängige Befunde sprechen für eine Beteiligung von Klasse IA PI3-Kinasen an unkontrollierten Zellwachstums- und Differenzierungsvorgängen. So war die erste nachgewiesene PI3-Kinase Aktivität mit der transformierenden Aktivität von viralen Onkogenen assoziiert, wie z.B. dem middle T Antigen von Polyomaviren, Src Tyrosinkinasen oder aktivierten Wachstumsfaktoren (Workman, Biochem Soc Trans. 2004; 32(Pt 2):393-6). Bei vielen Tumoren, wie z.B. Brustkrebs, Ovar- oder auch Pankreaskarzinom findet man eine Überaktivität von Akt/PKB, welches direkt durch die Lipidprodukte der Klasse I PI3-Kinasen aktiviert wird und so die Signale in die Zelle weiterleitet. Darüber hinaus wurde erst kürzlich gefunden, dass das PIK3CA-Gen, das für die p110-Untereinheit von PI3Kα codiert, in verschiedenen Tumorarten, wie z.B. Kolon-, Mamma- oder Lungenkarzinomen, eine hohe Mutationshäufigkeit aufweist, von denen einige repräsentativ als aktivierende Mutationen charakterisiert werden konnten (Samuels et al., Science 2004; 304(5670):554).

Inhibitoren von PI3-Kinasen sind bereits in WO 0 303 618 und US 2002/151544 beschrieben worden.

### Detaillierte Beschreibung der Erfindung

Es wurde nun überraschenderweise gefunden, dass Verbindungen der allgemeinen Formel (1), worin die Reste R¹ bis R³ die nachstehend genannten Bedeutungen haben, als Inhibitoren spezifischer Zellzykluskinasen wirken. Somit können die erfindungsgemäßen Verbindungen beispielsweise zur Behandlung von Erkrankungen, die mit der Aktivität spezifischer Zellzykluskinasen in Zusammenhang stehen und durch exzessive oder anomale Zellproliferation charakterisiert sind, verwendet werden.

Die vorliegende Erfindung betrifft Verbindungen der allgemeinen Formel (1) 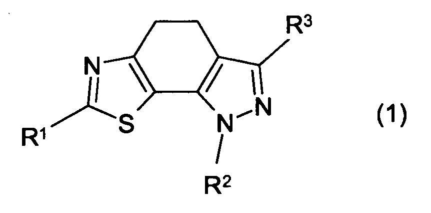 worin
R¹ ausgewählt aus der Gruppe bestehend aus -NHR^{c}, -NHC(O)R^{c}, -NHC(O)OR^{c}, -NHC(O)NR^{c}R^{c} und -NHC(O)SR^{c};
R² ein Rest, gegebenenfalls substituiert mit einem oder mehreren R⁴, ausgewählt aus der Gruppe bestehend aus C₁₋₆Alkyl, C₃₋₈Cycloalkyl, 3-8 gliedriges Heterocycloalkyl, C₆₋₁₀Aryl und 5-10 gliedriges Heteroaryl;
R³ ein gegebenenfalls mit einem oder mehreren R^{e} und/oder R^{f} substituiert Rest ausgewählt aus der Gruppe bestehend aus C₆₋₁₀Aryl und 5-10 gliedriges Heteroaryl;
R⁴ ein Rest ausgewählt aus der Gruppe bestehend aus R^{a}, R^{b} und R^{a} substituiert mit einem oder mehreren, gleich oder verschiedenen R^{c} und/oder R^{b};
jedes R^{a} unabhängig voneinander ausgewählt aus der Gruppe bestehend aus C₁₋₆Alkyl, C₃₋₈Cycloalkyl, C₄₋₁₁Cycloalkylalkyl, C₆₋₁₀Aryl, C₇₋₁₆Arylalkyl, 2-6 gliedriges Heteroalkyl, 3-8 gliedriges Heterocycloalkyl, 4-14 gliedriges Heterocycloalkylalkyl, 5-10 gliedriges Heteroaryl und 6-16 gliedriges Heteroarylalkyl;
jedes R^{b} ein geeigneter Rest und jeweils unabhängig voneinander ausgewählt aus der Gruppe bestehend aus =O, -OR^{c}, C₁₋₃Haloalkyloxy, -OCF₃, =S, -SR^{c}, =NR^{c}, =NOR^{c}, -NR^{c}R^{c}, Halogen, -CF3, -CN, -NC, -OCN, -SCN, -NO, -NO₂, =N₂, -N₃, -S(O)R^{c}, -S(O)₂R^{c}, -S(O)₂OR^{c}, -S(O)NR^{c}R^{c}, -S(O)₂NR^{c}R^{c}, -OS(O)R^{c}, -OS(O)₂R^{c}, -OS(O)₂OR^{c}, -OS(O)₂NR^{c}R^{c}, -C(O)R^{c}, -C(O)OR^{c}, -C(O)NR^{c}R^{c}, -C(O)N(R^{g})NR^{c}R^{c}, -C(O)N(R^{g})OR^{c}, -CN(R^{g})NR^{c}R^{c}, -OC(O)R^{c}, -OC(O)OR^{c}, -OC(O)NR^{c}R^{c}, -OCN(R^{g})NR^{c}R^{c}, -N(R^{g})C(O)R^{c}, -N(R^{g})C(O)R^{c}, -N(R^{g})C(S)R^{c}, -N(R^{g})S(O)₂R^{c}, -N(R^{g})S(O)₂NR^{c}R^{c}, -N[S(O)₂]₂R^{c},-N(R^{g})C(O)OR^{c}, -N(R^{g})C(O)NR^{c}R^{c}, und -N(R^{g})CN(R^{g})NR^{c}R^{c};
jedes R^{c} unabhängig voneinander Wasserstoff oder ein gegebenenfalls mit einem oder mehreren, gleich oder verschiedenen R^{d} und/oder R^{e} substituierter Rest ausgewählt aus der Gruppe bestehend aus C₁₋₆Alkyl, C₃₋₈Cycloalkyl, C₄₋₁₁Cycloalkylalkyl, C₆₋₁₀Aryl, C₇₋₁₆Arylalkyl, 2-6 gliedriges Heteroalkyl, 3-8 gliedriges Heterocycloalkyl, 4-14 gliedriges Heterocycloalkylalkyl, 5-10 gliedriges Heteroaryl und 6-16 gliedriges Heteroarylalkyl,
jedes R^{d} unabhängig voneinander Wasserstoff oder ein gegebenenfalls mit einem oder mehreren, gleich oder verschiedenen R^{e} und/oder R^{f} substituierter Rest ausgewählt aus der Gruppe bestehend aus C₁₋₆Alkyl, C₃₋₈Cycloalkyl, C₄₋₁₁Cycloalkylalkyl, C₆₋₁₀Aryl, C₇₋₁₆Arylalkyl, 2-6 gliedriges Heteroalkyl, 3-8 gliedriges Heterocycloalkyl, 4-14 gliedriges Heterocycloalkylalkyl, 5-10 gliedriges Heteroaryl und 6-16 gliedriges Heteroarylalkyl,
jedes R^{e} ein geeigneter Rest und jeweils unabhängig voneinander ausgewählt aus der Gruppe bestehend aus =O, -OR^{f}, C₁₋₃Haloalkyloxy, -OCF₃, =S, -SR^{f}, -NR^{f}, =NOR^{f}, -NR^{f}R^{f}, Halogen, -CF3, -CN, -NC, -OCN, -SCN, -NO, -NO₂, =N₂, -N₃, -S(O)R^{f}, -S(O)₂R^{f}, -S(O)₂OR^{f}, -S(O)NR^{f}R^{f}, -S(O)₂NR^{f}R^{f}, -OS(O)R^{f}, -OS(O)₂R^{f}, -OS(O)₂OR^{f}, -OS(O)₂NR^{f}R^{f}, -C(O)R^{f}, -C(O)OR^{f}, -C(O)NR^{f}R^{f}, -CN(R^{g})NR^{f}R^{f}, -OC(O)R^{f}, -OC(O)OR^{f}, -OC(O)NR^{f}R^{f}, -OCN(R^{g})NR^{f}R^{f}, -N(R^{g})C(O)R^{f}, -N(R^{g})C(S)R^{f}, -N(R^{g})S(O)₂R^{f}, -N(R^{g})C(O)OR^{f}, -N(R^{g})C(O)NR^{f}R^{f}, und -N(R^{g})CN(R^{g})NR^{f}R^{f};
jedes R^{f} unabhängig voneinander Wasserstoff oder ein gegebenenfalls mit einem oder mehreren, gleich oder verschiedenen R^{g} substituierter Rest ausgewählt aus der Gruppe bestehend aus C₁₋₆Alkyl, C₃₋₈Cycloalkyl, C₄₋₁₁Cycloalkylalkyl, C₆₋₁₀Aryl, C₇₋₁₆Arylalkyl, 2-6 gliedriges Heteroalkyl, 3-8 gliedriges Heterocycloalkyl, 4-14 gliedriges Heterocycloalkylalkyl, 5-10 gliedriges Heteroaryl und 6-16 gliedriges Heteroarylalkyl
jedes R^{g} unabhängig voneinander Wasserstoff, C₁₋₆Alkyl, C₃₋₈Cycloalkyl, C₄₋₁₁Cycloalkylalkyl, C₆₋₁₀Aryl, C₇₋₁₆Arylalkyl, 2-6 gliedriges Heteroalkyl, 3-8 gliedriges Heterocycloalkyl, 4-14 gliedriges Heterocycloalkylalkyl, 5-10 gliedriges Heteroaryl und 6-16 gliedriges Heteroarylalkyl
bedeuten, gegebenenfalls in Form ihrer Tautomeren, ihrer Racemate, ihrer Enantiomere, ihrer Diastereomere und ihrer Gemische, sowie gegebenenfalls ihrer pharmakologisch unbedenklichen Säureadditionssalze.

Ein Aspekt der Erfindung sind Verbindungen der allgemeinen Formel (1), wobei R³ ein Rest ausgewählt aus der Gruppe bestehend aus Phenyl, Furyl, Pyridyl, Pyrimidinyl und Pyrazinyl, gegebenenfalls substituiert mit einem oder mehreren R⁴, bedeutet.

Ein weiterer Aspekt der Erfindung sind Verbindungen der allgemeinen Formel (1), wobei R³ Pyridyl bedeutet.

Ein weiterer Aspekt der Erfindung sind Verbindungen der allgemeinen Formel (1), wobei R¹ -NHC(O)R^{c} bedeutet.

Ein weiterer Aspekt der Erfindung sind Verbindungen der allgemeinen Formel (1), wobei R¹ -NHC(O)CH₃ bedeutet.

Ein Aspekt der Erfindung sind Verbindungen der Formel (A) 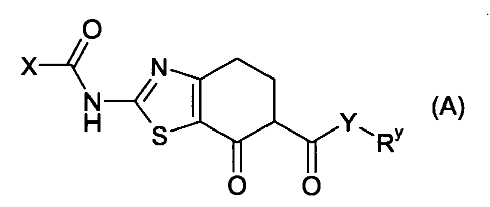 wobei
X -CH₃, -OR⁴ oder -SR⁴ und
Y Phenyl, 5-10 gliedriges Heteroaryl oder die Gruppe -C(O)O und
R^{y} Wasserstoff ,-NO₂ oder C₁₋₆Alkyl bedeuten und R⁴ wie vorstehend definiert ist.

Ein weiterer Aspekt der Erfindung sind Verbindungen der allgemeinen Formel (A), wobei R⁴ gleich - C₁₋₆Alkyl bedeutet.

Ein weiterer Aspekt der Erfindung ist die Verwendung von Verbindungen der Formel (A) als Syntheseintermediate.

Ein Aspekt der Erfindung sind Verbindungen der allgemeinen Formel (1), oder deren pharmazeutisch wirksamen Salze, als Arzneimittel.

Ein Aspekt der Erfindung ist die Verwendung von Verbindungen der allgemeinen Formel (1), oder deren pharmazeutisch wirksamen Salze, zur Herstellung eines Arzneimittels mit antiproliferativer Wirkung.

Ein Aspekt der Erfindung ist eine pharmazeutische Zubereitungen, enthaltend als Wirkstoff eine oder mehrere Verbindungen der allgemeinen Formel (1), oder deren physiologisch verträglichen Salze, gegebenenfalls in Kombination mit üblichen Hilfs- und/oder Trägerstoffen.

Ein weiterer Aspekt der Erfindung ist die Verwendung von Verbindungen der allgemeinen Formel (1) zur Herstellung eines Arzneimittels zur Behandlung und/oder Prävention von Krebs.

Ein Aspekt der Erfindung ist eine pharmazeutische Präparation umfassend eine Verbindung der allgemeinen Formel (1) und mindestens eine weitere zytostatische oder zytotoxische, von Formel (1) verschiedene Wirksubstanz, gegebenenfalls in Form ihrer Tautomeren, ihrer Racemate, ihrer Enantiomere, ihrer Diastereomere und ihrer Gemische, sowie gegebenenfalls ihrer pharmakologisch unbedenklichen Säureadditionssalze.

### DEFINITIONEN

Wie hierin verwendet treffen folgenden Definitionen zu, falls nicht anders beschrieben.

Unter Alkyl-Substitutenten sind jeweils gesättigte, ungesättigte, geradkettige oder verzweigte aliphatische Kohlenwasserstoffreste (Alkylrest) zu verstehen und umfasst sowohl gesättigte Alkylreste als auch ungesättigte Alkenyl- und Alkinylreste. Alkenyl-Substituenten sind jeweils geradkettige oder verzweigte, ungesättigte Alkylreste, die mindestens eine Doppelbindung aufweisen. Unter Alkinyl-Substituenten sind jeweils geradkettige oder verzweigte, ungesättigte Alkylreste, die mindestens eine Dreifachbindung aufweisen, zu verstehen.

Heteroalkyl repräsentiert geradkettige oder verzweigte aliphatische Kohlenwasserstoffketten, die durch 1 bis 3 Heteroatome unterbrochen sind, wobei jedes der verfügbaren Kohlenstoff- und Stickstoffatome in der Heteroalkylkette gegebenenfalls jeweils unabhängig voneinander substituiert sein kann und die Heteroatome jeweils unabhängig voneinander ausgewählt werden aus der Gruppe bestehend aus O, N und S (z. B. Dimethylaminomethyl, Dimethylaminoethyl, Dimethylaminopropyl, Diethylaminomethyl, Diethylaminoethyl, Diethylaminopropyl, 2-Disopropylaminoethyl, Bis-2-methoxyethylamino, [2-(Dimethylamino-ethyl)-ethyl-amino]-methyl, 3-[2-(Dimethylamino-ethyl)-ethyl-amino]-propyl, Hydroxymethyl, 2-Hydroxyethyl, 3-Hydroxypropyl, Methoxy, Ethoxy, Propoxy, Methoxymethyl, 2-Methoxyethyl).

Halogenalkyl bezieht sich auf Alkylreste, in denen ein oder mehrere Wasserstoffatome durch Halogenatome ersetzt sind. Halogenalkyl umfasst sowohl gesättigte Alkylreste als auch ungesättigte Alkenyl- und Alkinylreste, wie beispielsweise -CF₃, -CHF₂, -CH₂F, -CF₂CF₃,-CHFCF₃, -CH₂CF₃, -CF₂CH₃, -CHFCH₃, -CF₂CF₂CF₃, -CF₂CH₂CH₃, -CF=CF₂, -CCl=CH₂, -CBr=CH₂, -CJ=CH₂, -C≡C-CF₃, -CHFCH₂CH₃ und -CHFCH₂CF₃.

Halogen bezieht sich auf Fluor-, Chlor-, Brom- und/oder Jodatome.

Unter Cycloalkyl ist ein mono- oder bizyklischer Ring zu verstehen, wobei das Ringsystem ein gesättigter Ring aber auch ein ungesättigter, nichtaromatischer Ring sein kann, welcher gegebenenfalls auch Doppelbindungen enthalten kann, wie zum Beispiel Cyclopropyl, Cyclopropenyl, Cyclobutyl, Cyclobutenyl, Cyclopentyl, Cyclopentenyl, Cyclohexyl, Cyclohexenyl, Norbornyl und Norbornenyl.

Cycloalkylalkyl umfasst eine nicht-zyklische Alkylgruppe, in der ein an einem Kohlenstoffatom gebundenes Wasserstoffatom, üblicherweise an einem terminalen C-Atom, durch eine Cycloalkylgruppe ersetzt ist.

Aryl bezieht sich auf monozyklische oder bizyklische Ringe mit 6 - 12 Kohlenstoffatomen wie beispielsweise Phenyl und Naphthyl.

Arylalkyl umfasst eine nicht-zyklische Alkylgruppe, in der ein an einem Kohlenstoffatom gebundenes Wasserstoffatom, üblicherweise an einem terminalen C-Atom, durch eine Arylgruppe ersetzt ist.

Unter Heteroaryl sind mono- oder bizyklische Ringe zu verstehen, welche anstelle eines oder mehrere Kohlenstoffatome ein oder mehrere, gleich oder verschiedene Heteroatome enthalten, wie z.B. Stickstoff, Schwefel- oder Sauerstoffatome. Beispielsweise genannt seien Furyl, Thienyl, Pyrrolyl, Oxazolyl, Thiazolyl, Isoxazolyl, Isothiazolyl, Pyrazolyl, Imidazolyl, Triazolyl, Tetrazolyl, Oxadiazolyl, Thiadiazolyl, Pyridyl, Pyrimidyl, Pyridazinyl, Pyrazinyl und Triazinyl. Beispiele für bizyklische Heteroarylreste sind Indolyl, Isoindolyl, BenzoFuryl, Benzothienyl, Benzoxazolyl, Benzothiazolyl, Benzisoxazolyl, Benzisothiazolyl, Benzimidazolyl, Indazolyl, Isoquinolinyl, Quinolinyl, Quinoxalinyl, Cinnolinyl, Phthalazinyl, Quinazolinyl und Benzotriazinyl, Indolizinyl, OxazoloPyridyl; ImidazoPyridyl, Naphthyridinyl, Indolinyl, Isochromanyl, Chromanyl, Tetrahydroisochinolinyl, Isoindolinyl, IsobenzotetrahydroFuryl, Isobenzotetrahydrothienyl, Isobenzothienyl, Benzoxazolyl, PyridoPyridyl, BenzotetrahydroFuryl, Benzotetrahydrothienyl, Purinyl, Benzodioxolyl, Triazinyl, Phenoxazinyl, Phenothiazinyl, Pteridinyl, Benzothiazolyl, ImidazoPyridyl, Imidazothiazolyl, Dihydrobenzisoxazinyl, Benzisoxazinyl, Benzoxazinyl, Dihydrobenzisothiazinyl, Benzopyranyl, Benzothiopyranyl, Coumarinyl, Isocoumarinyl, Chromonyl, Chromanonyl, Pyridyl-*N*-oxid Tetrahydroquinolinyl, Dihydroquinolinyl, Dihydroquinolinonyl, Dihydroisoquinolinonyl, Dihydrocoumarinyl, Dihydroisocoumarinyl, Isoindolinonyl, Benzodioxanyl, Benzoxazolinonyl, Pyrrolyl-*N-*oxid, Pyrimidinyl-*N*-oxid, Pyridazinyl-*N*-oxid, Pyrazinyl-*N*-oxid, Quinolinyl-*N*-oxid, Indolyl-*N*-oxid, Indolinyl-*N*-oxid, Isoquinolyl-*N*-oxid, Quinazolinyl-*N*-oxid, Quinoxalinyl-*N*-oxid, Phthalazinyl-*N*-oxid, Imidazolyl-*N*-oxid, Isoxazolyl-*N*-oxid, Oxazolyl-*N*-oxid, Thiazolyl-*N*-oxid, Indolizinyl-*N*-oxid, Indazolyl-*N*-oxid, Benzothiazolyl-*N*-oxid, Benzimidazolyl-*N*-oxid, Pyrrolyl-*N*-oxid, Oxadiazolyl-*N*-oxid, Thiadiazolyl-*N*-oxid, Triazolyl-*N*-oxid, Tetrazolyl-*N*-oxid, Benzothiopyranyl-*S*-oxid und Benzothiopyranyl-*S,S-*dioxid.

Heteroarylalkyl umfasst eine nicht-zyklische Alkylgruppe, in der ein an einem Kohlenstoffatom gebundenes Wasserstoffatom, üblicherweise an einem terminalen C-Atom, durch eine Heteroarylgruppe ersetzt ist.

Heterocycloalkyl bezieht sich auf 3 - 12 Kohlenstoffatome umfassende gesättigte oder ungesättigte, nicht aromatische mono-, bizyklische oder überbrückte bizyklische Ringe, welche anstelle eines oder mehrere Kohlenstoffatome Heteroatome, wie Stickstoff, Sauerstoff oder Schwefel, tragen. Beispiele für solche Heterocyloalkylreste sind TetrahydroFuryl, Pyrrolidinyl, Pyrrolinyl, Imidazolidinyl, Imidazolinyl, Pyrazolidinyl, Pyrazolinyl, Piperidinyl, Piperazinyl, Indolinyl, Isoindolinyl, Morpholinyl, Thiomorpholinyl, Homomorpholinyl, Homopiperidinyl, Homopiperazinyl, Homothiomorpholinyl, Thiomorpholinyl-*S*-oxid, Thiomorpholinyl-*S,S*-dioxid, Tetrahydropyranyl, Tetrahydrothienyl, Homothiomorpholinyl-*S,S*-Dioxid, Oxazolidinonyl, Dihydropyrazolyl, Dihydropyrrolyl, Dihydropyrazinyl, DihydroPyridyl, Dihydropyrimidinyl, Dihydrofuryl, Dihydropyranyl, Tetrahydrothienyl-S-oxid, Tetrahydrothienyl-S,S-dioxid, Homothiomorpholinyl-S-oxid, 2-Oxa-5-azabicyclo[2.2.1]heptan, 8-Oxa-3-aza-bicyclo[3.2.1]octan, 3,8-Diaza-bicyclo[3.2.1]octan, 2,5-Diaza-bicyclo[2.2.1]heptan, 3,8-Diaza-bicyclo[3.2.1]octan, 3,9-Diazabicyclo[4.2.1]nonan und 2,6-Diaza-bicyclo[3.2.2]nonan.

Heterocycloalkylalkyl bezieht sich auf eine nicht-zyklische Alkylgruppe, in der ein an einem Kohlenstoffatom gebundenes Wasserstoffatom, üblicherweise an einem terminalen C-Atom, durch eine Heterocycloalkylgruppe ersetzt ist,

Die nachfolgenden Beispiele illustrieren die vorliegende Erfindung ohne sie jedoch in ihrem Umfang einzuschränken.

### Synthese der Reagenzien

### R-1) cis-1-Methylamino-4-(pyrrolidin-1-yl)-cyclohexan

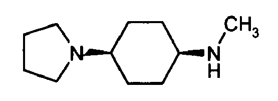

### R-1a) cis-4-(Pyrrolidin-1-yl)-cyclohexancarbamidsäure-tert-butylester

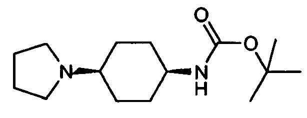

Eine Lösung von *cis*-4-Aminocyclohexancarbamidsäure-*tert*-butylester (10 g, 46 mmol) und 1,4-Dibrombutan (12.1 g, 56 mmol) in 400 mL DMF wird mit 25 g Kaliumhydrogencarbonat versetzt und 24 h bei RT gerührt. Anschließend wird die Reaktionsmischung eingeengt, in Diethylether aufgenommen und mit Wasser gewaschen. Die organische Phase wird getrocknet und im Vakuum eingeengt. Ausbeute: 12 g R-1b) *N*-Methyl-*cis*-4-(Pyrrolidin-1-yl)-cyclohexancarbamidsäure-*tert*-butylester

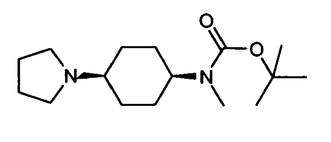

R-1a (5 g, 18 mmol) wird in 20 mL *N,N*-Dimethylacetamid gelöst und zu einer Suspension von Natriumhydrid (60 % in Paraffinöl, 0.8 g, 20 mmol) in 20 mL *N,N*-Dimethylacetamid bei 37 °C gegeben, so dass die Temperatur 48 °C nicht übersteigt. Nach Beenden der Schaumbildung wird Methyljodid (2.9 g, 20 mmol) zugegeben und 10 min bei RT gerührt. Die Reaktionsmischung wird mit Ethylacetat versetzt und mit Wasser gewaschen. Anschließend wird die organische Phase mit Oxalsäure versetzt und mit Ethylacetat gewaschen. Danach wird mit Kaliumhydrogencarbonat basisch gestellt und mit Ethylacetat extrahiert. Die organischen Phasen werden eingeengt und ohne weitere Reinigung umgesetzt. Ausbeute: 1.4 g
R-1b (1.4 g, 5 mmol) wird in 50 mL Dichlormethan gelöst, mit 25 mL Trifluoressigsäure versetzt und 4 h bei RT gerührt. Nach Einengen der Reaktionsmischung wird das gewünschte Produkt mit Salzsäure (1 N in Diethylether) als Dihydrochlorid gefällt. Ausbeute: 1 g

### R-2) trans-1-Amino-4-(8-oxa-3-aza-bicyclo[3.2.1]oct-3-yl)-cyclohexan

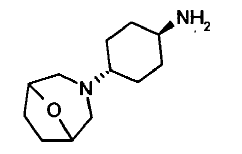

Zu einer Lösung von 2,5-Bis(p-tosyloxymethyl)tetrahydrofuran (44 g, 0.1 mol) in 440 mL Toluol wird nacheinander Triethylamin (21 g, 0.21 mol), *trans*-4-Aminocyclohexyl-carbamidsäurebenzylester (24.8 g, 0.1 mol) sowie eine katalytische Menge DMAP gegeben Die Reaktionsmischung wird 6 d unter Rückfluss erhitzt. Nach Abkühlen wird die organische Phase vom unlöslichen Harz dekantiert und der Rückstand chromatographisch gereinigt. Das so erhaltene Zwischenprodukt wird im Autoklav in 300 mL Methanol suspendiert und mit 37 mL Salzsäure (6 N in Isopropanol) sowie mit 6 g Palladium auf Aktivkohle versetzt. Die Reaktionsmischung wird 15 h bei RT unter Wasserstoffatmosphäre (50 bar) gerührt. Nach Filtration über Celite® wird das Filtrat eingeengt, in heißem Ethylacetat aufgenommen und mit 37 mL Salzsäure (6 N in Isopropanol) versetzt. Beim Abkühlen fällt das gewünschte Produkt als Hydrochloridsalz aus, welches filtriert und getrocknet wird.

### R-3) 1-Amino-4-(methylpropylamino)cyclohexan

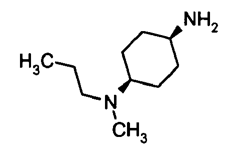

### R-3a) cis-4-(2,2,2-Trifluoracetylamino)-cyclohexancarbamidsäure-tert-butylester

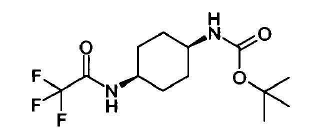

Eine Lösung von *cis*-4-Aminocyclohexancarbamidsäure-*tert*-butylester (22.1 g, 103 mmol) und Methyltrifluoracetat (11 mL, 110 mmol) in 110 mL Methanol werden 4 h bei RT gerührt. Nach Abkühlung der Reaktionsmischung auf 0 °C wird der Niederschlag filtriert, mit Diethylether gewaschen und getrocknet. Ausbeute: 17.6 g.

### R-3b) cis-4-Methyl-(2,2,2-trifluoracetyl)amino-cyclohexancarbamidsäure-tert-butylester

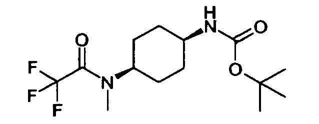

Eine Suspension von R-3a (8.3 g, 27 mmol) in 100 mL *N,N*-Dimethylacetamid wird bei RT unter Stickstoffatmosphäre mit Natriumhydrid (60% in Paraffinöl, 1.3 g, 32 mmol) versetzt. Nach 20 min wird Methyljodid (4.5 g, 32 mmol) zugefügt und 15 h bei RT gerührt. Nach Hydrolyse mit 800 mL Eiswasser wird der Niederschlag filtriert und mit Wasser sowie Petrolether gewaschen. Der Rückstand wird aus 200 mL Diisopropylether versetzt mit 10 mL Acetonitril umkristallisiert. Ausbeute: 11 g

### R-3c) cis-4-Methylamino-cyclohexancarbamidsäure-tert-butylester

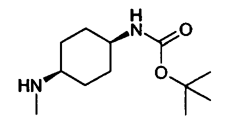

Zur Abspaltung der Trifluoracetatgruppe wird R-3b (39.7 g, 123 mmol) in 536 mL Methanol mit 117 mL Natronlauge (2 N) versetzt und 5 h bei RT gerührt. Die Reaktionsmischung wird eingeengt, mit Wasser und Ethylacetat ausgeschüttelt. Die organische Phase wird getrocknet, filtriert und im Vakuum eingeengt. Ausbeute: 28.4 g

### R-3d) cis-4-(Methylpropylamino)-cyclohexancarbamidsäure-tert-butylester

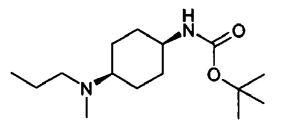

Zu einer Lösung von R-3c (2 g, 8.8 mmol) in 5 mL Acetonitril wird Triethylamin (0.98 g, 9.7 mmol) und *n*-Propylbromid (1.2 g, 9.7 mmol) gegeben und 3 h im Druckrohr bei 60 °C gerührt. Die Reaktionsmischung wird anschließend eingeengt und mit Wasser und Ethylacetat ausgeschüttelt. Ausbeute: 1 g
Die Abspaltung der BOC-Schutzgruppe erfolgt analog der Darstellung von R-1 unter Verwendung von R-3d (1 g, 3.7 mmol), 20 mL Trifluoressigsäure und 20 mL Dichlormethan. Ausbeute: 0.6 g

### R-4) Cyclopropyl-piperidin-4-yl-amin

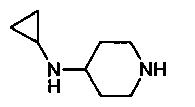

Eine Lösung von 1-*tert*-Butoxycarbonyl-piperidin-4-on (1 g, 5 mmol) und Cyclopropylamin (352 µL) in 15 mL 1,2-Dichlorethan wird 20 min bei RT gerührt und anschließend mit Natriumtrisacetoxyborhydrid (1.6 g, 7 mmol) und 0.3 mL Essigsäure versetzt. Nach 15 h Rühren bei RT wird die Reaktionsmischung mit gesättigter Natriumhydrogencarbonat-Lösung hydrolysiert und mit 2 x 50 mL Dichlormethan extrahiert. Die vereinigten organischen Phasen werden mit gesättigter Natriumchloridlösung gewaschen, getrocknet, filtriert und eingeengt. Der Rückstand wird in 4 mL Diethylether aufgenommen und mit 8 mL Salzsäure (4 N in Dioxan) versetzt. Nach 15 h Rühren bei RT wird der Niederschlag filtriert und mit Diethylether gewaschen. Man erhält das gewünschte Produkt als Hydrochlorid. Ausbeute: 0.96 g

### R-5) 1-Cyclopentyl-piperidin-4-carbonsäure

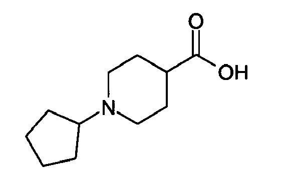

Zu einer Lösung von Piperidin-4-carbonsäureethylester (22.9 g, 145 mmol) und Cyclopentanon (13.5 g, 160 mmol) in 400 mL THF werden katalytische Mengen *p-*Toluolsulfonsäure (750 mg) und 12.5 mL Eisessig gegeben. Nach 30 min Rühren bei RT wird Natriumtriacetoxyborhydrid (42 g, 190 mmol) portionsweise zugefügt. Die Reaktionsmischung wird 15 h bei RT gerührt und nach Einengen mit Natriumcarbonatlösung und Dichlormethan ausgeschüttelt. Die organische Phase wird getrocknet, filtriert und eingeengt. Ausbeute: 32 g 1-Cyclopentylpiperidin-4-carbonsäureethylester. Anschließend wird die Zwischenverbindung (1 g, 4.4 mmol) in 10 mL EtOH mit 10 mL NaOH (5 N) 15 h bei 80 °C verseift. Nach Abkühlen wird die Reaktionsmischung angesäuert und der entstandene Niederschlag filtriert.

### R-6) cis-4-(Pyrrolidin-1-yl)cyclohexancarbonsäure

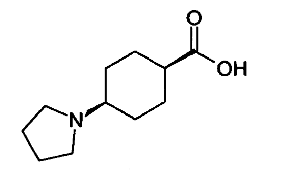

Eine Lösung von cis-4-Aminocyclohexancarbonsäuremethylester Hydrochlorid (4 g, 21 mmol) in 32 mL DMF wird mit 1,4-Dichlorbutan (2.3 mL, 21 mmol), Kaliumcarbonat (12.6 g, 91 mmol) und Kaliumjodid (400 mg) versetzt, 6 h bei 100 °C und anschließend 3 d bei RT gerührt. Die Reaktionsmischung wird mit 200 mL Wasser verdünnt, mit Eisessig neutralisiert, mit Natriumchlorid gesättigt und mit Dichlormethan extrahiert. Die organischen Phasen werden getrocknet, filtriert und eingeengt. Ausbeute: 3.8 g *cis-*4-(Pyrrolidin-1-yl)cyclohexancarbonsäuremethylester. Anschließend wird das Zwischenprodukt in 10 mL Methanol mit 25 mL Natronlauge (1 N) 15 h bei RT gerührt. Nach Entfernen des Methanols im Vakuum wird die Reaktionsmischung mit Salzsäure auf pH 6 eingestellt und weiter eingeengt. Der Rückstand wird in Methanol aufgenommen und durch Filtration über Kieselgel gereinigt. Ausbeute: 3.5 g

### R-7) 3-Morpholin-4-yl-cyclobutylamin

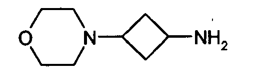

### R-7a) Toluol-4-sulfonsäure-(3-tert-butoxycarbonylamino-cyclobutyl)ester

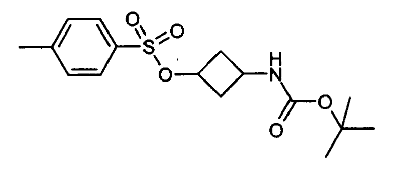

Eine Lösung von 3-*tert*-Butoxycarbonylaminocyclobutanol (18.7 g, 0.1 mol) und Triethylamin (12.1g, 0.12 mol) in 500 mL Chloroform wird bei 0 °C mit einer Lösung von Toluol-4-sulfonsäurechlorid (20.5 g, 0.105 mol) in 150 mL Chloroform getropft und anschließend auf RT erwärmt. Die organische Phase wird nacheinander mit Wasser, verdünnter Salzsäure, Natriumhydrogencarbonat-Lösung und wieder mit Wasser gewaschen, bevor sie getrocknet, filtriert und eingeengt wird.

### R-7b) 1-Morpholin-1-yl-3-tert-butoxycarbonylamino-cyclobutan

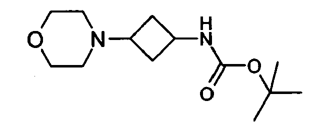

R-7a (34 g, 0.1 mol) wird in 750 mL Morpholin gelöst, mit einer katalytischen Menge DMAP versetzt und unter Argonatmosphäre 3 h bei 100 °C gerührt. Anschließend wird der Reaktionsansatz im Vakuum eingeengt, mit 100 mL Toluol coevaporiert und in 500 mL Ethylacetat aufgenommen. Die organische Phase wird mit gesättigter Natriumhydrogencarbonat-Lösung gewaschen, getrocknet, filtriert und eingeengt, wobei die gewünschte Verbindung erhalten wird, die ohne weitere Reinigung verwendet wird. R-7b (25.6 g 0.1 mol) wird mit 260 mL Salzsäure (2 N) versetzt und 2 h bei 40 °C gerührt. Nach vollständiger Reaktion wird die Reaktionsmischung mit methanolischer Ammoniaklösung alkalisch gestellt, filtriert und eingeengt. Der Rückstand wird anschließend aus Ethanol umkristallisiert, wobei R-7 erhalten wird.

### H-1) 4-Hydrazino-3-methyl-benzoesäuremethylester

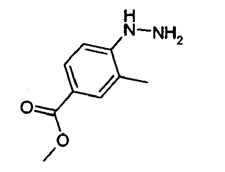

Methyl-4-amino-3-methyl-benzoat (10 g, 61 mmol) wird mit 50 mL konz. Salzsäure versetzt und auf -15 °C abgekühlt. Eine Lösung von Natriumnitrit (6.3 g, 91 mmol) in 50 mL Wasser wird so zugetropft, dass die Temperatur -5 °C nicht übersteigt. Nach 4 h Rühren bei -10 °C wird zu der Suspension eine Lösung von Zinn(II)chloriddihydrat in 50 mL konz. Salzsäure zugetropft, wobei die Reaktionstemperatur -5 °C nicht übersteigt. Die dickflüssige Suspension wird 15 h bei RT gerührt, bevor sie mit 200 mL Natronlauge (10N) auf pH 14 eingestellt wird. Die Reaktionsmischung wird über Kieselgur und Extrelut® (60 g) filtriert und mit 2 L Chloroform gespült. Die erhaltene organische Phase wird mit Wasser gewaschen (2 x 200 mL), über Natriumsulfat getrocknet und im Vakuum eingeengt. Der Rückstand wird mit 120 mL Petrolether verrührt und filtriert. Ausbeute: 6.3 g

### H-2) 4-Hydrazino-3-fluor-benzoesäuremethylester

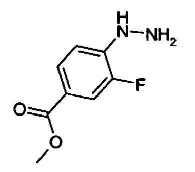

Analog zu Darstellung von H-1 wird ausgehend von Methyl-4-amino-3-fluor-benzoat (3.9 g, 23 mmol), Natriumnitrit (2.4 g, 34 mmol) und Zinn(II)chloriddihydrat (20.8 g, 92 mmol) die gewünschte Verbindung erhalten. Ausbeute: 3.4 g

### H-3) 3-Jod-phenylhydrazin

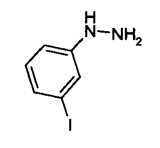

Analog zur Darstellung von H-1 wird ausgehend von 3-Jodanilin (2.75 g, 22.8 mmol), Natriumnitrit (1.58 g, 22.8 mmol) und Zinn(II)chloriddihydrat (15.4 g, 68.5 mmol) die gewünschte Verbindung als Hydrochlorid erhalten. Ausbeute: 3.55 g

### H-4) 3-Hydrazino-phenylessigsäure

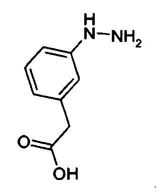

Analog zur Darstellung von H-1 wird ausgehend von 3-Aminophenylessigsäure (2 g, 13.2 mmol), Natriumnitrit (0.91 g, 13.2 mmol) und Zinn(II)chloriddihydrat (6.1 g, 26.4 mmol) die gewünschte Verbindung erhalten.

### H-5) Piperidin-4-yl-hydrazin

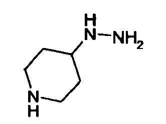

4-Oxo-piperidin-1-*tert*-butyoxycarbonyl (500 mg, 2.5 mmol) wird unter Argonatmosphäre in Hexan gelöst und mit *tert*-Butylcarbazat (332 mg, 2.5 mmol) versetzt. Nach 20 min Erhitzen unter Rückfluss wird die Reaktionsmischung abgekühlt und der entstandene Niederschlag filtriert. 4-(*tert*-Butoxycarbonylhydrazono)-piperidin-1-*tert*-butoxycarbonyl (707 mg, 2.3 mmol) wird mit Boran-THF-Komplex (1 M in THF; 2.2 mL) versetzt und 1 h bei RT gerührt. Anschließend wird das gewünschte Produkt mit 6 mL Salzsäure (4 N in Dioxan) als Hydrochlorid gefällt und filtriert.

### H-6) 4-Hydrazino-cyclohexancarbonsäureethylester

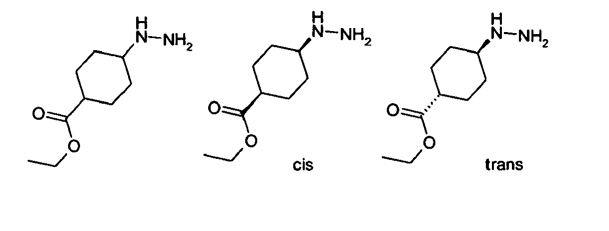

Analog zur Darstellung von H-5 wird ausgehend von 4-Oxo-cyclohexancarbonsäureethylester (4.5 g, 26.4 mmol), tert-Butylcarbazat (3.5 g, 26.4 mmol) Boran-THF-Komplex (1 M in THF; 26.5 mL) das gewünschte Produkt als cis/trans-Gemisch erhalten, welches ohne weitere Reinigung weiterverwendet wird oder chromatographisch an Kieselgel mit 0 - 33 % Ethylacetat in Cyclohexan getrennt wird.

| | |
|---|---|
| Ausbeute: | *cis:* 2.2 g |
| | *trans:* 2.6 g |

### H-7) 2-Chlor-4-hydroxymethylphenylhydrazin

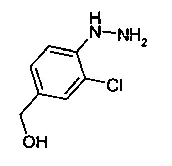

Zu einer Lösung von 3-Chlor-4-hydrazino-benzoesäuremethylester (9.5 g, 47 mmol) in 1 L Toluol wird unter Stickstoffatmosphäre bei -73 °C Diisobutylaluminiumhydrid (1M in Toluol, 190 mL) zugetropft, so dass die Temperatur nicht über -70 °C steigt. Die Reaktionsmischung wird 30 min bei -73 °C gerührt und anschließend auf-5 °C erwärmt. Nach Hydrolyse mit 500 mL Wasser wird der Niederschlag filtriert und mit Ethylacetat (5 x 500 mL) gewaschen. Die organische Phase wird eingeengt, der Rückstand in wenig Ethylacetat aufgenommen und mit Petrolether/Diethylether gefällt und filtriert. Der so erhaltene Feststoff wird an Kieselgel mit Cyclohexan/Ethylacetat chromatographisch gereinigt. Ausbeute: 2.9 g

### H-8) 3-Hydrazinomethylbenzoesäuremethylester

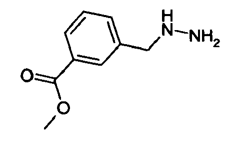

Eine Lösung von 3 g 3-Brommethylbenzoesäuremethylester (13 mmol), 2.6 g *tert-*Butoxycarbonylhydrazin (19 mmol) und 3.1 g Kaliumcarbonat (23 mmol) in 30 mL DMF wird 24 h bei RT gerührt. Nach Zugabe von Wasser wird die Reaktionsmischung mit Dichlormethan extrahiert. Die organische Phase wird getrocknet, filtriert und eingeengt und der Rückstand chromatographisch gereinigt. Anschließend wird die BOC-Schutzgruppe mit 30 mL Salzsäure (4 M in Dioxan) abgespalten. Ausbeute: 1.25 g

### H-9) (4-Trifluormethyl-pyridin-3-yl)-hydrazin

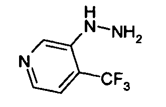

Analog zur Darstellung von H-1 wird ausgehend von 3-Amino-4-trifluormethylpyridin (5.1 g, 31 mmol), Natriumnitrit (2.2 g, 31 mmol) und Zinn(II)chloriddihydrat (21.6 g, 94 mmol) die gewünschte Verbindung erhalten. Ausbeute: 3.8 g

### H-10) N-(4-Hydrazinophenyl)-N-methyl-acetamid

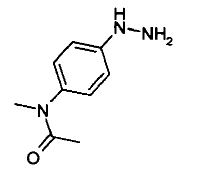

Analog zur Darstellung von H-1 wird ausgehend von *N*-(4-Aminophenyl)-*N*-methyl-acetamid (0.4 g, 2.4 mmol), Natriumnitrit (0.2 g, 2.7 mmol) und Zinn(II)chloriddihydrat (1.6 g, 7.3 mmol) die gewünschte Verbindung erhalten. Ausbeute: 0.2 g

### H-11) (4-Morpholin-4-ylmethyl-phenyl)-hydrazin

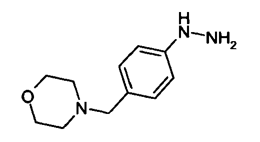

Analog zur Darstellung von H-1 wird ausgehend von 4-Morpholin-4-ylmethyl-phenylamin (1.1 g, 5 mmol), Natriumnitrit (0.3 g, 5 mmol) und Zinn(II)chloriddihydrat (5.8 g, 25 mmol) die gewünschte Verbindung erhalten. Ausbeute: 0.3 g

### H-12) Pyrrolidin-3-yl-hydrazin

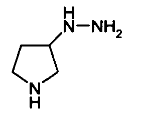

Analog der Darstellung von H-5 wird ausgehend von 3-Oxo-pyrrolidin-1-carbonsäure-*tert-*butylester die gewünschte Verbindung als Hydrochlorid erhalten.

### H-13) [3-(2-Methylpropan-1-sulfonyl)-phenyl]-hydrazin

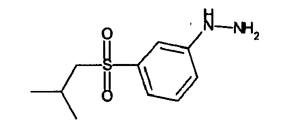

Analog der Darstellung von H-1 wird ausgehend von 3-(2-Methylpropan-1-sulfonyl)-phenylamin (1.5 g, 7 mmol), welches analog Literatur (A. Courtin, *Helv. Chim.Acta* **1981***,* 64, 1849) darstellbar ist, Natriumnitrit (0.5 g, 7 mmol) und Zinn(II)chloriddihydrat (4.9 g, 21 mmol) die gewünschte Verbindung als Hydrochlorid erhalten. Ausbeute: 1.8 g

### H-14) 4-Hydrazinophenylacetonitril

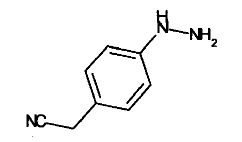

Analog der Darstellung von **H-1** wird ausgehend von 4-Aminophenylacetonitril (5 g 37.8 mmol), Natriumnitrit (2.6 g, 37.4 mmol) und Zinn(II)chloriddihydrat (25.8 g, 112 mmol) die gewünschte Verbindung als Hydrochlorid erhalten. Ausbeute: 5.4 g

### H-15) (1-Methyl-1H-indazol-5-yl)-hydrazin

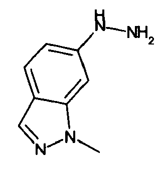

Analog der Darstellung von H-1 wird ausgehend von 1-Methyl-1*H*-indazol-5-ylamin (0.9 g 6 mmol), Natriumnitrit (0.44 g, 6.4 mmol) und Zinn(II)chloriddihydrat (2.8 g, 12.6 mmol) die gewünschte Verbindung als Hydrochlorid erhalten. Ausbeute: 1.2 g

### Synthese der Zwischenverbindungen

### Z-1) N-[7-Oxo-6-(pyrimidin-5-carbonyl)-4,5,6,7-tetrahydro-benzthiazol-2-yl]-acetamid

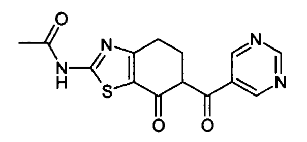

Eine Lösung von 10 g (48 mmol) Z-6 in 1 L THF wird auf -40 °C gekühlt und tropfenweise mit 143 mL (143 mmol) Li-HMDS (1 N in THF) versetzt, so dass die Temperatur -20 °C nicht überschreitet. Nach 3.5 h bei -40 bis -20 °C wird zur Suspension Pyrimidin-5-carbonylchlorid Hydrochlorid (10.2 g, 57 mmol) zugegeben und 16 h bei RT gerührt. Anschließend wird zur klaren Reaktionsmischung 200 mL Salzsäure (1 N in Et₂O) hinzugefügt, wobei ein Niederschlag gebildet wird. Die Suspension wird mit 300 mL Phosphatpuffer versetzt und nach Abtrennung der organischen Phase mit Ethylacetat extrahiert. Die vereinigten organischen Phasen werden über Magnesiumsulfat getrocknet und zur Trockene eingedampft. Der Rückstand wird aus Acetonitril kristallisiert. Ausbeute: 13.2 g. Das Rohprodukt wird ohne weitere Reinigung für weitere Synthesen verwendet

### Z-3) N-[7-Oxo-6-(pyrazin-2-carbonyl)-4,5,6,7-tetrahydro-benzthiazol-2-yl]-acetamid

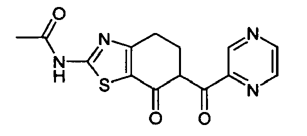

Zu einer Suspension von 8.1 g (143 mmol) Natriummethylat in 100 mL DMF werden 10 g (48 mmol) Z-6 portionsweise zugegeben, so dass die Temperatur nicht über 30 °C steigt. Nach 1 h Rühren bei RT wird die Reaktionsmischung auf 55 °C geheizt und mit einer Lösung von 10 g (72 mmol) Methylpyrazin-2-carboxylat in 40 mL Benzol versetzt. Die Lösung wird weitere 3 h bei 55 °C und 15 h bei RT gerührt, bevor sie mit Salzsäure (4 N in Dioxan) auf pH 3 eingestellt wird. Nach Hydrolyse mit Phosphatpuffer wird das Reaktionsgemisch mit Ethylacetat extrahiert. Die vereinigten organischen Phasen werden getrocknet und im Vakuum eingeengt. Der Rückstand wird aus Ether/Petrolether umkristallisiert. Ausbeute: 8.7 g

### Z-4) N-[6-(3-Nitrobenzoyl)-7-oxo-4,5,6,7-tetrahydrobenzthiazol-2-yl]-acetamid

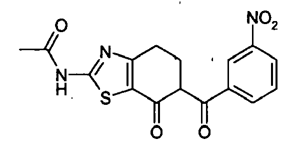

Analog zur Herstellung von Zwischenverbindung Z-1 werden aus 2.5 g (12 mmol) Z-6, 4.2 g (22 mmol) 3-Nitrobenzoylchlorid, 36 mL (36 mmol) Li-HMDS (1N in THF) 5.2 g Z-4 erhalten. Das Rohprodukt wird ohne weitere Reinigung für weitere Synthesen verwendet.

### Z-5) N-[6-(4-Nitrobenzoyl)-7-oxo-4,5,6,7-tetrahydrobenzthiazol-2-yl]-acetamid

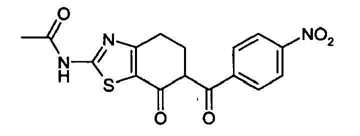

Analog zur Herstellung von Zwischenverbindung Z-1 werden aus 1 g (4.8 mmol) Z-6, 1.2 g (6.2 mmol) 4-Nitrobenzoylchlorid, 14.6 mL (14.6 mmol) Li-HMDS (1 N in THF) 2.2 g Z-5 erhalten. Das Rohprodukt wird ohne weitere Reinigung für weitere Synthesen verwendet.

### Z-6) N-(7-Oxo-4,5,6,7-tetrahydro-benzothiazol-2-yl)-acetamid

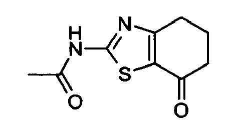

a) 112 g (1 mol) 1,3-Cyclohexandion werden in 700 mL Eiswasser suspendiert und 51.6 mL (1 mol) Brom bei 0 C innerhalb von 45 min zugetropft. Die Suspension wird 3.5 h bei max. 10 C nachgerührt. Anschließend wird abgesaugt und der Feststoff in 800 mL Wasser ausgerührt, abgesaugt, mit 3 L Wasser gewaschen und getrocknet. Der erhaltene Feststoff wird aus Ethanol umkristallisiert. Ausbeute: 37 g (Z-6a)
b) 15.5 g (0.2 mol) Thioharnstoff werden bei Raumtemperatur in 200 mL Ethanol vorgelegt. Zu dieser Suspension werden 37.1 g (0.2 mol) Z-6a portionsweise gegeben, dann wird mit 60 mL Ethanol nachgespült. Die allmählich entstehende Lösung wird 2 h unter Rückfluss gerührt und anschließend eingedampft. Der Rückstand wird mit Wasser und Diethylether extrahiert, die Wasserphase mit Natriumcarbonatlösung basisch gestellt. Der hierbei entstandene Feststoff wird abgesaugt, mit Wasser gewaschen, dann mit Methanol ausgerührt und zur Trockene eingedampft. Ausbeute: 22 g (Z-6b)
c) 230 mL (2.4 mol) Essigsäureanhydrid werden bei Raumtemperatur vorgelegt, 22 g (0.13 mol) Z-6b zugegeben und 3 h unter Rückfluss gerührt. Die Suspension geht dabei teilweise in Lösung. Nach dem Abkühlen mittels Eis/Kochsalzbad wird der Feststoff abgesaugt, 2x in je 150 mL Aceton aufgekocht, abgesaugt und getrocknet. Ausbeute: 25 g (Smp.: 268-272 °C)

### Z-7) N-(6-Formyl-7-oxo-4,5,6,7-tetrahydro-benzothiazol-2-yl)-acetamid

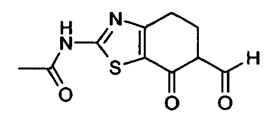

20 g (0.37 mol) Natriummethylat werden in 50 mL Dimethylformamid suspendiert, eine Suspension aus 21 g (0.1 mol) Z-6 in 100 mL DMF zugetropft. Es wird 15 min nachgerührt, dann auf 0 °C gekühlt. Ein Gemisch aus 29.9 ml (0.37 mol) Ameisensäureethylester und 60 mL Benzol wird zugetropft und die Reaktionsmischung mit weiteren 100 mL Benzol verdünnt. Allmählich fällt ein Niederschlag aus und es wird bei 0 °C 3.5 h weitergerührt. Die Suspension wird mit 370 mL 1 molarer Salzsäure hydrolysiert, der dabei ausgefallene Feststoff wird abgesaugt. Die zwei Phasen der Mutterlauge werden getrennt, die Wasserphase mit Dichlormethan extrahiert. Die daraus resultierende organische Phase wird getrocknet und zur Trockene eingedampft. Der Feststoff und der Rückstand aus der Extraktion werden aus Acetonitril umkristallisiert. Ausbeute: 20 g

### Z-8) N-[6-(Furän-2-carbonyl)-7-oxo-4,5,6,7-tetrahydro-benzothiazol-2-yl]-acetamid

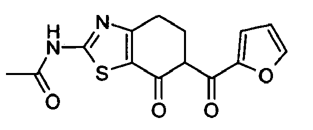

Analog zur Herstellung von Z-7 werden aus 2 g (10 mmol) Z-6, 1.6 g (30 mmol) Natriummethylat und 3.8 g (30 mmol) 2-Furansäuremethylester 1.7 g Z-8 erhalten. (Smp.: 255-256 °C)

### Z-10) (2-Acetylamino-7-oxo-4,5,6,7-tetrahydro-benzothiazol-6-yl)-oxo-essigsäuremethylester

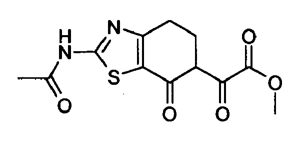

Analog zur Herstellung von Z-7 werden aus 40 g (190 mmol) Z-6, 38 g (0.7 mol) Natriummethylat und 84 g (0.7 mol) Dimethyloxalat 52 g Z-10 erhalten.

### Z-11) N-(6-Benzoyl-7-oxo-4,5,6,7-tetrahydro-benzothiazol-2-yl)-acetamid

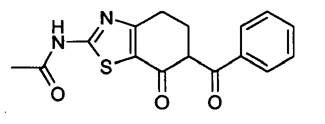

Analog zur Herstellung von Z-7 werden aus 10 g (50 mmol) Zwischenverbindung 1, 7.8 g (140 mmol) Natriummethylat und 17.9 ml (140 mmol) Benzoesäuremethylester 3.6 g Z-11 erhalten.

### Z-12) N-[7-Oxo-6-(pyridin-3-carbonyl)-4,5,6,7-tetrahydro-benzothiazol-2-yl]-acetamid

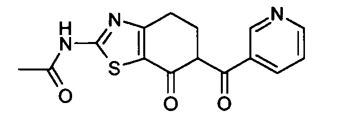

Analog zur Herstellung von Z-7 werden aus 4 g (19 mmol) Z-6, 3.9 g (57 mmol) Natriummethylat und 7.9 g (57 mmol) Nicotinsäuremethylester 3.1 g Produkt Z-12 erhalten.

### Z-13) [7-Oxo-6-(pyridin-3-carbonyl)-4,5,6,7-tetrahydro-benzthiazol-2-yl]-carbamidsäuremethylester

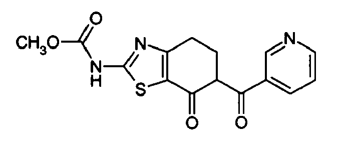

a) 2-Amino-5,6-dihydro-4*H*-benzthiazol-7-on Eine Suspension von 23 g Z-6 (109 mmol) in einem Gemisch aus 300 mL Salzsäure (4 M in Dioxan) und 30 mL Wasser werden 15 h bei 60 °C gerührt. Nach Abkühlung auf 0 °C wird die Reaktionsmischung mit 8 N Natronlauge basisch gestellt (pH 10). Der Niederschlag wird filtriert, mit Diethylether gewaschen und ohne weitere Reinigung weiterverwendet. Ausbeute: 22.4 g
b) (7-Oxo-4,5,6,7-tetrahydro-benzthiazol-2-yl)-carbamidsäuremethylester Zu einer Lösung von 500 mg 2-Amino-5,6-dihydro-4*H*-benzthiazol-7-on (2.4 mmol) in 5 mL Pyridin werden 572 µL Methylchlorformiat (7.3 mmol) gegeben. Die Reaktionsmischung wird 15 h bei 50 °C gerührt, anschließend mit Ethylacetat verdünnt, je 2 x mit Wasser und kalter 1 N Salzsäure ausgeschüttelt. Die organische Phase wird getrocknet und eingeengt. Ausbeute: 290 mg
c) Analog zur Darstellung von Z-1 wird aus 340 mg 7-Oxo-4,5,6,7-tetrahydro-benzthiazol-2-yl)-carbamidsäuremethylester (1.5 mmol), 4.7 mL Li-HMDS (1N in THF) und 520 mg Imidazol-1-yl-pyridin-3-yl-methanon (3 mmol) in 30 mL THF die gewünschte Verbindung erhalten. Ausbeute: 480 mg

### Z-14) [7-Oxo-6-(pyridin-3-carbonyl)-4,5,6,7-tetrahydro-benzthiazol-2-yl]-thiocarbamidsäureethylester

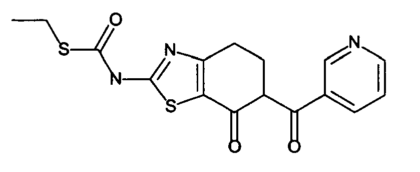

a) (7-Oxo-4,5,6,7-tetrahydro-benzthiazol-2-yl)-thiocarbamidsäureethylester Analog zur Darstellung von Z-13 b wird ausgehend von 101 g 2-Amino-5,6-dihydro-4*H-*benzthiazol-7-on (602 mmol) in 3.4 L Pyridin, 75 g Ethylchlorthioformiat (602 mmol) das gewünschte Thiocarbamat erhalten. Ausbeute: 84 g
b) Analog zur Darstellung von Z-1 wird aus 17 g (7-Oxo-4,5,6,7-tetrahydro-benzthiazol-2-yl)-thiocarbamidsäureethylester (67 mmol), 200 mL Li-HMDS (1N in THF) und 23 g Imidazol-1-yl-pyridin-3-yl-methanon (133 mmol) in 400 mL THF die gewünschte Verbindung erhalten. Ausbeute: 18 g

### Z-15) [7-Oxo-6-(pyrimidin-5-carbonyl)-4,5,6,7-tetrahydro-benzthiazol-2-yl]-thiocarbamidsäureethylester

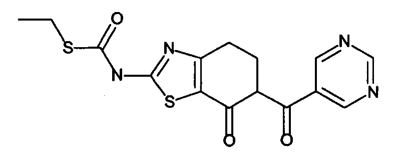

Analog zur Darstellung von Z-1 wird aus 12 g (7-Oxo-4,5,6,7-tetrahydro-benzthiazol-2-yl)-thiocarbamidsäureethylester (Z-14a, 46 mmol), 140 mL Li-HMDS (1N in THF) und 12 g Imidazol-1-yl-pyrimidin-5-yl-methanon (56 mmol) in 300 mL THF die gewünschte Verbindung erhalten. Ausbeute: 13 g

### I-1) 4-(7-Acetylamino-4,5-dihydro-pyrazolo[3',4':3,4]benz[1,2-d]thiazol-1-yl)-3-chlorbenzoesäure

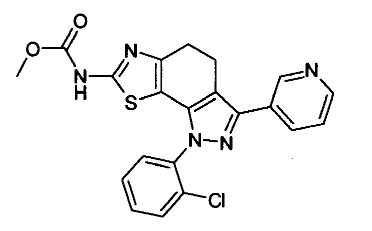

Eine Suspension von Z-13 (480 mg, 1.5 mmol) und 2-Chlorphenylhydrazin Hydrochlorid (267 mg, 1.5 mmol) in 10 mL Eisessig wird 4 h bei 100 °C gerührt. Anschließend wird die Reaktionsmischung mit 500 mL Wasser verdünnt und der Niederschlag abfiltriert. Ausbeute: 116 mg

### I-3) N-[1-(2-Chlor-pyridin-4-yl)-3-furan-2-yl-4,5-dihydro-1H-pyrazolo[3',4':3,4]benz[1,2-d]thiazol-7-yl]-acetamid

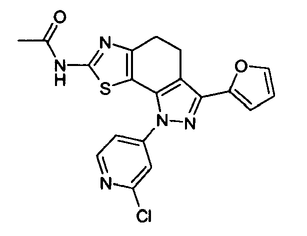

Analog zur Darstellung von 1-1 wird ausgehend von Z-8 (1.5 g, 5 mmol), 2-Chlorpyridin-4-yl-hydrazin Hydrochlorid in 25 mL Eisessig das gewünschte Produkt erhalten. Ausbeute: 1.6 g

### I-4) 4-(7-Acetylamino-3-furan-2-yl-4,5-dihydro-pyrazolo[3',4':3,4] benz[1,2-d]thiazol-1-yl)-3-chlor-benzoesäure

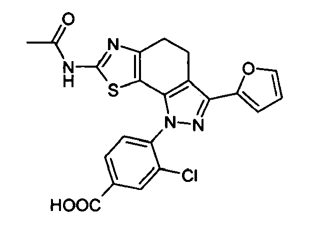

Analog zur Darstellung von 1-1 wird ausgehend von Z-8 (12 g, 35 mmol), 3-Chlor-4-hydrazinobenzoesäuremethylester (8.5 g, 35 mmol) in 80 mL Eisessig 4 d bei RT gerührt. Der nach Ausfällen in Eiswasser erhaltene Methylester (1.1 g) wird anschließend mit Lithiumhydroxid (178 mg in 15 mL Dioxan) verseift. Durch Ansäuern mit 2 N Salzsäure wird das gewünschte Produkt als Feststoff erhalten. Ausbeute: 0.8 g

### I-6) 7-Acetylamino-1-phenyl-4,5-dihydro-1H-pyrazolo[3',4':3,4] benz[1,2-d] thiazole-3-carbonsäure

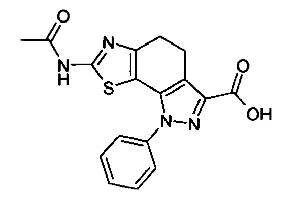

Analog zur Darstellung von **I-1** werden aus 30 g (0.1 mol) **Z-10** und 10.3 mL (0.1 mol) Phenylhydrazin 27 g Produkt erhalten (Schmp.: 298 - 300 °C). Von diesem werden 0.1 g (0.3 mmol) in 12 mL Methanol/Wasser (1:1) suspendiert und mit 0.4 mL 10%iger Kaliumhydroxid-Lösung versetzt. Nach 1.5 h wird die Reaktionsmischung eingeengt und die Lösung mit verdünnter Salzsäure sauer gestellt. Der entstandene Niederschlag wird aus Acetonitril umkristallisiert. Ausbeute: 0.1 g (Schmp.: > 300 °C)

### I-8) 4-(7-Acetylamino-3-phenyl-4,5-dihydro-pyrazolo[3',4':3,4] benz[1,2-d]thiazol-1-yl)-3-chlorbenzonitril

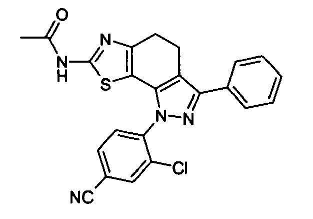

Analog zur Darstellung von I-1 wird ausgehend von Z-11 (10 g; 12.7 mmol), 3-Chlor-4-hydrazinobenzonitril (4.5 g, 26.8 mmol) in 50 mL Eisessig das gewünschte Produkt erhalten. Ausbeute: 1.6 g

### I-9) 4-(7-Acetylamino-3-phenyl-4,5-dihydro-pyrazolo[3',4':3,4] benz[1,2-d]thiazol-1-yl)-3-chlorbenzoesäure

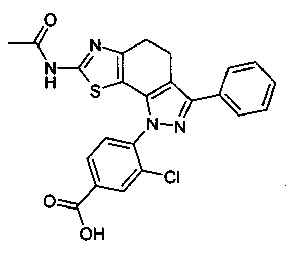

Analog zur Darstellung von I-4 wird ausgehend von Z-11 (2.3 g, 7.1 mmol) und 3-Chlor-4-hydrazinobenzoesäuremethylester (1.8 g, 8.5 mmol) die gewünschte Verbindung erhalten. Ausbeute: 0.36 g

### I-10) N-[1-(2-Chlor-4-nitro-phenyl)-3-phenyl-4,5-dihydro-1H-pyrazolo [3',4':3,4]benz[1,2-d]thiazol-7-yl]-acetamid

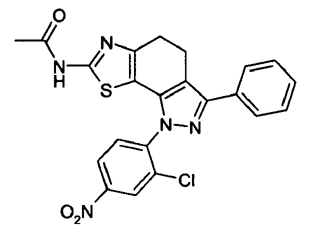

Analog zur Darstellung von 1-1 wird ausgehend von Z-11 (7.5 g; 13.1 mmol), 3-Chlor-4-hydrazinonitrobenzol (3.2 g, 14.4 mmol) in 100 mL Eisessig das gewünschte Produkt erhalten. Ausbeute: 1.1 g

### I-11) N-(1-Piperidin-4-yl-3-pyridin-3-yl-4,5-dihydro-1H-pyrazolo[3',4':3,4] benz[1,2-d]thiazol-7-yl)-acetamid

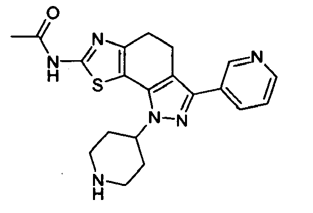

Analog zur Darstellung von I-1 wird ausgehend von Z-12 (2.5 g, 7.9 mmol), H-5 (1.2 g, 7.9 mmol) in 50 mL Eisessig 15 h bei 60 °C gerührt. Die Mischung wird auf Eiswasser gegossen und mit 1 N Natronlauge basisch gestellt (pH 12). Der entstandene Niederschlag wird filtriert, mit Wasser gewaschen und bei 40 °C im Vakuum getrocknet. Ausbeute: 2 g

### I-12) 4-(7-Acetylamino-3-pyridin-3-yl-4,5-dihydro-pyrazolo [3',4':3,4]benz[1,2-d]thiazol-1-yl)-cyclohexancarbonsäure

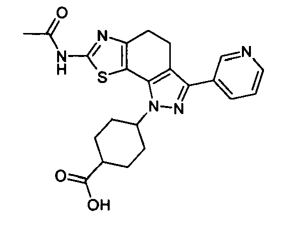

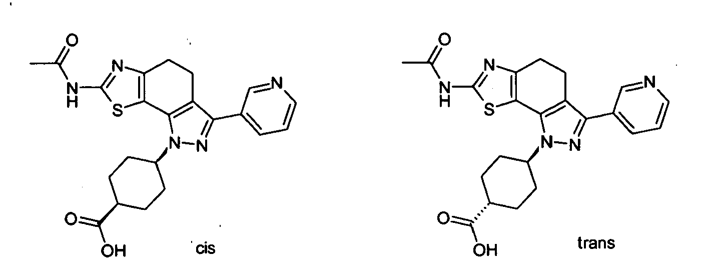

Analog zur Darstellung von I-4 wird ausgehend von Z-12 (1.9 g, 5.8 mmol) und H-6 (1.3 g, 5.8 mmol) die gewünschte Verbindung erhalten. Ausbeute: 0.78 g.

Durch Verwendung des isomerenreinen *cis*H-6 beziehungsweise *trans*H-6 werden die entsprechenden *cis-* beziehungsweise *trans*-Verbindungen erhalten.

Weitere Zwischenverbindungen, die analog zu den oben beschriebenen Synthesen hergestellt werden.

| **Nr.** | **Edukt** | **Struktur** | **Nr.** | **Edukt** | **Struktur** |
|---|---|---|---|---|---|
| I-13 | Z-12 | 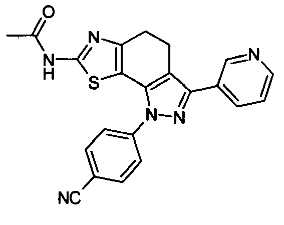 | I-30 | Z-3 | 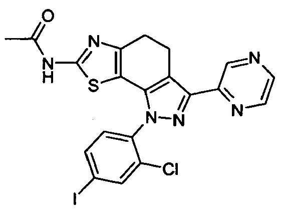 |
| I-14 | Z-12 | 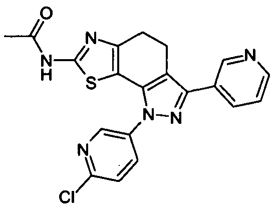 | I-31 | Z-3 | 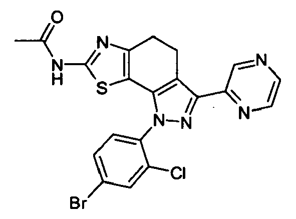 |
| I-15 | Z-12 | 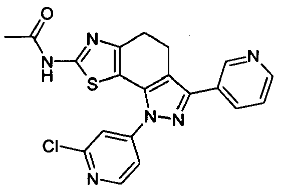 | I-32 | Z-4 | 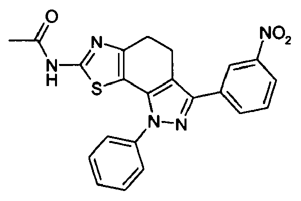 |
| I-16 | Z-12 | 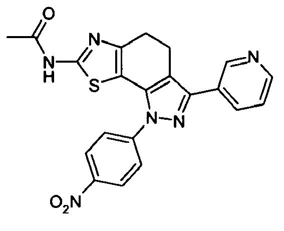 | I-33 | Z-4 | 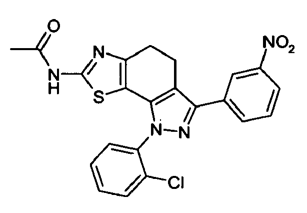 |
| I-17 | Z-12 | 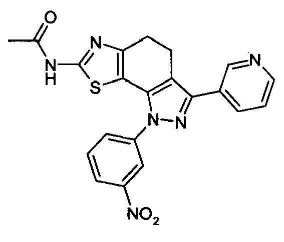 | I-34 | Z-5 | 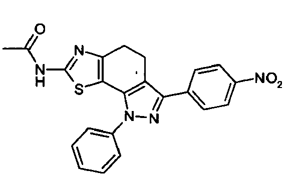 |
| I-18 | Z-12 | 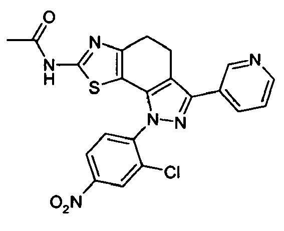 | I-35 | Z-5 | 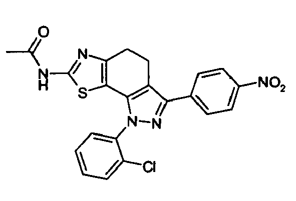 |
| I-19 | Z-12 | 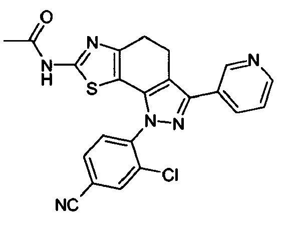 | I-36 | Z-12 | 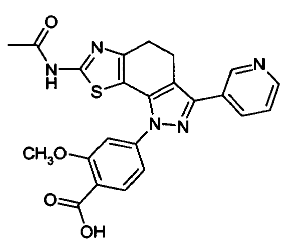 |
| I-20 | Z-12 | 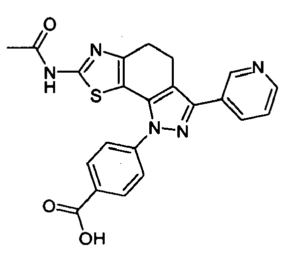 | I-37 | Z-11 | 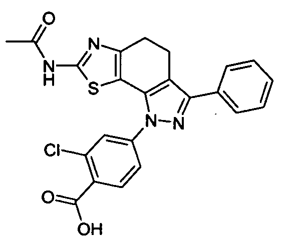 |
| I-21 | Z-12 | 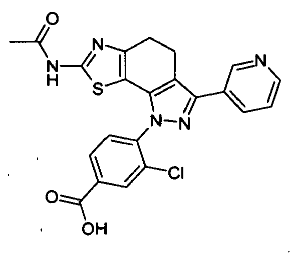 | I-38 | Z-11 | 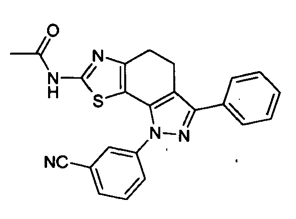 |
| I-22 | Z-12 | 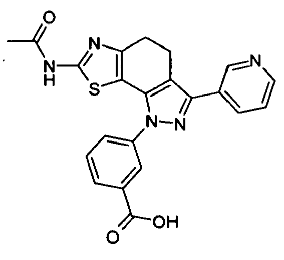 | I-39 | Z-12 | 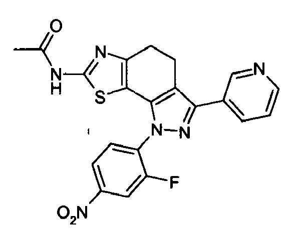 |
| I-23 | Z-12 H-2 | 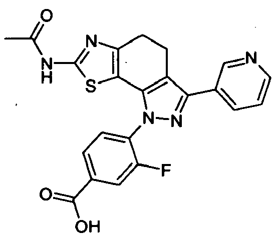 | I-40 | Z-12 H-12 | 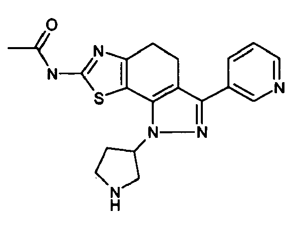 |
| I-24 | Z-12 H-3 | 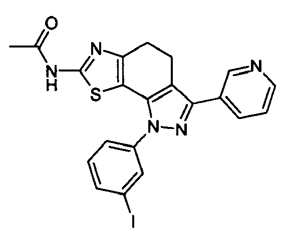 | I-41 | Z-14 | 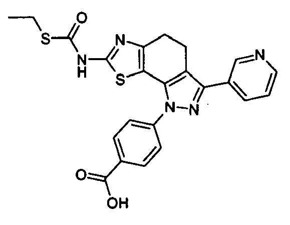 |
| I-25 | Z-12 | 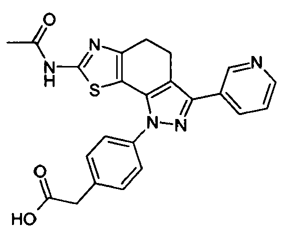 | I-42 | Z-14 | 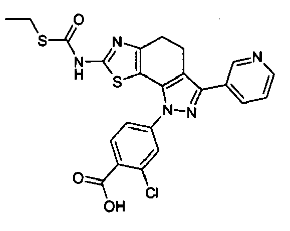 |
| I-26 | Z-12 H-4 | 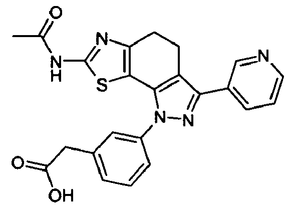 | I-43 | Z-14 | 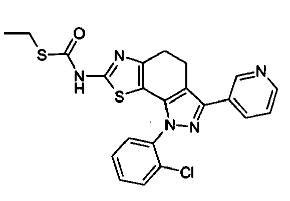 |
| I-27 | Z-1 | 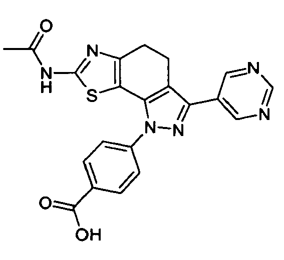 | I-44 | Z-15 | 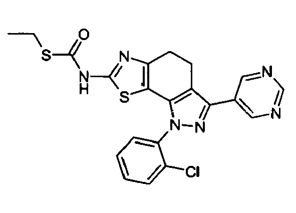 |
| I-28 | Z-3 H-1 | 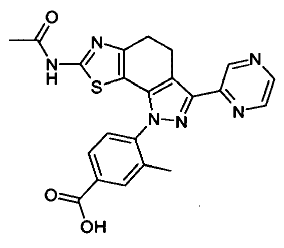 | I-45 | Z-3 | 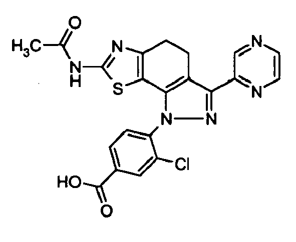 |
| I-29 | Z-3 H-7 | 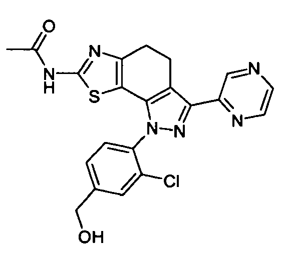 | I-46 | Z-12 | 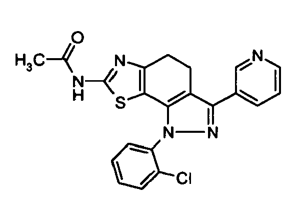 |

### II-1) N-[1-(4-Amino-2-chlorphenyl)-3-furan-2-yl-4,5-dihydro-1H-pyrazolo [3',4':3,4]benz[1,2-d]thiazol-7-yl]-acetamid

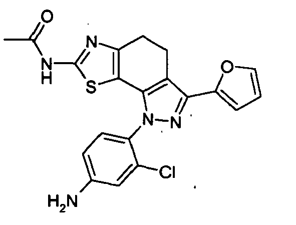

Eine Lösung von I-4 (2.1 g, 4.6 mmol) in 20 mL DMF wird mit Phosphorsäurediphenylesterazid (1.1 mL, 5 mmol) und 0.7 mL Triethylamin versetzt und 6 h bei 50 °C gerührt. Zur Reaktionsmischung wird *p*-Toluolsulfonsäure (1.5 g, 9.1 mmol) und 3 mL Wasser gegeben und 39 h bei 50 °C gerührt. Anschließend wird der Ansatz auf 300 mL Eiswasser gegossen und der entstehende Niederschlag filtriert. Der Rückstand wird chromatographisch an Kieselgel mit Dichlormethan:Methanol:Ammoniak 98:2:0.2 gereinigt. Ausbeute: 0.6 g

### II-3) N-[1-(4-Amino-2-chlorphenyl)-3-phenyl-4,5-dihydro-1H-pyrazolo [3',4':3,4]benz[1,2-d]thiazol-7-yl]-acetamid

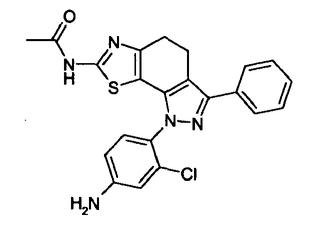

Eine Lösung von I-10 (1.1 g, 2 mmol) in 20 mL Eisessig wird mit Eisenpulver (0.77 g, 13.8 mmol) versetzt und 4 h bei 70 °C gerührt. Nach Filtration über Kieselgur wird das Lösungsmittel im Vakuum entfernt und der Rückstand chromatographisch an Kieselgel mit Dichlormethan:Methanol 99:1 gereinigt. Ausbeute: 0.8 g

### II-4) N-[1-(4-Aminophenyl)-3-pyridin-3-yl-4,5-dihydro-1H-pyrazolo[3',4':3,4] benz[1,2-d]thiazol-7-yl]-acetamid

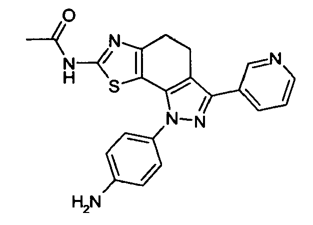

Analog der Darstellung von II-2 wird ausgehend von I-16 (0.2 g, 0.5 mmol) das gewünschte Produkt erhalten. Ausbeute: 0.14 g

### II-5) N-[1-(3-Aminophenyl)-3-pyridin-3-yl-4,5-dihydro-1H-pyrazolo[3',4':3,4] benz[1,2-d]thiazol-7-yl]-acetamid

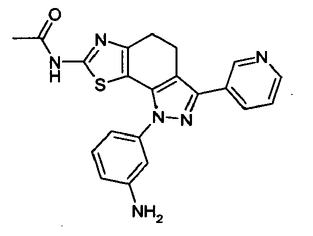

In einem 250 ml Hydrierreaktor wird I-17 (1.2 g, 2.8 mmol) in 150 mL Methanol suspendiert. Die Suspension wird mit Palladium (5% auf Aktivkohle, 120 mg) versetzt und bei RT und 50 psi Wasserstoffdruck gerührt. Nach 18 und nach 40 h wird jeweils nochmals dieselbe Menge an Katalysator zugegeben. Nach weiteren 15 h wird der Katalysator abfiltriert und das Lösungsmittel im Vakuum entfernt. Ausbeute 0.89 g

### II-6) N-[1-(4-Amino-2-chlorphenyl)-3-pyridin-3-yl-4,5-dihydro-1H-pyrazolo [3',4':3,4] benz[1,2-d]thiazol-7-yl]-acetamid

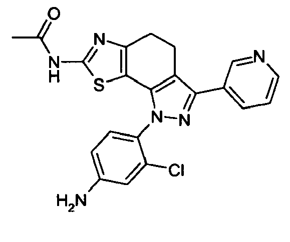

Analog der Darstellung von II-3 wird ausgehend von I-18 (10 g, 21 mmol) das gewünschte Produkt erhalten. Ausbeute: 8.4 g

### II-9) [4-(7-Acetylamino-3-phenyl-4,5-dihydro-pyrazolo[3',4':3,4]benz[1,2-d]thiazol-1-yl)-3-chlorphenyl]-thiocarbamidsäureethylester

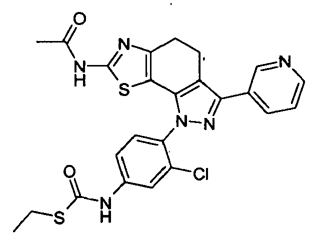

Zu einer Lösung von II-6 (3 g, 5.8 mmol) in 75 mL Pyridin wird Ethylchlorthioformiat (0.7 mL) getropft und 4 h bei RT gerührt. Nach Entfernen des Pyridins am Rotationsverdampfer wird der Rückstand in Wasser aufgenommen und der Niederschlag filtriert. Man erhält einen Feststoff, der 15 h bei 60 °C im Vakuum getrocknet wird. Ausbeute: 3 g

### II-10) N-[1-(4-Aminomethyl-2-chlorphenyl)-3-phenyl-4,5-dihydro-1H-pyrazolo [3',4':3,4]benz[1,2-d]thiazol-7-yl]-acetamid

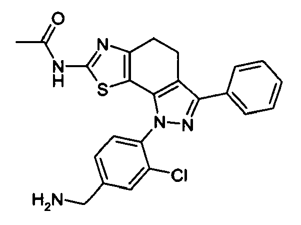

Eine Lösung von I-8 (1.5 g, 3.3 mmol) in 150 mL ammoniakalischem Methanol wird mit 20 mg Raney-Nickel versetzt und 15 h unter Wasserstoffatmosphäre (3.5 bar) bei RT gerührt. Der Ansatz wird in Dichlormethan aufgenommen und über Kieselgel filtriert. Nach Entfernung des Lösungsmittels im Vakuum wird der Rückstand aus Diethylether/Petrolether kristallisiert. Ausbeute: 1.1 g

### II-11) N-[1-(2-Chlor-4-formyl-phenyl)-3-pyrazin-2-yl-4,5-dihydro-1H-pyrazolo [3',4':3,4]benz[1,2-d]thiazol-7-yl]-acetamid

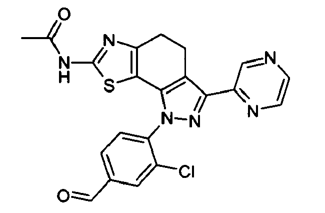

Eine Lösung von 1-29 (0.5 g, 1.1 mmol) in 25 mL Aceton wird mit 2 g Mangandioxid versetzt und 3 h refluxiert. Anschließend wird die Reaktionsmischung filtriert und das Lösungsmittel im Vakuum entfernt. Ausbeute 0.25 g

### II-12) N-[1-(3-Aminomethyl)-3-phenyl-4,5-dihydro-1H-pyrazolo [3',4':3,4]benz [1,2-d] thiazol-7-yl]-acetamid

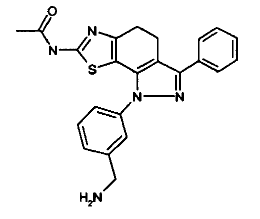

Analog der Darstellung von II-10 wird ausgehend von I-38 (2.3 g, 5.6 mmol) das gewünschte Produkt erhalten. Ausbeute: 1.4 g

### II-13) N-[1-(4-Amino-2-Fluorphenyl)-3-pyridin-3-yl-4,5-dihydro-1H-pyrazolo [3',4':3,4] benz[1,2-d]thiazol-7-yl]-acetamid

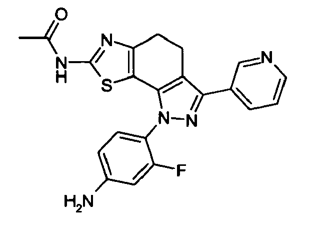

Analog der Darstellung von II-3 wird ausgehend von I-39 (1 g, 2.2 mmol) das gewünschte Produkt erhalten. Ausbeute: 1 g

### II-14) 4-(7-Amino-3-pyridin-3-yl-4,5-dihydro-pyrazolo[3',4':3,4] benz[1,2-d]thiazol-1-yl)-N,N-dimethyl-benzamid

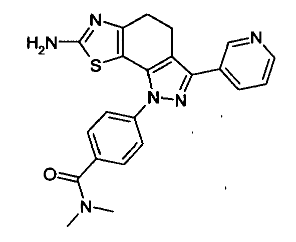

Eine Lösung von I-20 (6.3 g, 14.5 mmol) in 150 mL Dioxan wird mit 10 mL konz. Salzsäure und 100 mL Wasser versetzt und 6 h unter Rückfluss erhitzt. Die klare Lösung wird weitere 16 h bei 50 °C gerührt und anschließend im Vakuum bis zur Trockene eingeengt. Das Deacetylierungsprodukt (6.6 g, 14 mmol) wird in 150 mL DMF gelöst und mit TBTU (5.1 g, 15 mmol) und 10 mL Triethylamin versetzt. Die Reaktionsmischung wird 30 min bei RT gerührt, mit Dimethylamin Hydrochlorid (1.25 g, 15 mmol) versetzt und weitere 6 h bei RT gerührt. Nach Hydrolyse mit 1 L Wasser wird der entstandene Niederschlag filtriert und im Vakuum getrocknet. Ausbeute: 5.5 g

### II-15) 4-(7-Amino-3-pyridin-3-yl-4,5-dihydro-pyrazolo[3',4':3,4] benz[1,2-d]thiazol-1-yl)-3-chlor-N,N-dimethyl-benzamid Dihydrochlorid

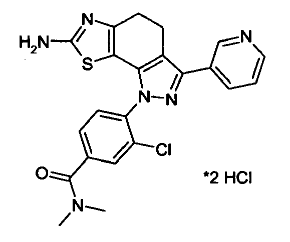

Eine Lösung von Beispiel 2.180 (500 mg, 1 mmol) mit 5.5 mL Salzsäure (4 M in Dioxan) und 5.5 mL Wasser versetzt und 2.5h bei 80 °C erhitzt. Die klare Lösung wird weitere 15 h bei 70 °C gerührt und anschließend im Vakuum bis zur Trockene eingeengt. Ausbeute: 580 mg

### II-16) 4-(7-Amino-3-pyridin-3-yl-4,5-dihydro-pyrazolo[3',4':3,4] benz[1,2-d]thiazol-1-yl)-3-fluor-N,N-dimethyl-benzamid

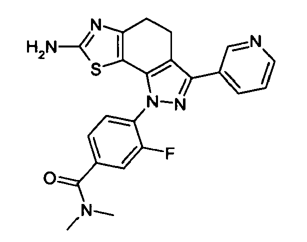

Eine Lösung von Beispiel 2.182 (1.37 g, 2.9 mmol) mit 30 mL Salzsäure (37 %) und 35 mL Wasser versetzt und 12 h bei 50 °C erhitzt. Nach Einengen im Vakuum wird der Rückstand in Wasser aufgenommen und mit 4 N Natronlauge versetzt. Der entstehende Niederschlag wird filtriert und getrocknet. Ausbeute: 1.1 g

### II-17) 1-(2-Chlorphenyl)-3-pyridin-3-yl-4,5-dihydro-1H-pyrazolo [3',4':3,4]benz [1,2-d]thiazol-7-ylamin

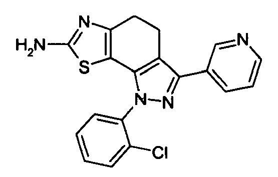

Analog zur Darstellung von Beispiel II-16 wird ausgehend von **I-46** (10.3 g, 24 mmol), 280 mL Salzsäure (37%) in 330 mL Wasser das gewünschte Produkt erhalten. Ausbeute: 7.6 g

### II-18) 1-Phenyl-3-pyridin-3-yl-4,5-dihydro-1H-pyrazolo[3',4':3,4] benz[1,2-d]thiazol-7-ylamin

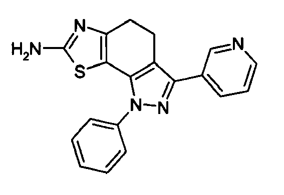

Analog zur Darstellung von II-16 wird ausgehend von Beispiel 1.13 (7.7 g, 20 mmol), 50 mL Salzsäure (37%) in 30 mL Wasser das gewünschte Produkt erhalten. Ausbeute: 6.8 g **II-19)** 4-(7-Amino-3-pyridin-3-yl-4,5-dihydro-pyrazolo[3',4':3,4] benz[1,2-*d*]thiazol-1-yl)-3-chlorbenzoesäure

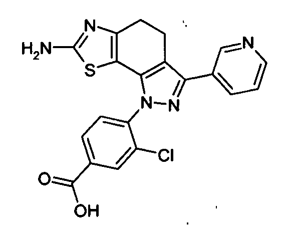

Analog zur Darstellung von Zwischenverbindung II-16 wird ausgehend von Beispiel 1.21 (2.9 g, 5 mmol), 2 mL Salzsäure (32%) in einem Gemisch aus 10 mL Wasser und 20 mL Dioxan das gewünschte Produkt erhalten. Ausbeute: 2.7 g

### Analytische Methoden

### Methode AM1:

HPLC: Agilent 1100 Series; MS: 1100 Series LC/MSD (API-ES (+/- 3000V, Quadrupol, G1946D); Mode: Scan pos 100-1000, neg 100-1000
- Säule:: Waters; Part No. 186000594; XTerra MS C18 2.5µm; 2.1x50mm column
- Lösungsmittel:: A: H₂O entsalzt mit 0, 1 % Ameisensäurezusatz
B: Acetonitril HPLC grade mit 0,1% Ameisensäurezusatz
- Detektion:: peakwide >0, 1 min (2s); 190-450nm
UV 254nm (bandwide 8, reference off)
UV 230nm (bandwide 8, reference off)

Injektion: 1 µL standard injection Fluss: 0.6 mL/min Säulentemperatur: 35 C°
Pumpengradient:

| | |
|---|---|
| 0.0 - 0.5min | 5% B |
| 0.5 - 1.5min | 5% -> 50% B |
| 1.5 - 4.Omin | 50% -> 95% B |
| 4.0 - 6.0m | 95% B |
| 6.0 - 6.5min | 95% -> 5% B |
| 1.5 min post run | 5% B |

### Methode AM2

HPLC: Agilent Series 1100 (G1379A/G1310A umgerüstet auf G1311A/G1313A/G1316A/ G1948D/G1315B/G1946D) Mode: Scan pos 100-1000, neg 100-1000
- Säule:: Agilent Zorbax SB-C8, 2.1x50 mm, 3.5µm
- Lösungsmittel:: A: H₂O entsalzt mit 0, 1 % Ameisensäurezusatz
B: Acetonitril HPLC grade mit 0, 1 % Ameisensäurezusatz
- Detektion:: peakwide >0, 1 min (2s); 190-450nm
UV 254nm (bandwide 8, reference off)
UV 230nm (bandwide 8, reference off)

Injektion: 2.5 µL standard injection
Fluss: 0. 6mL/min Säulentemperatur: 35 C°
Pumpengradient:

| | |
|---|---|
| 0-3,0 min | 10% -> 90% B |
| 3.0 - 4.0 min | 90% B |
| 4.0-5.0min | 90% -> 10% B |

### Methode AM3

HPLC: Agilent Series 1100 (G1312A/G1315A/G1316A/G1367A) Agilent MSD SL ESI
Mode: Scan pos 150-750
Säule: Agilent Zorbax SB-C8, 2.1x50 mm, 3.5µm
- Lösungsmittel:: A: H₂O entsalzt mit 0,1 % Ameisensäurezusatz
B: Acetonitril HPLC grade mit 0,1 % Ameisensäurezusatz
- Detektion:: peakwidth >0,01min (0.2s); 190-450nm
UV 254nm (bandwide 16, reference off)
UV 230nm (bandwide 8, reference off)
UV 214nm (bandwide 8, reference off)
- Injektion:: 3.0 µl overlap injection
- Fluss:: 1.1 ml/min

Säulentemperatur: 45C°
Pumpengradient:

| | |
|---|---|
| 0 - 1.75 min | 15% -> 95% B |
| 1.75 -1.90 min | 95% B |
| 1.90 - 1.92min | 950% -> 15% B |

### Methode AM4

HPLC: Agilent 1100 Series
MS: Agilent LC/MSD SL (LCMS1: 1100 series LC/MSD)
Säule: Waters, Xterra MS C18, 2.5µm, 2.1x30 mm, Part.No.186000592
Lösungsmittel A: H₂O entsalzt mit 0, 1 % Ameisensäurezusatz
B: Acetonitril HPLC grade mit 0,1% Ameisensäurezusatz
Detektion:

| | | |
|---|---|---|
| MS: | Positive and negative | |
| Mass range: | 120 - 900 m/z | |
| Fragmentor: | 120 | |
| Gain EMV: | 1 | |
| Threshold: | 150 | |
| Stepsize: | 0.25 | |
| UV: | 254 nm | |
| Bandwide: | 1 | (LCMS1: 2) |
| Reference: | off | |
| Spectrum: | | |
| Range: | 250-400 nm | |
| Range step: | 1.00 nm | |
| Threshold: | 4.00 mAU | |
| Peakwidth: | < 0.01 min | (LCMS1: >0.05 min) |
| Slit: | 1 nm | (LCMS1: 2 nm) |

Injektion: 5 µL
Fluß: 1.10 mL/min
Säulentemperatur: 40 °C
Gradient:

| | |
|---|---|
| 0.00 min | 5 % B |
| 0.00 - 2.50 min | 5 % -> 95 % B |
| 2.50 - 2.80 min | 95 % B |
| 2.81 - 3.10 min | 95 % -> 5 % B |

### Methode AM5

HPLC: Agilent 1100 Series
MS: Agilent LC/MSD SL (LCMS1: 1100 series LC/MSD)
Column: Phenomenex, Synergi Polar RP 80A, 4 µm, 2.0x30 mm, Part.No.00A-4336-B0
Solvent: A: H2O (Millipore purified purest water) with 0, 1 % HCOOH
B: Acetonitril (HPLC grade)
Detection:

| | |
|---|---|
| MS: | Positive and negative |
| Mass range: | 120 - 900 m/z |
| Fragmentor: | 120 |

Gain EMV: 1
Threshold: 150
Stepsize: 0.25
UV: 254 nm
Bandwide: 1 (LCMS1: 2)
Reference: off
Spectrum:

| | | |
|---|---|---|
| Range: | 250-400 nm | |
| Range step: | 1.00 nm | |
| Threshold: | 4.00 mAU | |
| Peakwidth: | < 0.01 min | (LCMS1: >0.05 min) |
| Slit: | 1 nm | (LCMS1: 2 nm) |

Injection: Inj. Vol.: 5 µL
Inj. mode: Needle wash
Separation:

| | | |
|---|---|---|
| Flow: | 1.10 mL/min | |
| Column temp.: | 40 °C | |
| Gradient: | 0.00 min | 5 % solvent B |
| | 0.00 - 2.50 min | 5 % -> 95 % solvent B |
| | 2.50 - 2.80 min | 95 % solvent B |
| | 2.81 - 3.10 min | 95 % -> 5 % solvent B |

### Methode AM6

HPLC: Waters Alliance 2695
Säule: Waters, Xterra MS C18, 2.5 µm, 4.6x30 mm, Part.No.186000600
Lösungsmittel A: H₂O entsalzt mit 0,1% Ameisensäurezusatz
B: Acetonitril HPLC grade mit 0,08 % Ameisensäurezusatz
Fluß: 1 mL/min
Säulentemperatur: 25 °C
Gradient:

| | |
|---|---|
| 0.00 min | 5 % B |
| 0.00-3.10 min | 5%->98%B |
| 3.10-4.50 min | 98%B |
| 4.50 - 5.00 min | 98 % -> 5 % B |

### Verwendete Abkürzungen

- d: Tag
- DC: Dünnschichtchromatographie
- DCM: Dichlormethan
- DMAP: *N,N*-Dimethylaminopyridin
- DMF: *N,N*-Dimethylformamid
- DMSO: Dimethylsulfoxid
- EtOH: Ethanol
- h: Stunde
- HATU: *O*-(7-Azabenztriazol-1-yl)-*N,N,N,N'-*tetramethyluroniumhexafluorphosphat
- HPLC: Hochdurchsatzflüssigchromatographie
- konz.: konzentriert
- Li-HMDS: Lithiumhexamethyldisilazan
- M: Molar
- MeOH: Methanol
- min: Minute
- mL: Milliliter
- MS: Massenspektrometrie
- N: Normal
- NMR: Kernresonanzspektroskopie
- ppm: part per million
- Rf: Retentionsfaktor
- RP: Reversed Phase
- RT: Raumtemperatur
- Rt: Retentionszeit
- Smp: Schmelzpunkt
- TBTU: *O*-Benztriazol-1-yl-*N*,*N*,*N*',*N*'-tetramethyluroniumtetrafluorborat
- *tert*: tertiär
- THF: Tetrahydrofuran

### Beispiele 1.1 - 1.13

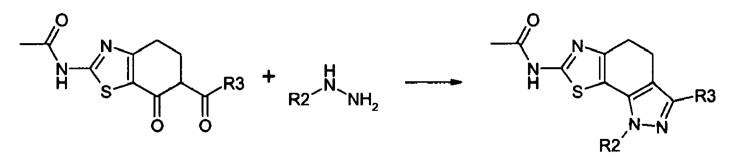

Die Darstellung der Beispiele 1.1 - 1.13 erfolgt analog der Synthese I-1.

| **#** | **Edukt** | **Struktur** | **Masse [M+1]⁺** | **HPLC Rt [min]** |
|---|---|---|---|---|
| **1.1** | **Z-12** | 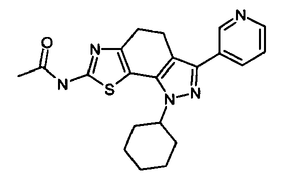 **¹H-NMR DMSO-d6,** □ [ppm]: 8.86 (s, 1H), 8.53 (d, 4 Hz, 1H), 8.02 (d, 7.7 Hz, 1H), 7.46 (dd, 7.7 u. 4.8 Hz, 1H), 4.23 (m, 1H), 3.05-3.00 (m, 2H), 2.98-2.93 (m, 2H), 2.18 (s, 3H), 2.05-1.96 (m, 2H), 1.95-1.87 (m, 4H), 1.5-1.4 (m, 2H), 1.32-1.23 (m, 2H). | 394 | 1.88 |
| **1.2** | **Z-12** | 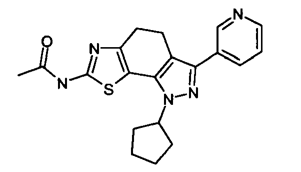 | 380 | 1.67 |
| **1.3** | **Z-8 H-8** | 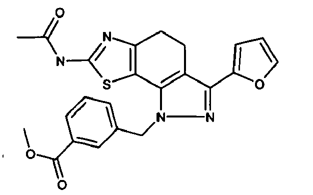 | 449 | 2.1 |
| **1.4** | **Z-12 H-9** | 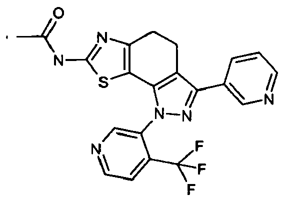 | 457 | 1,54+1,71 |
| **1.5** | **Z-12 H-10** | 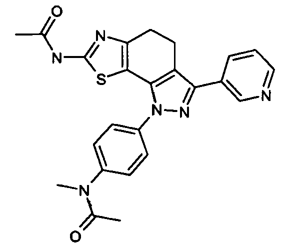 | 459 | 1.45 |
| **1.6** | **Z-12 H-11** | 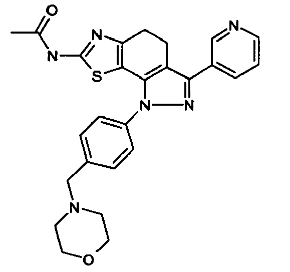 | 487 | 2.53 |
| **1.7** | **Z-1** | 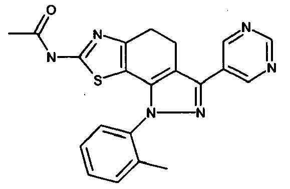 | 403 | 1.81 |
| **1.8** | **Z-1** | 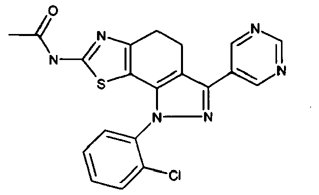 | 423 | 1.82 |
| **1.9** | **Z-12** | 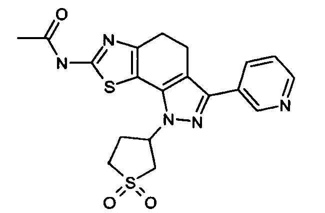 | 430 | 1.53 |
| **1.10** | **Z-12 H-13** | 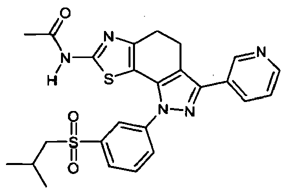 | 508 | 1.67 |
| **1.11** | **Z-12 H-14** | 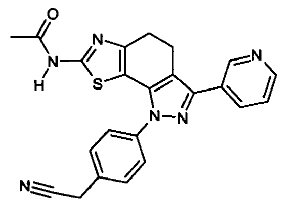 | 427 | 1.49 |
| **1.12** | **Z-12 H-15** | 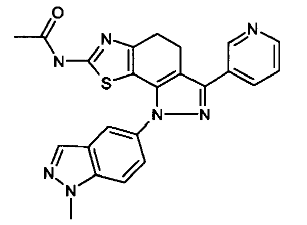 | 442 | 1.48 |
| 1.13 | | 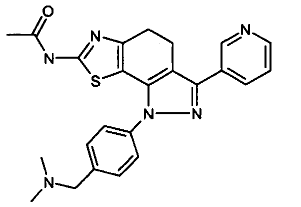 | 445 | 1.19 |

### Beispiele 2 - Umsetzung der Carbonsäuren mit Aminen

### Synthesemethode A

Eine Lösung der Carbonsäure (0.1 mmol) in 5 mL Dichlormethan wird mit TBTU (0.15 mmol) und Triethylamin (0.65 mmol) versetzt und 15 min bei RT gerührt. Anschließend wird das entsprechende Amin (0.1 mmol) hinzugegeben und bei RT bis zum vollständigen Umsatz gerührt. Die Reaktionsmischung wird mit wässriger 5%iger Kaliumcarbonatlösung versetzt und mit Dichlormethan extrahiert. Die vereinigten organischen Phasen werden getrocknet und im Vakuum eingeengt. Der Rückstand wird aus Petrolether kristallisiert oder chromatographisch gereinigt.

### Synthesemethode B

Eine Lösung der Carbonsäure (0.35 mmol) in 5 mL DMF (oder Dichlormethan, oder THF) wird mit HATU (0.55 mmol) und Diisopropylethylamin (1.8 mmol) versetzt und 15 min bei RT gerührt. Nach Zugabe des entsprechenden Amins (0.39 mmol) wird 15 h bei RT gerührt, mit wässriger 5%iger Kaliumcarbonatlösung versetzt und mit Dichlormethan extrahiert. Die vereinigten organischen Phasen werden getrocknet und im Vakuum eingeengt. Der Rückstand wird chromatographisch gereinigt.

### Synthesemethode C

Die Synthese erfolgt analog Synthesemethode B, allerdings wird Triethylamin statt Diisopropylethylamin verwendet.

### Synthesemethode D

Die Carbonsäure wird zunächst an ein Polymer immobilisiert. Dazu werden 1.2 g PL-TFP-Harz (1.25 mmol/g, 150-300µm; Polymer Laboratories) mit 12 mL Dichlormethan versetzt und 5 min später nacheinander die entsprechende Carbonsäure (1.2 mmol in 6 mL DMF), DMAP (0.7 mmol in 6 mL Dichlormethan) und 0.8 mL Diisopropylcarbodiimid zupipettiert. Der Ansatz wird 36 h bei RT stehengelassen. Das Harz wird über eine Glasfritte (Porosität 4) abfiltriert und 4 x mit je 15 mL DMF, 4 x mit je 20 mL Dichlormethan, und 4 x mit je 20 mL THF gewaschen, wobei jeweils das Lösungsmittel ohne Vakuum/Druck durch die Glasfritte abtropft und vor jeder neuen Lösungsmittelaufgabe trocken gesaugt wird. Das gewaschene Harz wird 2 d bei RT und 0.2 mbar getrocknet. Ausbeute trockenes Harz: 2.204 g

Zur Umsetzung der immobilisierten Carbonsäure werden 110 mg (0.15 mmol) des so präparierten Harzes in 1 mL Dichlormethan und 0.5 mL DMF vorgelegt und mit Amin (0.1 mmol) und Diisopropylethylamin (0.1 mmol) versetzt. Anschließend wird der Ansatz 15 h bei RT langsam gerührt. Nach Beendigung der Reaktion wird das Harz wie oben beschrieben filtriert und mit 8 x 3 mL Dichlormethan gewaschen. Das Filtrat wird im Vakuum eingeengt und der Rückstand mittels RP-HPLC gereinigt.

### Beispiele 2.1 - 2.183

| **#** | **Edukt** | **Struktur** | **Masse [M+1]⁺** | **HPLC Rt [min] bzw. Smp. bzw.** ***DC:R**_{f}* |
|---|---|---|---|---|
| **2.1** | **I-9** | 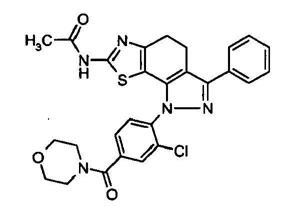 **¹H-NMR DMSO-d6, d [ppm]:** 7.86 (s, 1H), 7.78 (d, 7.9 Hz, 1H), 7.75 (d, 7.7 Hz, 1H), 7.63-7.61 (m, 1H), 7.49-7.46 (m, 2H), 7.41-7.37 (m, 1H), 3.72-3.62 (m, 6H), 3.42-3.38 (m, 2H), 2.07 (s, 3H)., | 534 | 2.06 |
| **2.5** | **I-9** | 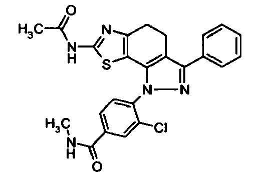 | 478 | Smp: 206 °C |
| **2.6** | **I-9** | 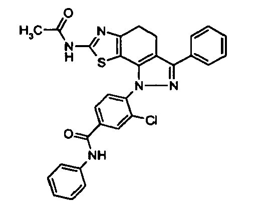 | 540 | R_{f}= 0.5 DCM:MeOH 9:1 |
| **2.7** | **I-9** | 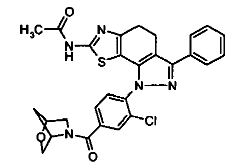 | 546 | 1.94 |
| **2.8** | **I-9** | 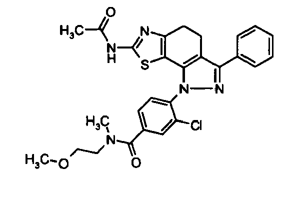 | 536 | 2.1 |
| **2.9** | **I-9** | 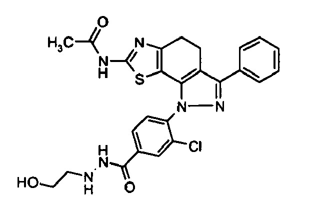 | 523 | 1.88 |
| **2.10** | **I-9** | 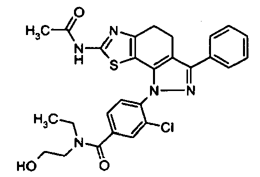 | 536 | 2.02 |
| **2.11** | **I-21** | 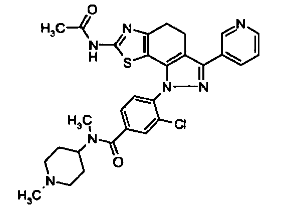 | 576 | 2.06 |
| **2.12** | **I-21** | 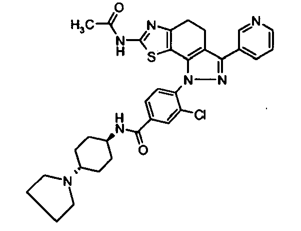 | 616 | 1.4 |
| **2.13** | **I-21** | 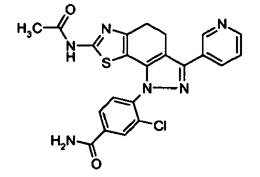 | 465 | 1.42 |
| **2.14** | **I-21** | 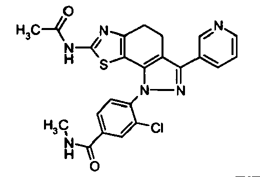 | 479 | 2.18 |
| **2.15** | **I-21** | 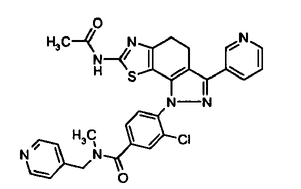 | 570 | 2.02 |
| **2.16** | **I-21** | 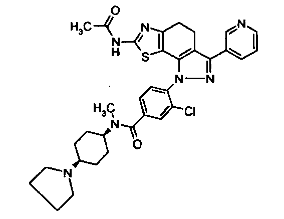 | 630 | 1.39 |
| **2.17** | **I-21** | 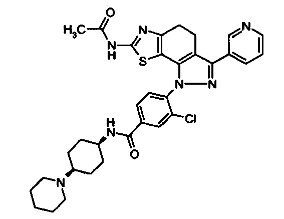 | 630 | 1.28 |
| **2.18*** | **1-21** | 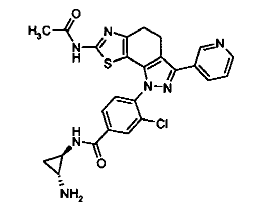 | 520 | 1.98 |
| **2.19** | **I-21** | 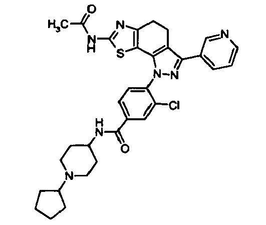 | 616 | 1.32 |
| **2.20** | **I-21** | 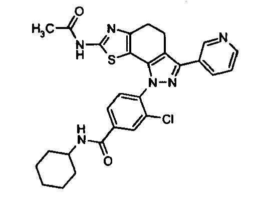 | 547 | 1.89 |
| **2.21** | **I-21** | 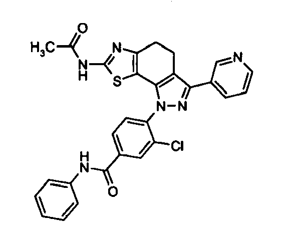 | 541 | 1.86 |
| **2.22** | **I-21** | 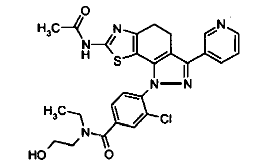 | 537 | 1.54 |
| **2.23** | **I-21** | 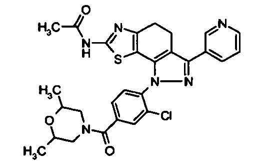 | 563 | 3.2 |
| **2.24** | **I-21** | 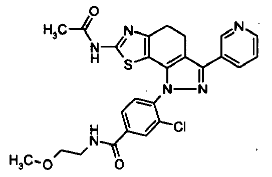 | 523 | 1.58 |
| **2.25** | **I-21** | 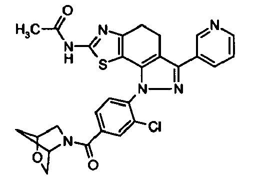 | 547 | 1.5 |
| **2.26** | **I-21** | 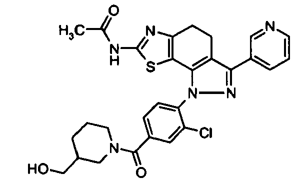 | 563 | 1.6 |
| **2.27** | **I-21** | 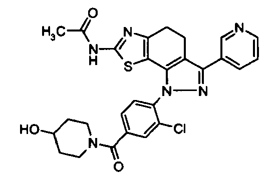 | 549 | 1.45 |
| **2.28** | **I-21** | 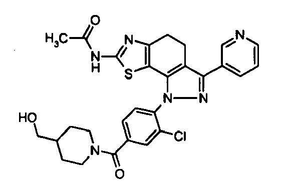 | 563 | 1.55 |
| **2.29** | **I-21** | 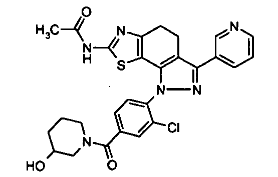 | 549 | 1.53 |
| **2.30** | **I-21** | 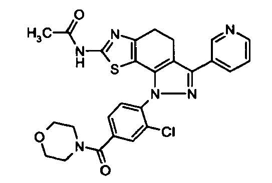 | **535** | 1.58 |
| **2.31** | **I-21** | 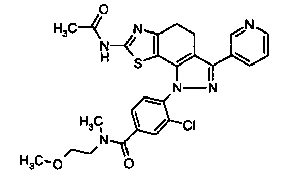 | **537** | 1.58 |
| **2.32** | **I-21** | 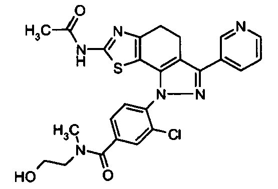 | 523 | 1.47 |
| **2.33** | **I-21** | 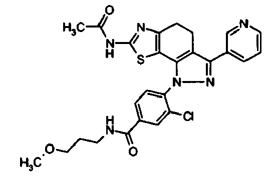 | 537 | 1.58 |
| **2.34** | **I-21** | 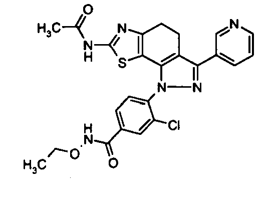 | 509 | 1.5 |
| **2.35** | **I-21** | 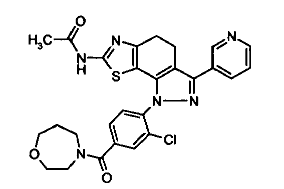 | 549 | 1.55 |
| **2.36** | **I-21** | 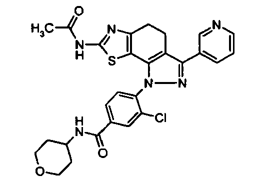 | 549 | 1.59 |
| **2.37** | **I-21** | 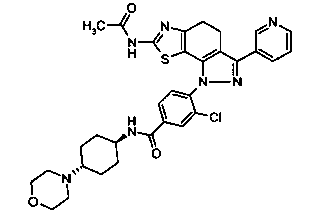 | 632 | 0.55 |
| **2.38** | **I-21** | 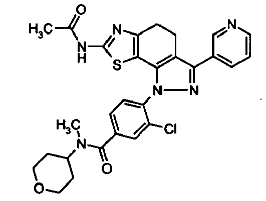 | 563 | 1.53 |
| **2.39** | **I-21** | 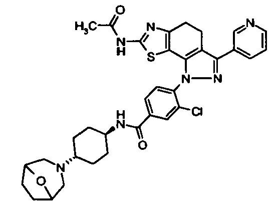 | 658 | 0.52 |
| **2.40** | **I-21** | 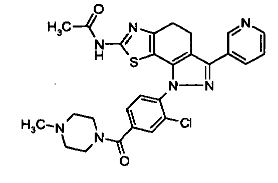 | 548 | 2.53 |
| **2.41** | **I-21** | 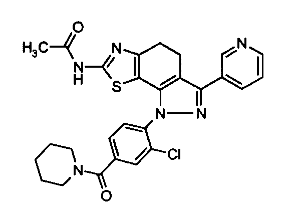 | 533 | 1.76 |
| **2.42** | **I-21** | 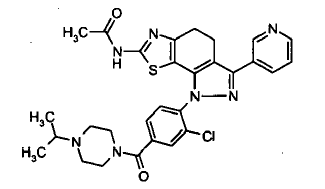 | 576 | 2.57 |
| **2.43** | **I-21** | 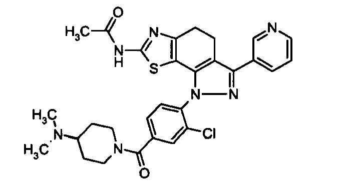 | 576 | 2.56 |
| **2.44** | **I-21** | 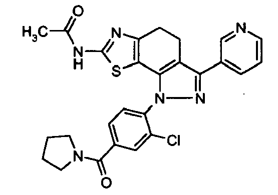 | 519 | 1.62 |
| **2.45** | **I-21** | 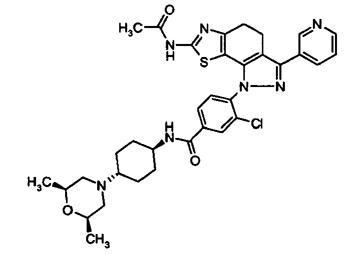 | 660 | 1.39 |
| **2.46** | **1-21** | 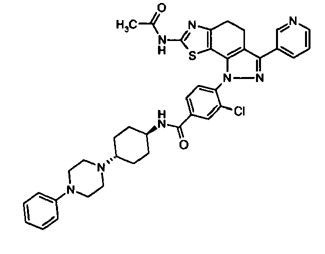 | 707 | 1.5 |
| **2.47** | **I-21** | 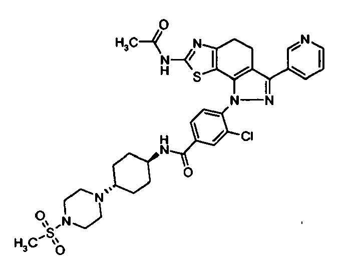 | 709 | 2.75 |
| **2.48** | **I-21** | 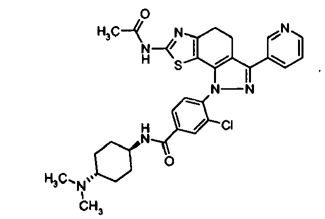 | 590 | 2.69 |
| **2.49** | **I-21** | 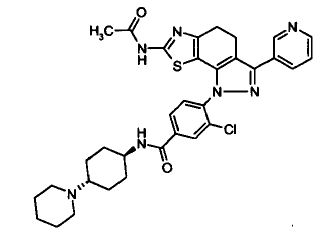 | 630 | 2.77 |
| **2.50** | **I-21** | 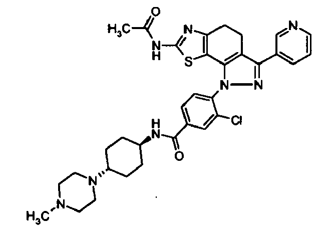 | 645 | 2.56 |
| **2.51** | **I-20** | 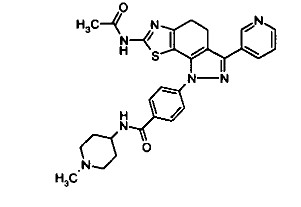 | 528 | 2 |
| **2.52** | **I-20** | 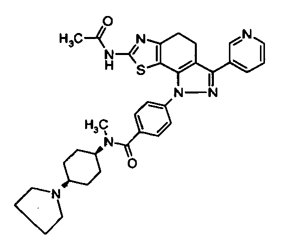 | 596 | 2.11 |
| **2.53** | **I-20** | 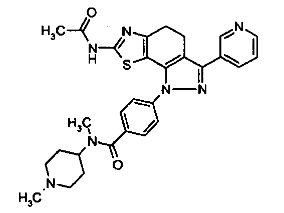 | 542 | 2.06 |
| **2.54** | **I-20** | 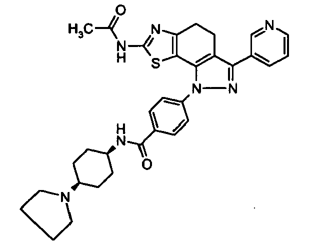 | 582 | 2.1 |
| **2.55** | **I-20** | 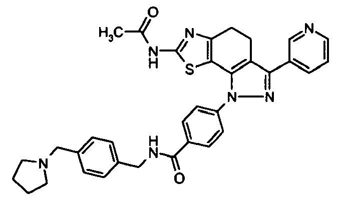 | 604 | 1.26 |
| **2.56** | **I-20** | 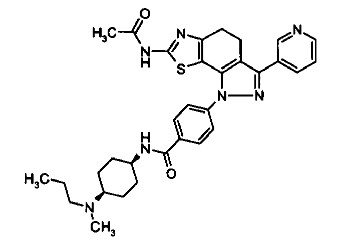 | 584 | 1.33 |
| **2.57** | **I-20** | 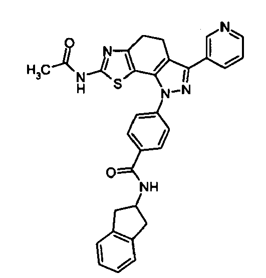 | 547 | 1,88 |
| **2.58** | **I-20** | 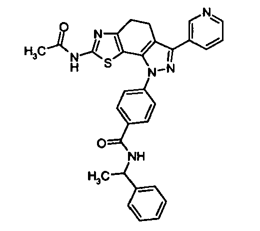 | 535 | 1,84 |
| **2.59** | **I-20** | 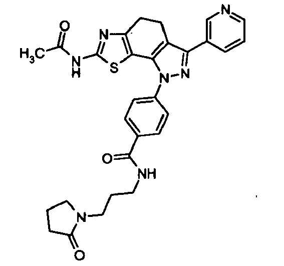 | 556 | 1.5 |
| **2.60** | **I-20** | 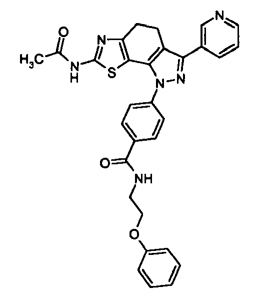 | 551 | 1.83 |
| **2.61** | **I-22** | 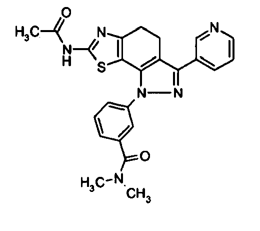 | 459 | 1.43 |
| **2.62** | **I-23** | 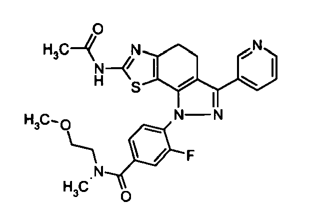 | 521 | 1.54 |
| **2.63** | **I-23** | 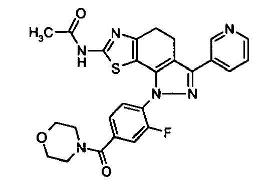 | 519 | 1.49 |
| **2.64** | **I-23** | 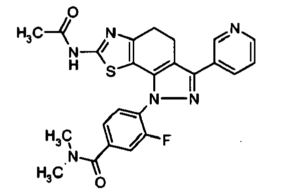 | 477 | 1.43 |
| **2.65** | **I-23** | 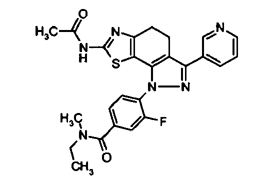 | 491 | 1.59 |
| **2.66** | **I-23** | 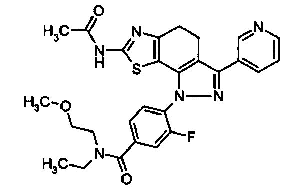 | 535 | 1.63 |
| **2.67** | **I-23** | 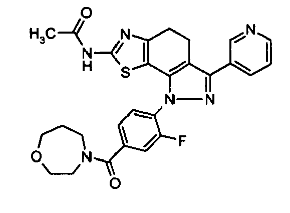 | 533 | 1.54 |
| **2.68** | **I-25** | 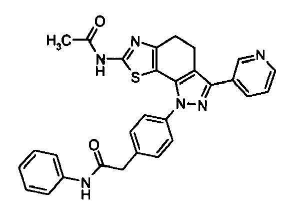 | 521 | 1.75 |
| **2.69** | **I-25** | 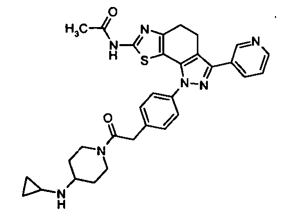 | 568 | 0.19 |
| **2.70** | **I-25** | 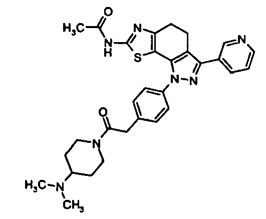 | 556 | Rf: 0.14 DCM:MeOH 7:3 |
| **2.71** | **I-25** | 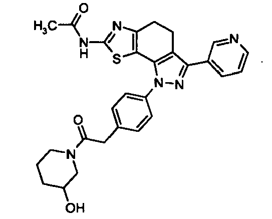 | 529 | 1.5 |
| **2.72** | **I-25** | 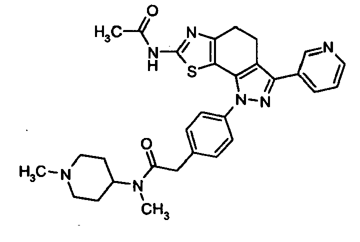 | 556 | R_{f}= 0.18 DCM:MeOH 7:3 |
| **2.73** | **I-25** | 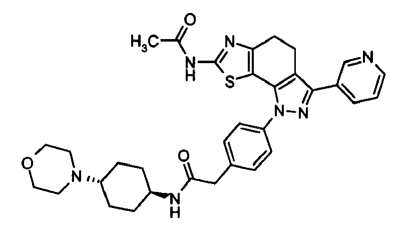 | 612 | R_{f}=0.27 DCM:MeOH 8:2 |
| **2.74** | **I-25** | 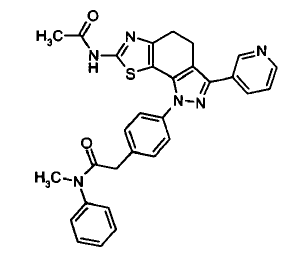 | 535 | 1.81 |
| **2.75** | **I-25** | 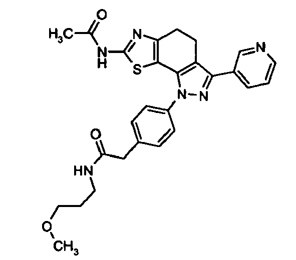 | 517 | 1.53 |
| **2.76** | **I-25** | 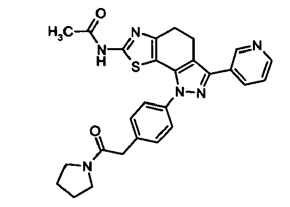 | 499 | 1.6 |
| **2.77** | **I-25** | 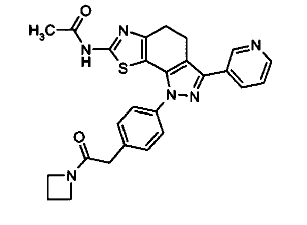 | 485 | 1.48 |
| **2.78** | **I-25** | 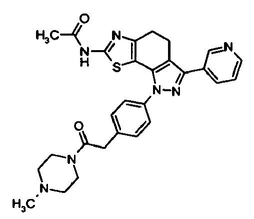 | 528 | 2.8 |
| **2.79** | **I-25** | 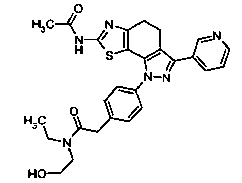 | 517 | 1.51 |
| **2.80** | **I-25 EtOH** | 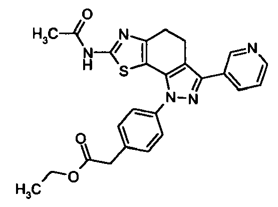 | 474 | 3.38 |
| **2.81** | **I-26** | 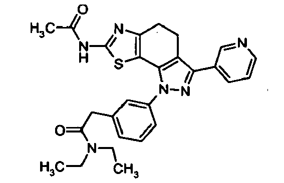 | 501 | 1.64 |
| **2.82** | **I-27** | 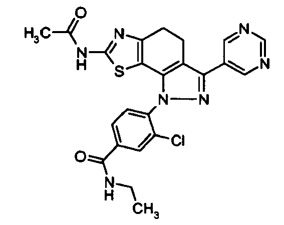 | 494 | 1.74 |
| **2.83** | **I-28** | 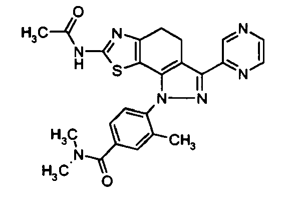 | 474 | Smp: 283 °C |
| **2.84** | **I-45** | 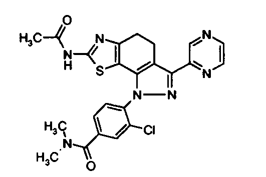 | 494 | 1.81 |
| **2.85** | **I-12** | 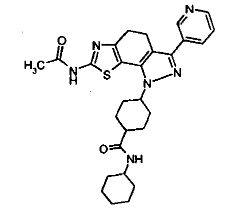 | 519 | 1.77 |
| **2.86** | **I-12** | 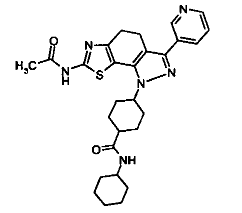 | 507 | 1.47 |
| **2.87** | **I-12** | 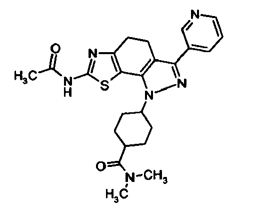 | 465 | 1.39 |
| **2.88** | **I-36** | 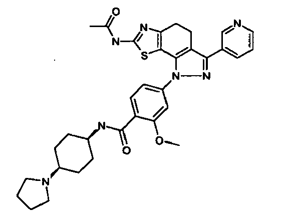 | 612 | 1.35 |
| **2.89** | **I-36** | 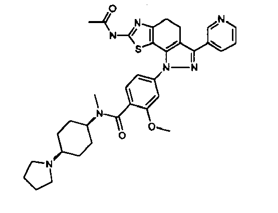 | 626 | 1.33 |
| **2.90** | **I-36** | 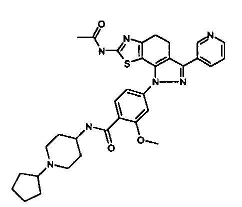 | 612 | 1.38 |
| **2.91** | **I-36** | 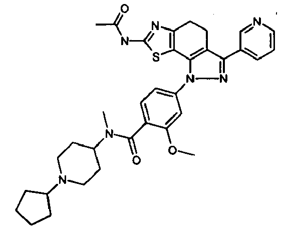 | 626 | 1.36 |
| **2.92** | **I-36** | 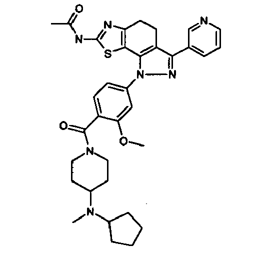 | 626 | 1.37 |
| **2.93** | **I-37** | 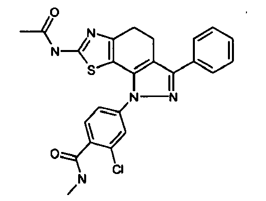 | 478 | 2.00 |
| **2.94** | **I-37** | 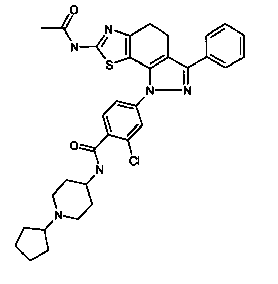 | 615 | 1.67 |
| **2.95** | **I-37** | 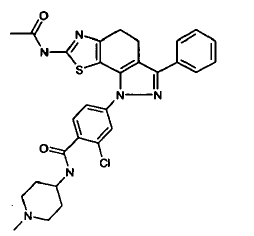 | 561 | 1.60 |
| **2.96** | **I-37** | 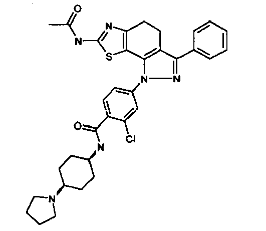 | 615 | 1.65 |
| **2.97** | **I-37** | 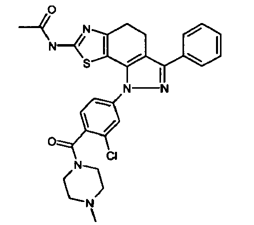 | 547 | 1.60 |
| **2.98** | **I-37** | 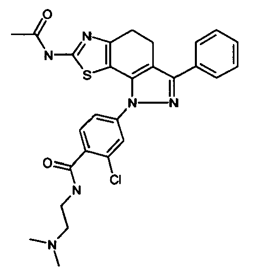 | 535 | 1.60 |
| **2.99** | **I-9** | 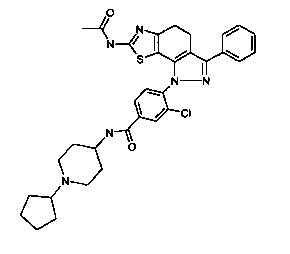 | 615 | Smp:280°C |
| **2.100** | **I-9** | 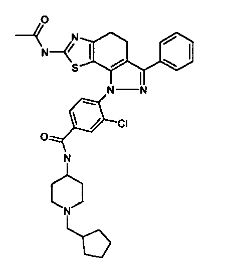 | 629 | Smp: 286 °C |
| **2.101** | **I-12** | 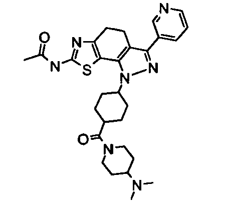 | 548 | 1.45 |
| **2.102** | **I-12** | 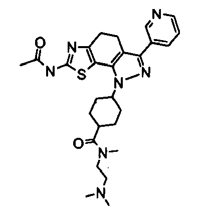 | 522 | 1.46 |
| **2.103** | ***trans-*I-12** | 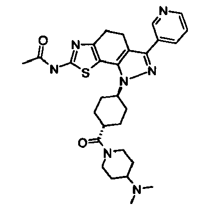 | 548 | 1.43 |
| **2.104** | ***trans-*I-12** | 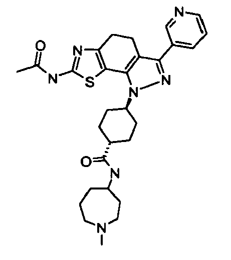 | 548 | 1.43 |
| **2.105** | ***trans-*I-12** | 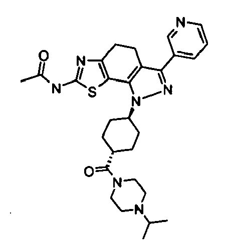 | 548 | 1.46 |
| **2.106** | ***trans-*I-12** | 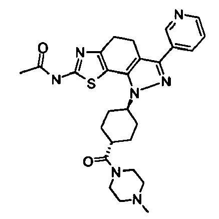 | 520 | 1.37 |
| **2.107** | ***trans-*I-12** | 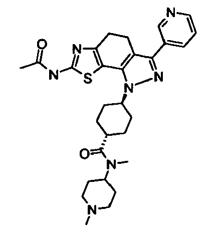 | 548 | 1.45 |
| **2.108** | ***trans-*I-12** | 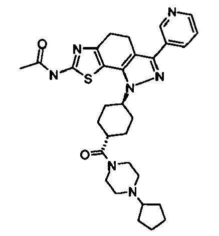 | 574 | 1.54 |
| **2.109** | **I-23** | 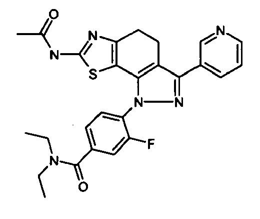 | 505 | 1.6 |
| **2.110** | **I-23** | 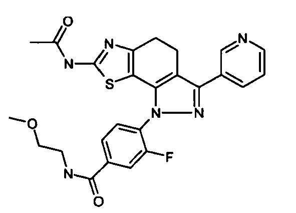 | 507 | 1.41 |
| **2.111** | **I-23** | 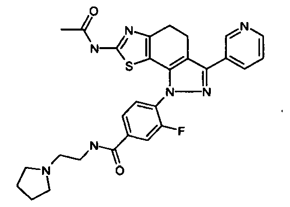 | 546 | 1.52 |
| **2.112** | **I-23** | 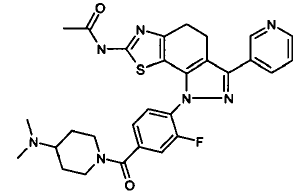 | 560 | 1.24 |
| **2.113** | **I-23** | 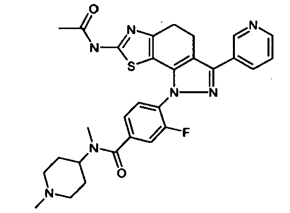 | 560 | 1.48 |
| **2.115** | **I-23** | 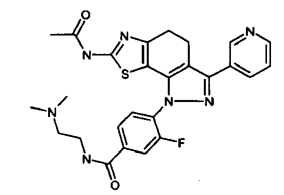 | 520 | 1.45 |
| **2.116** | **I-23** | 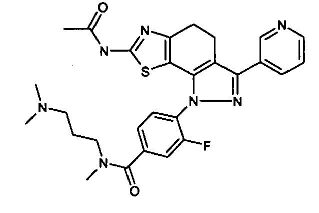 | 548 | 1.49 |
| **2.117** | **I-23** | 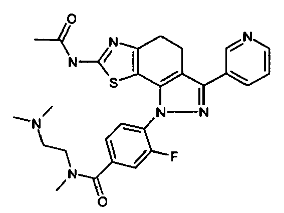 | 534 | 1.48 |
| **2.118** | **I-23** | 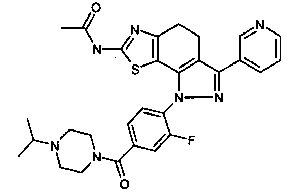 | 560 | 1.53 |
| **2.119** | **I-23** | 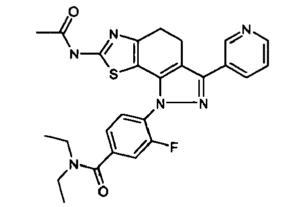 | 505 | 1.6 |
| **2.120** | **I-23** | 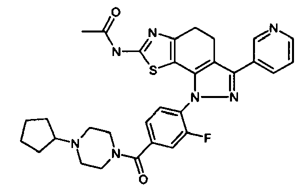 | 586 | 1.61 |
| **2.121** | **I-23** | 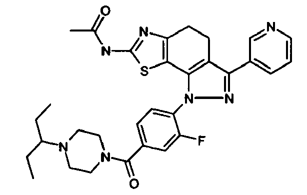 | 588 | 1.64 |
| **2.122** | **I-23** | 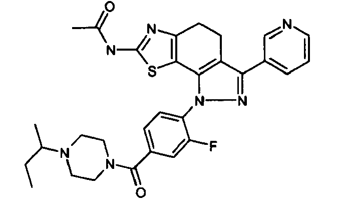 | 574 | 1.27 |
| **2.123** | **I-23** | 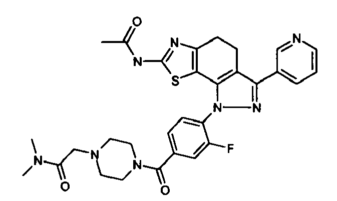 | 603 | 1.49 |
| **2.124** | **I-23** | 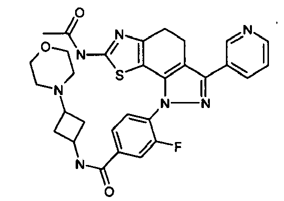 | 588 | 1.28 |
| **2.125** | **I-23** | 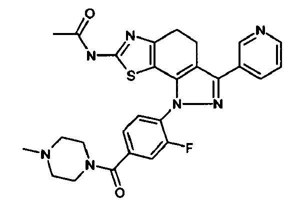 | 532 | 1.65 |
| **2.126** | **I-25** | 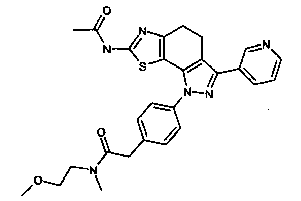 | 517 | R_{f} = 0.44 DCM: MEOH 9:1 |
| **2.127** | **1-25** | 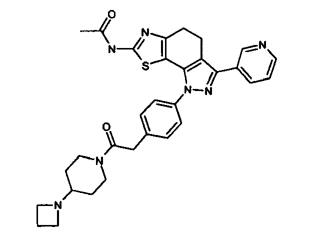 | 568 | R_{f}=0.18 DCM: MEOH 7:3 |
| **2.128** | **I-25** | 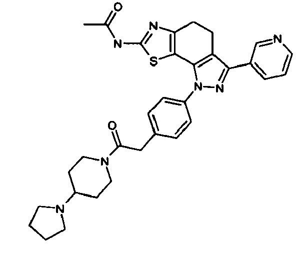 | 582 | R_{f} = 0.24 DCM: MEOH 7:3 |
| **2.129** | **I-25** | 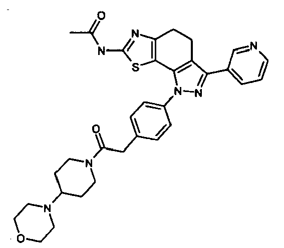 | 598 | 1.23 |
| **2.130** | **I-25** | 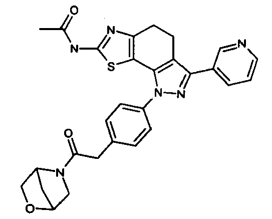 | 527 | 1.39 |
| **2.131** | **I-25** | 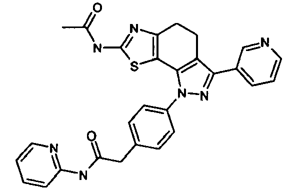 | 522 | 1.43 |
| **2.132** | **I-25** | 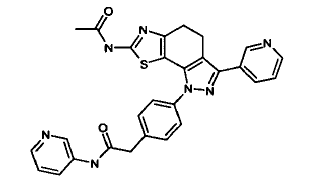 | 522 | R_{f} = 0.91 DCM: MEOH 9:1 |
| **2.133** | **I-25** | 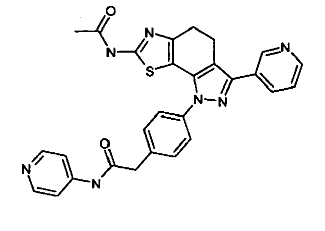 | 522 | R_{f} = 0.88 DCM: MEOH 9:1 |
| **2.134** | **I-25** | 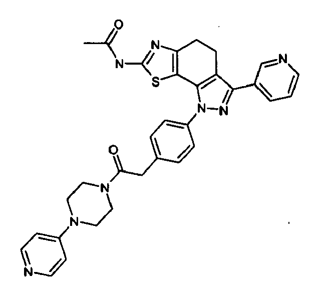 | 591 | R_{f} = 0.42 DCM: MEOH 9:1 |
| **2.135** | **I-25** | 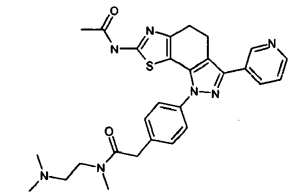 | 530 | R_{f}=0.4 DCM: MEOH 9:1 |
| **2.136** | **I-25** | 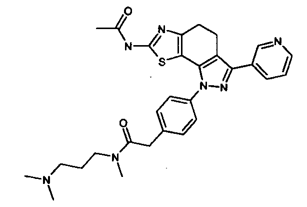 | 544 | R_{f}=0.25 DCM: MEOH 9:1 |
| **2.137** | **I-25** | 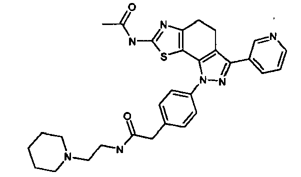 | 556 | 1.27 |
| **2.138** | **I-25** | 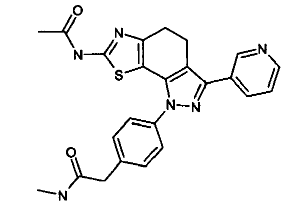 | 459 | R_{f} = 0.34 DCM: MEOH 9:1 |
| **2.139** | **I-25** | 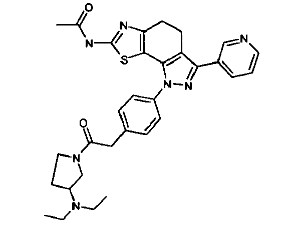 | 570 | R_{f}=0.18 DCM: MEOH 8:2 |
| **2.140** | **I-25** | 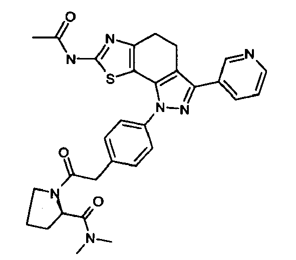 | 570 | 1.43 |
| **2.141** | **I-20** | 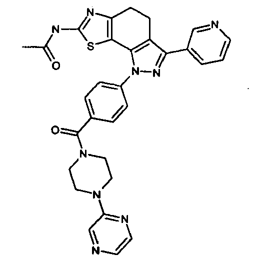 | 578 | 1.56 |
| **2.142** | **I-20** | 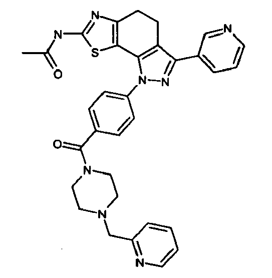 | 591 | 1.53 |
| **2.143** | **I-20** | 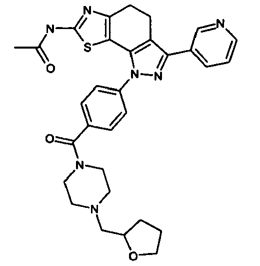 | 584 | 1.53 |
| **2.144** | **I-20** | 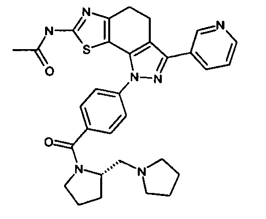 | 568 | 1.61 |
| **2.145** | **I-20** | 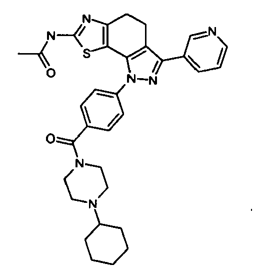 | 582 | 1.64 |
| **2.146** | **I-20** | 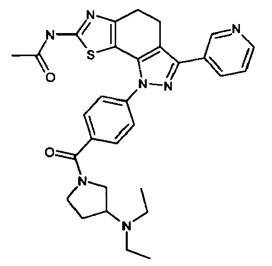 | 556 | 1.50 |
| **2.147** | **I-20** | 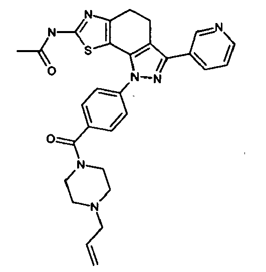 | 540 | 1.48 |
| **2.148** | **I-20** | 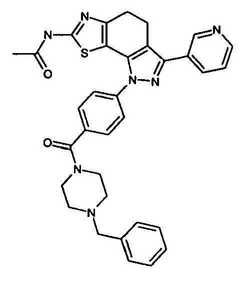 | 590 | 1.65 |
| **2.149** | **I-20** | 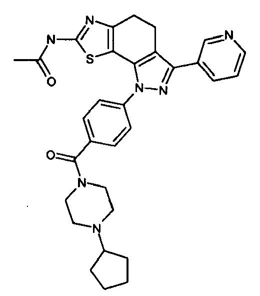 | 568 | 1.58 |
| **2.150** | **I-20** | 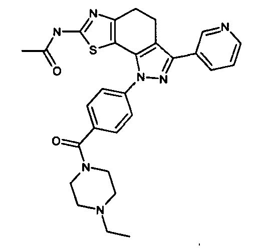 | 528 | 1.44 |
| **2.151** | **I-20** | 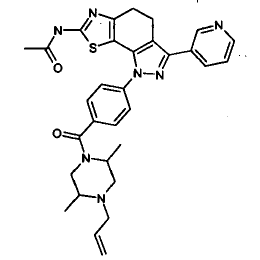 | 568 | 1.54 |
| **2.152** | **I-20** | 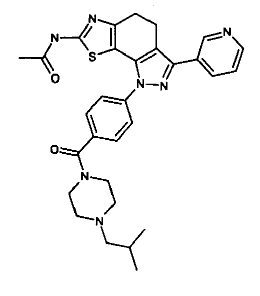 | 556 | 1.29 |
| **2.153** | **I-20** | 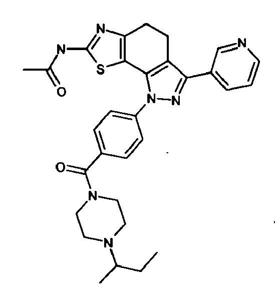 | 556 | 1.55 |
| **2.154** | **I-21** | 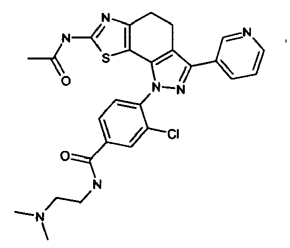 | 536 | 1.48 |
| **2.155** | **I-21** | 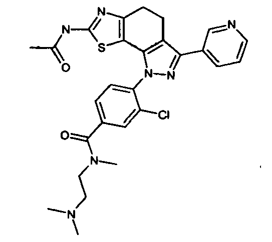 | 550 | 1.49 |
| **2.156** | **I-21** | 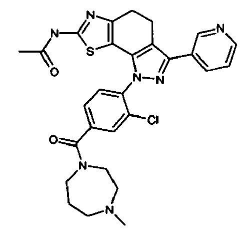 | 562 | 1.49 |
| **2.157** | **I-21** | 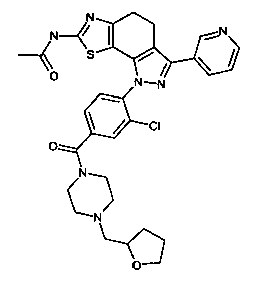 | 618 | 1.58 |
| **2.158** | **I-21** | 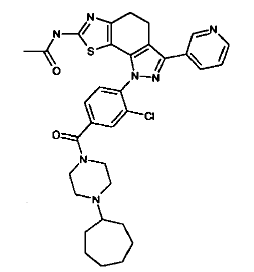 | 630 | 1.50 |
| **2.159** | **I-21** | 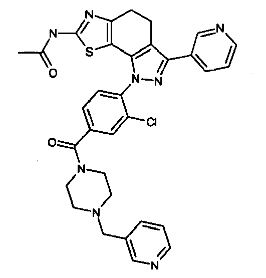 | 625 | 1.55 |
| **2.160** | **I-21** | 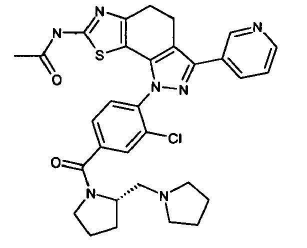 | 602 | 1.65 |
| **2.161** | **I-21** | 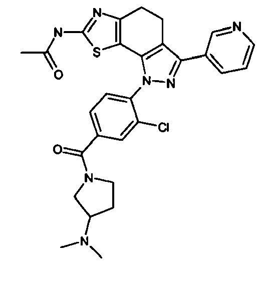 | 562 | 1.47 |
| **2.162** | **I-21** | 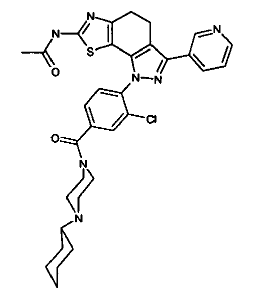 | 616 | 1.72 |
| **2.163** | **I-21** | 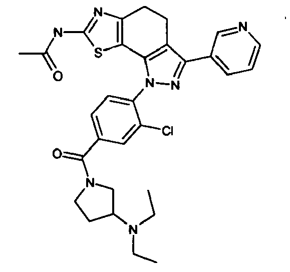 | 590 | 1.25 |
| **2.164** | **I-21** | 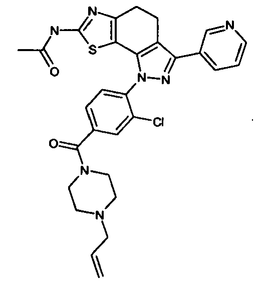 | 574 | 1.55 |
| **2.165** | **I-21** | 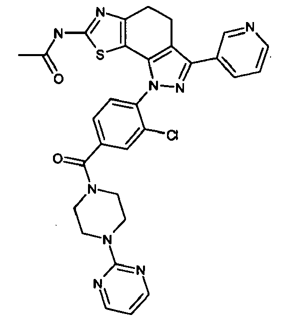 | 612 | 1.64 |
| **2.166** | **I-21** | 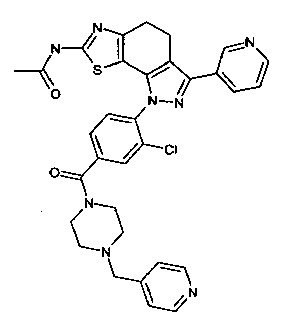 | 625 | 1.58 |
| **2.167** | **I-21** | 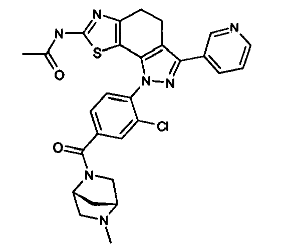 | 560 | 1.46 |
| **2.168** | **I-21** | 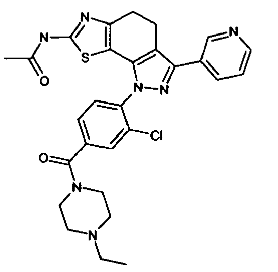 | 562 | 1.77 |
| **2.169** | **I-21** | 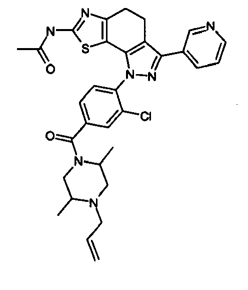 | 602 | 1.62 |
| **2.170** | **I-21** | 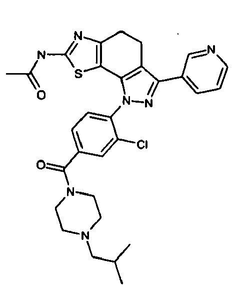 | 590 | 1.62 |
| **2.171** | **I-21** | 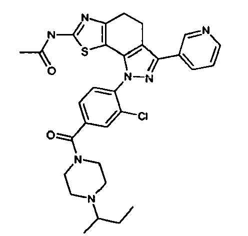 | 590 | 1.61 |
| **2.172** | **I-21** | 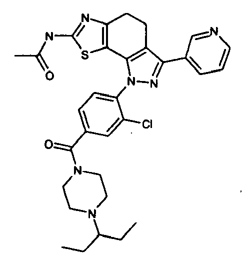 | 604 | 1.68 |
| **2.173** | **I-21** | 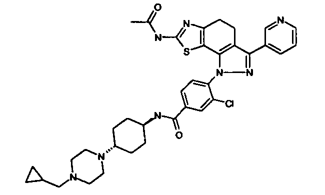 | 685 | 2.59 |
| **2.174** | **I-20** | 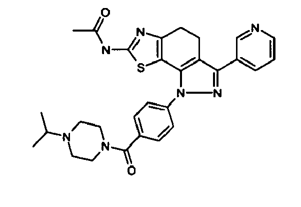 | 542 | 2.56 |
| **2.176** | **I-41** | 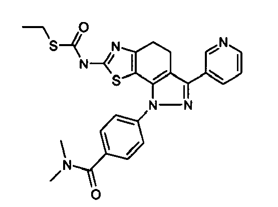 | 505 | |
| **2.177** | **I-42** | 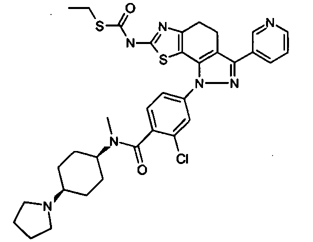 | 676 | |
| **2.178** | **I-42** | 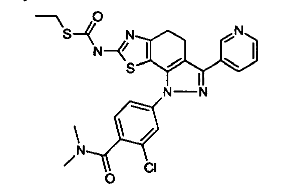 | 539 | |
| **2.179** | **I-12** | 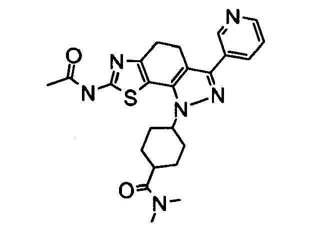 | 465 | 1.46 |
| **2.180** | **I-21** | 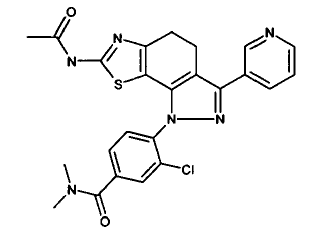 | 493 | 1.56 |
| **2.181** | **I-20** | 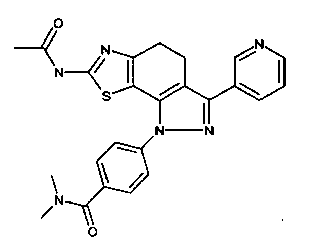 | 459 | 1.5 |
| **2.182** | **I-23** | 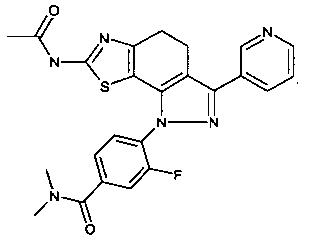 | 477 | 2.87 |
| **2.183** | **II-19** | 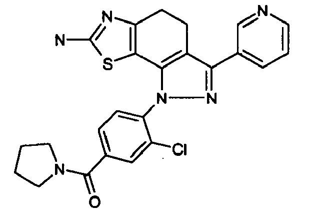 | 477 | 1.44 |

| | | | | |
|---|---|---|---|---|
| *Im Beispiel 2.18 erfolgt die Umsetzung der Carbonsäure mit (2-Amino-cyclopropyl)-carbamidsäure-*tert*-butylester. Im 2. Schritt erfolgt die Abspaltung der BOC-Schutzgruppe mit Trifluoressigsäure. | | | | |

### Beispiele 3 - Umsetzung der hergestellten Amine

### Synthesemethode E - Umsetzung mit Sulfonsäurechloriden

Eine Lösung von 0.2 mmol Amin in 3 mL Pyridin wird mit 0.5 mmol Sulfonsäurechlorid versetzt und 15 h bei RT gerührt. Das Reaktionsgemisch wird eingeengt und der Rückstand chromatographisch gereinigt.

### Synthesemethode F - Umsetzung mit Carbonsäuren

Eine Lösung der Carbonsäure (0.16 mmol) in 1.3 mL DMF wird mit HATU (0.55 mmol) und Diisopropylethylamin (1.8 mmol) versetzt und 1h bei RT gerührt. Nach Zugabe einer Lösung von 0.1 mmol des entsprechenden Amins in DMF wird weitere 15 h bei RT gerührt. Anschließend wird die Reaktionsmischung filtriert, eingeengt und der Rückstand chromatographisch gereinigt.

### Synthesemethode G - Umsetzung mit Carbonsäurechloriden

Eine Lösung von 0.2 mmol Amin in 3 mL Pyridin wird mit 0.5 mmol Carbonsäurechlorid versetzt und 15 bei RT gerührt. Das Reaktionsgemisch wird eingeengt und der Rückstand chromatographisch gereinigt.

### Beispiele 3.2 - 3.82

| **#** | **Edukt** | **Struktur** | **Masse [M+1]⁺** | **HPLC Rt [min] bzw. Smp. bzw. *DC:R_{f}*** |
|---|---|---|---|---|
| **3.2** | **II-1** | 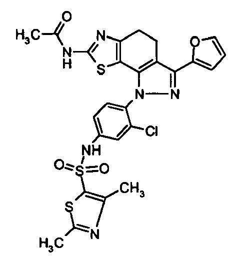 | 601 | 2.08 |
| **3.3** | **II-3** | 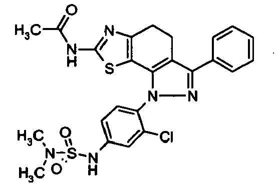 | 543 | 2.2 |
| **3.4** | **II-1** | 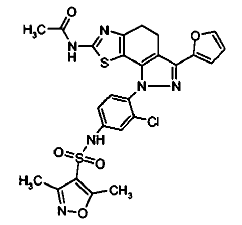 | 585 | 2.12 |
| **3.10** | **II-4** | 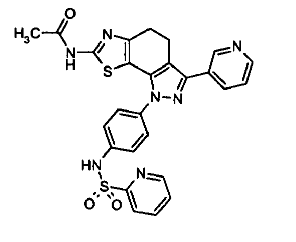 | 544 | 1.62 |
| **3.11** | **II-4** | 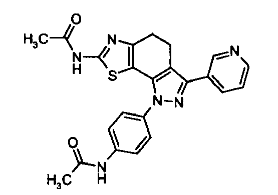 | 445 | 1.45 |
| **3.12** | **II-4** | 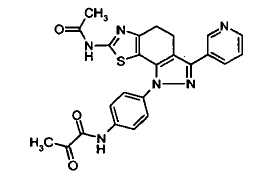 | 473 | 1.6 |
| **3.13** | **II-4** | 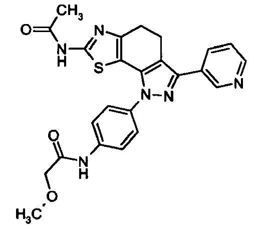 | 475 | 1.48 |
| **3.14** | **II-4** | 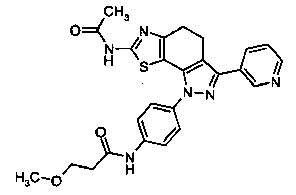 | 489 | 1.5 |
| **3.15** | **II-4** | 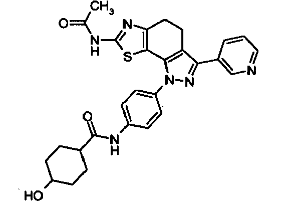 | 529 | 1.53 |
| **3.16** | **II-4** | 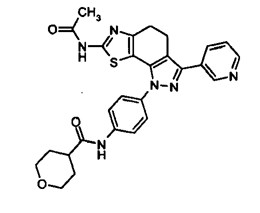 | 515 | 1.52 |
| **3.17** | **II-4** | 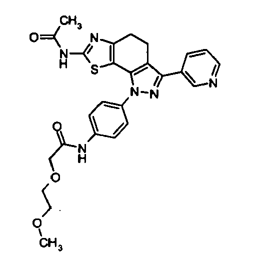 | 519 | 1.58 |
| **3.18** | **II-4** | 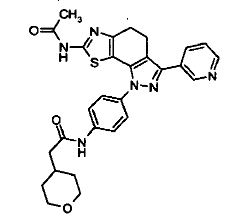 | 529 | 1.59 |
| **3.19** | **II-4** | 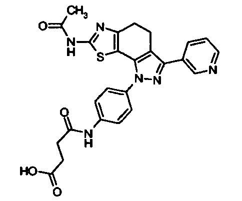 | 503 | 1.43 |
| **3.20** | **II-4** | 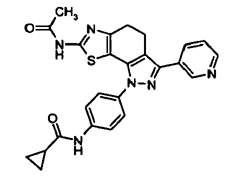 | 471 | 1.6 |
| **3.21** | **II-4** | 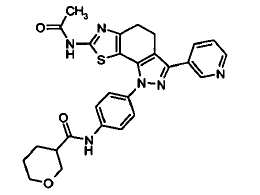 | 515 | 1.57 |
| **3.22** | **II-4** | 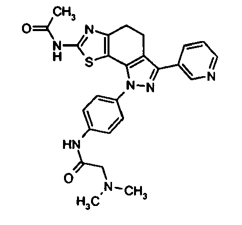 | 488 | 0.47 |
| **3.23** | **II-5** | 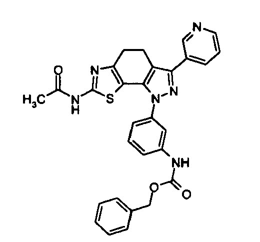 | 537 | 1.89 |
| **3.24** | **II-5** | 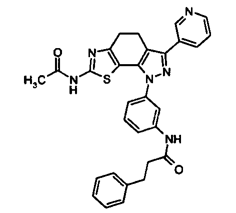 | 535 | 1.81 |
| **3.25** | **II-6** | 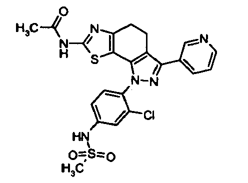 | 515 | 1.54 |
| **3.26** | **II-6** | 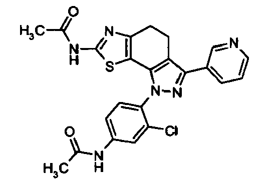 | 479 | 1.52 |
| **3.27** | **II-6** | 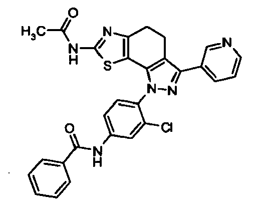 | 541 | 1.82 |
| **3.28** | **II-6** | 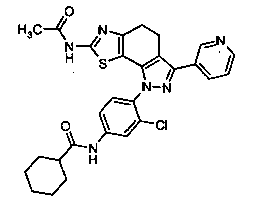 | 547 | 1.90 |
| **3.29** | **II-6** | 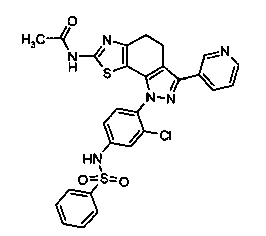 | 577 | 1.81 |
| **3.30** | **II-6** | 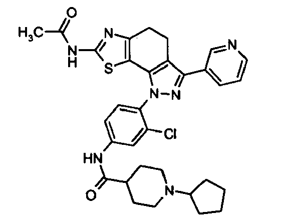 | 616 | 1.38 |
| **3.31** | **II-6** | 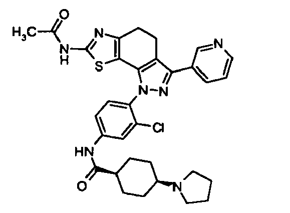 | 616 | Rf: 0.18 MeOH:NH₄OH 92,5:7,5:1 |
| **3.32** | **II-6** | 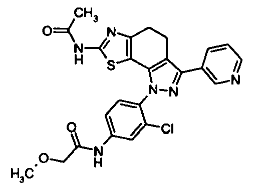 | 509 | 1.43 |
| **3.33** | **II-6** | 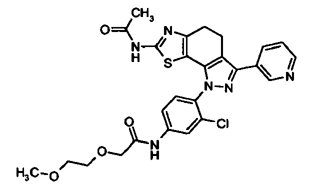 | 553 | 1.55 |
| **3.34** | **II-12** | 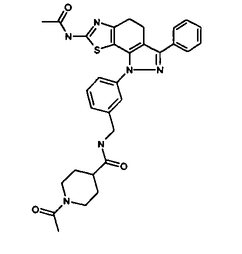 | 569 | Smp: 277 °C |
| **3.35** | **II-12** | 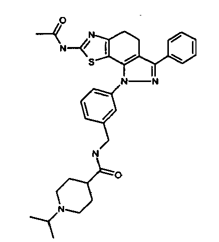 | 569 | 2.59 |
| **3.36** | **II-12** | 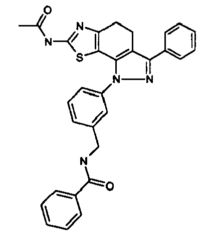 | 520 | Smp: 282 °C |
| **3.37** | **II-12** | 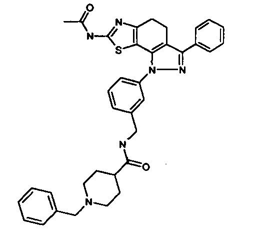 | 617 | Smp: 227 °C |
| **3.38** | **II-12** | 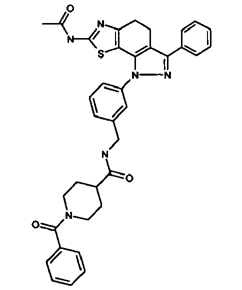 | 631 | Smp: 294 °C |
| **3.39** | **II-12** | 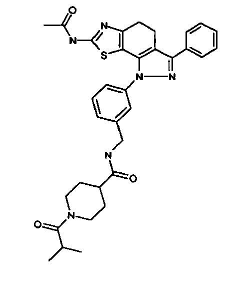 | 597 | 3.0 |
| **3.40** | **II-12** | 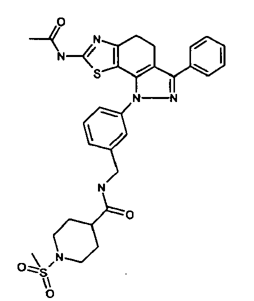 | 605 | 2.95 |
| **3.41** | **I-11** | 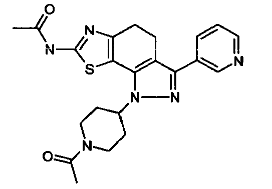 | 437 | 1.28 |
| **3.42** | **I-11** | 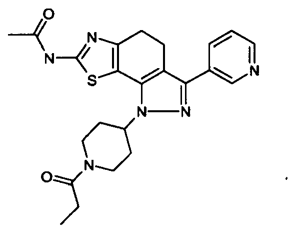 | 451 | 1.4 |
| **3.43** | **I-11** | 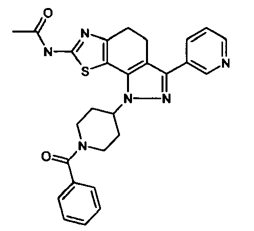 | 499 | 1.56 |
| **3.44** | **I-11** | 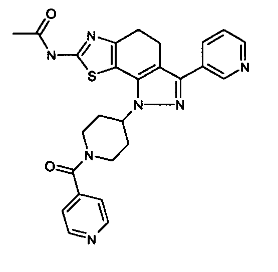 | 500 | 1.21 |
| **3.45** | **I-11** | 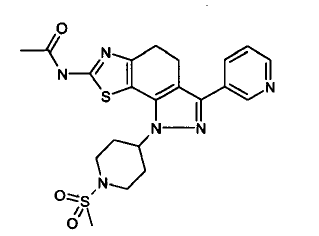 | 473 | 1.38 |
| **3.46** | **I-11** | 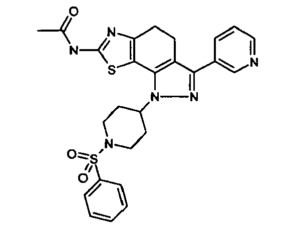 | 535 | 1.68 |
| **3.47** | **I-11** | 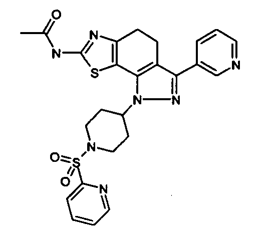 | 536 | 1.52 |
| **3.49** | **I-40** | 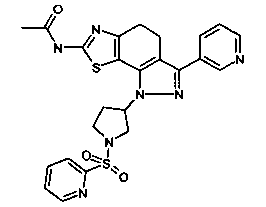 | 522 | 1.47 |
| **3.50** | **I-11** | 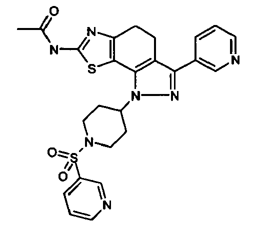 | 536 | 1.52 |
| **3.51** | **I-11** | 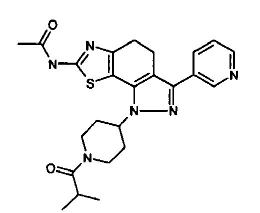 | 465 | 1.47 |
| **3.52** | **I-11** | 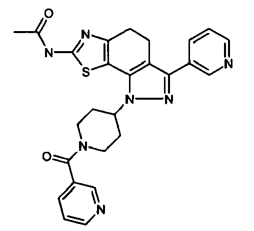 | 500 | 1.34 |
| **3.53** | **I-11** | 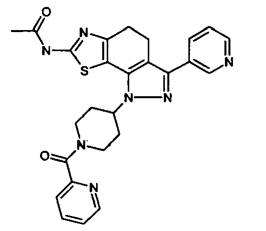 | 500 | 1.46 |
| **3.54** | **II-13** | 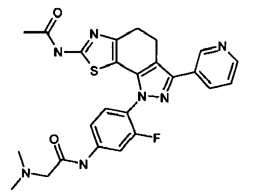 | 506 | 1.47 |
| **3.55** | **II-13** | 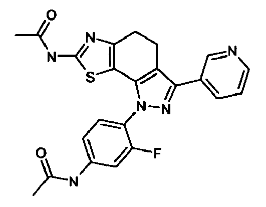 | 463 | 1.46 |
| **3.56** | **II-4** | 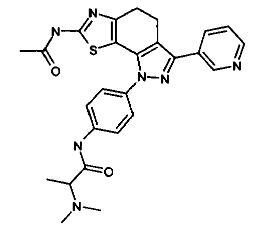 | 502 | 1.45 |
| **3.57** | **II-4** | 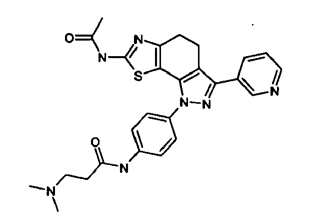 | 502 | 1.45 |
| **3.58** | **II-14** | 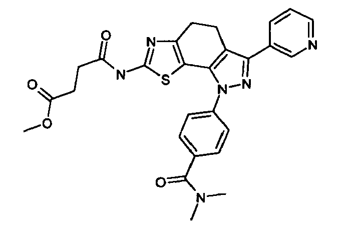 | 531 | 1.48 |
| **3.59** | **II-14** | 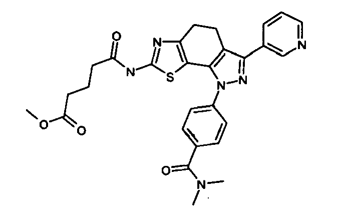 | 545 | 1.56 |
| **3.60** | **II-14** | 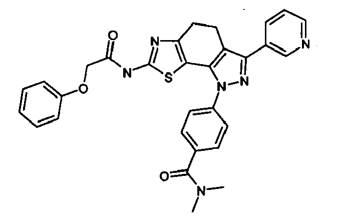 | 551 | 1.77 |
| **3.61** | **II-14** | 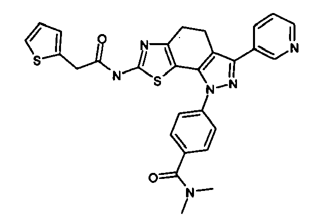 | 541 | 1.67 |
| **3.62** | **II-14** | 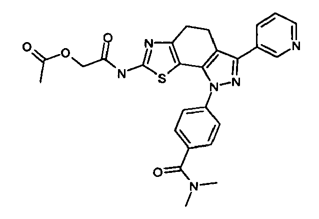 | 517 | 1.46 |
| **3.63** | **II-14** | 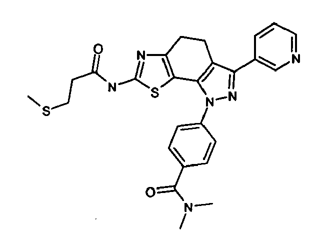 | 519 | 1.57 |
| **3.64** | **II-14** | 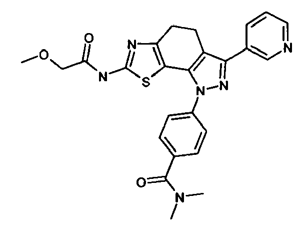 | 489 | 1.45 |
| **3.65** | **II-15** | 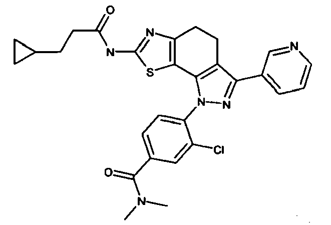 | 547 | 1.74 |
| **3.66** | **II-16** | 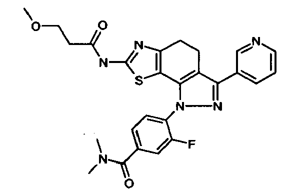 | 521 | 1.46 |
| **3.67** | **II-16** | 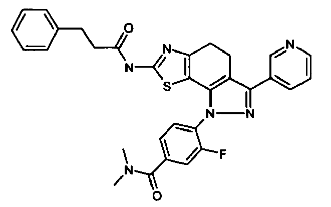 | 567 | 1.77 |
| **3.68** | **II-17** | 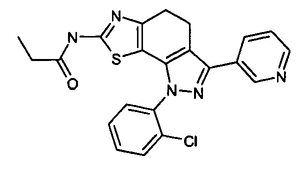 | 436 | 1.64 |
| **3.69** | **II-17** | 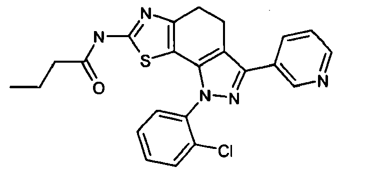 | 450 | 1.75 |
| **3.70** | **II-17** | 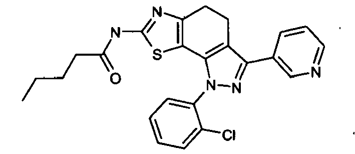 | 464 | 1.87 |
| **3.71** | **II-17** | 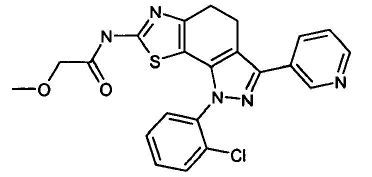 | 452 | 1.59 |
| **3.72** | **II-17** | 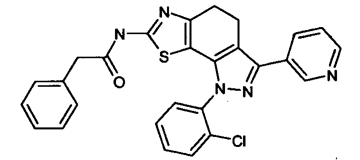 | 498 | 1.88 |
| **3.73** | **II-17** | 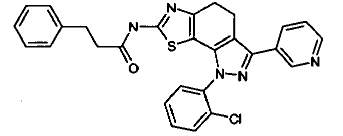 | 512 | 1.95 |
| **3.74** | **II-17** | 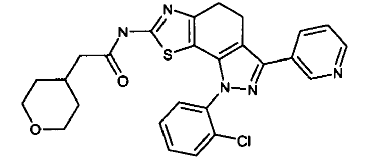 | 506 | 1.68 |
| **3.75** | **II-17** | 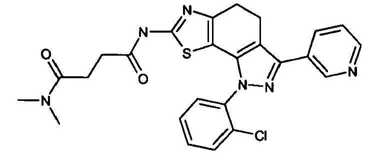 | 507 | 1.57 |
| **3.76** | **II-17** | 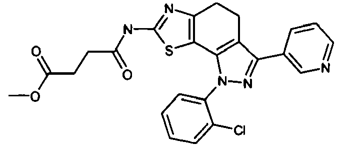 | 494 | 1.64 |
| **3.77** | **II-17** | 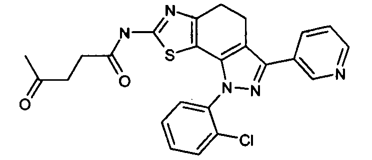 | 478 | 1.58 |
| **3.78** | **II-17** | 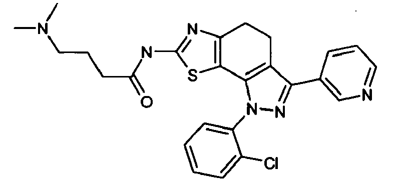 | 493 | 1.28 |
| **3.79** | **II-17** | 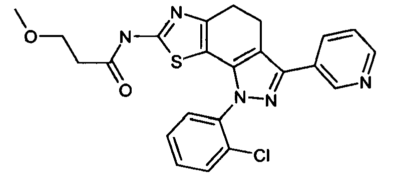 | 466 | 1.61 |
| **3.80** | **II-18** | 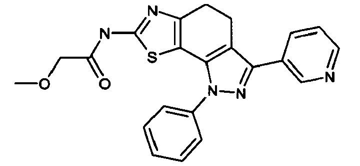 | 418 | 1.54 |
| **3.81** | **II-18** | 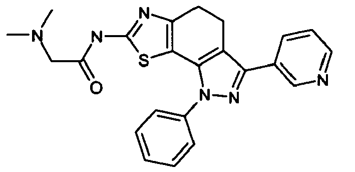 | 431 | 1.53 |
| **3.82** | **II-18** | 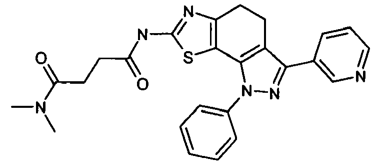 | 473 | 1.55 |

| | | | | |
|---|---|---|---|---|
| * Die Synthese von 3.56 erfolgt durch Umsetzung von II-4 mit 2-Brompropionylbromid und anschließender nukleophiler Substitution mit Dimethylamin. | | | | |

### Beispiele 4 - Umsetzung der Thiocarbamate mit Aminen und Alkoholen

### Darstellung von Harnstoffen

Eine Lösung von 0.11 mmol Thiocarbamat wird in 5 mL Ethanol mit 0.17 mmol Amin und 30 µL Diisopropylethylamin versetzt und 15 h bei 80 °C in einem Druckrohr gerührt. Nach Entfernen des Lösungsmittels im Vakuum wird der Rückstand chromatographisch gereinigt.

### Darstellung von Carbamaten

Eine Lösung von 0.11 mmol Thiocarbamat (oder Methylcarbamat) wird mit 5 mL des entsprechenden Alkohols versetzt und 15 h bei 80 °C in einem Druckrohr gerührt. Nach Entfernen des Lösungsmittels im Vakuum wird der Rückstand chromatographisch gereinigt.

### Beispiele 4.1 - 4.32

| **#** | **Edukt** | **Struktur** | **Masse [M+1]⁺** | **HPLC Rt [min] bzw. Smp. bzw. *DC:R_{f}*** |
|---|---|---|---|---|
| **4.1** | **II-9** | 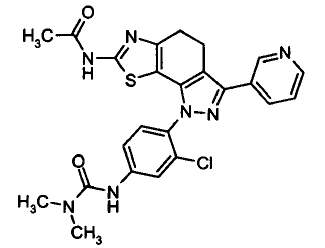 **¹H-NMR DMSO-d6, d [ppm]:** 8.93 (d, 1.7 Hz, 1H), 8.79 (s, 1H), 8.58 (dd, 1.5 und 4.8 Hz, 1H), 8.22 (s, 1H), 8.12-8.09 (m, 1H), 7.99 (d, 2.1 Hz, 1H), 7.70 (dd 2.3 und 8.8 Hz, 1H), 7.54-7.47 (m, 2H), 3.17-3.12 (m, 2H), 3.05-3.01 (m, 2H), 2.9 (s, 6H), 2.08 (s, 3H). | 508 | 1.43 |
| **4.2** | **II-9** | 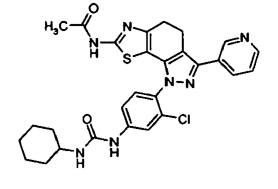 | 562 | 1.86 |
| **4.3** | **II-9** | 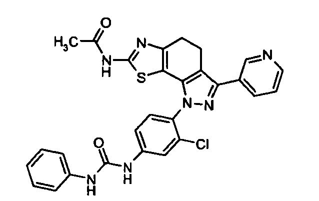 | 556 | 1.85 |
| **4.4** | **II-9** | 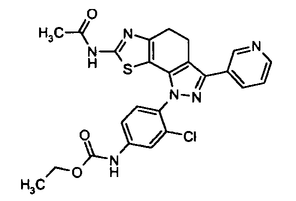 | 509 | 1.73 |
| **4.5** | **II-9** | 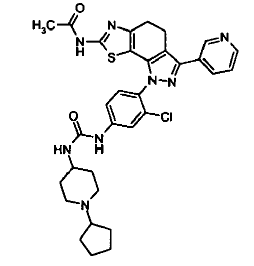 | 631 | 1.58 |
| **4.6** | **II-9** | 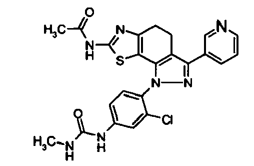 | 493 | 2.02 |
| **4.7** | **II-9** | 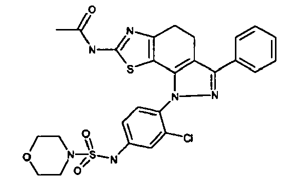 | 585 | Smp: 160 °C |
| **4.8** | **II-9** | 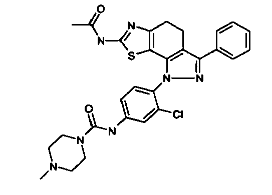 | 562 | 1.59 |
| **4.9** | **II-9** | 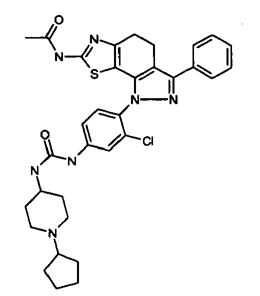 | 630 | 1.68 |
| **4.10** | **II-9** | 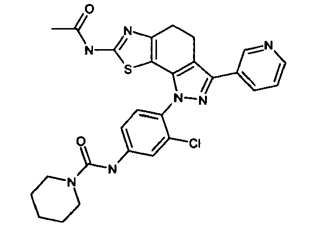 | 549 | 1.65 |
| **4.11** | **II-9** | 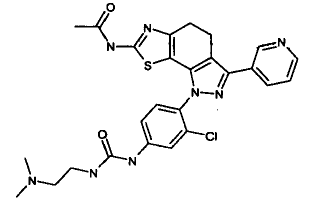 | 551 | R_{f}= 0.02 DCM:MeOH 9:1 |
| **4.12** | **II-9** | 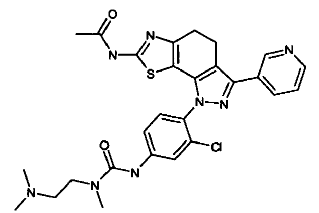 | 565 | R_{f}=0.05 DCM:MeOH 9:1 |
| **4.13** | **II-9** | 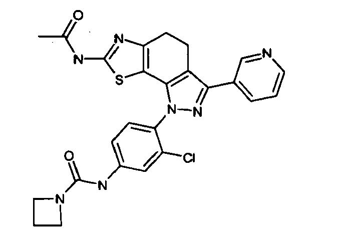 | 520 | 1.43 |
| **4.14** | **II-9** | 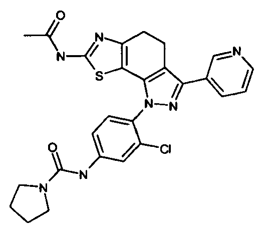 | 534 | 1.54 |
| **4.15** | **II-9** | 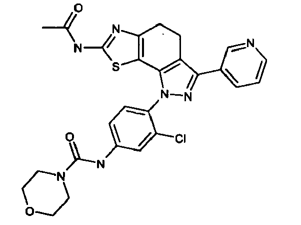 | 550 | 1.43 |
| **4.16** | **II-9** | 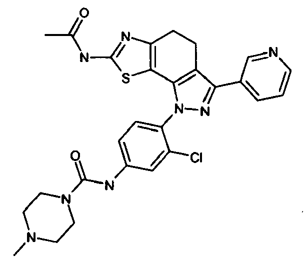 | 563 | 1.3 |
| **4.17** | **II-9** | 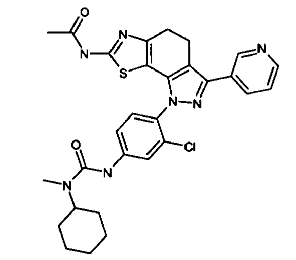 | 576 | 1.83 |
| **4.18** | **II-9** | 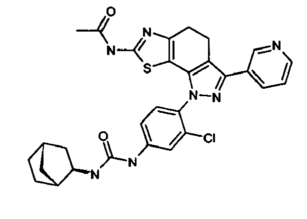 | 574 | 1.79 |
| **4.19** | **II-9** | 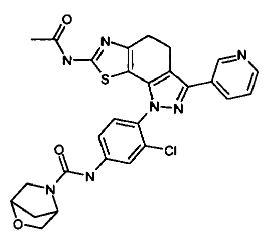 | 562 | 1.42 |
| **4.20** | **II-9** | 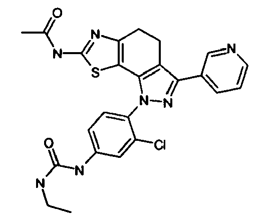 | 508 | 1.49 |
| **4.21** | **II-9** | 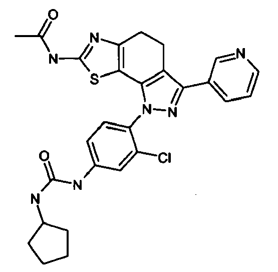 | 548 | 1.68 |
| **4.22** | **2.176** | 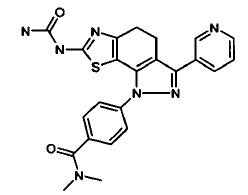 | 460 | 1.43 |
| **4.23** | **2.176** | 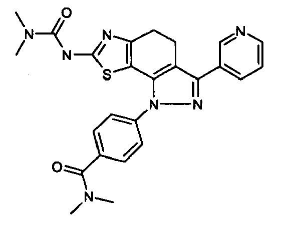 | 488 | 1.45 |
| **4.24** | **2.177** | 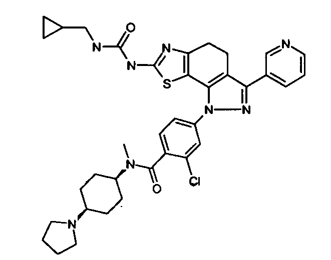 | 685 | 1.49 |
| **4.25** | **2.177** | 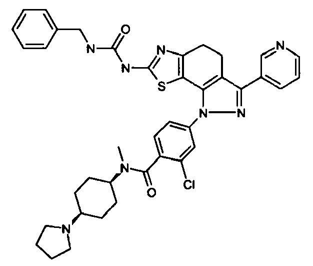 | 721 | 1.55 |
| **4.26** | **2.178** | 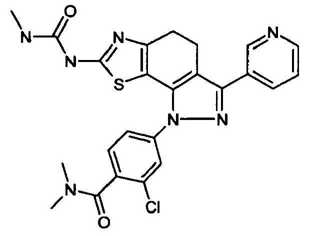 | 508 | 1.58 |
| **4.27** | **I-43** | 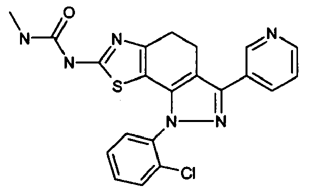 | 437 | 1.59 |
| **4.28** | **I-44** | 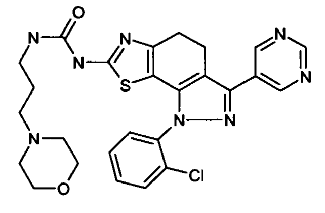 | 551 | Smp: 199 °C |
| **4.29** | **I-44** | 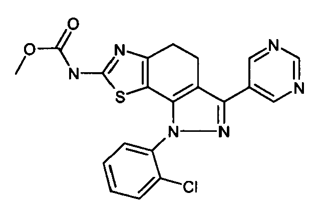 | 439 | Smp: 273 °C |
| **4.30** | **I-44** | 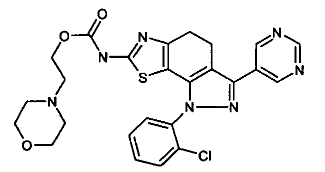 | 538 | Smp: 220 °C |
| **4.31** | **I-43** | 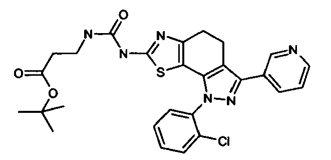 | 551 | 1.94 |
| **4.32** | **I-1** | 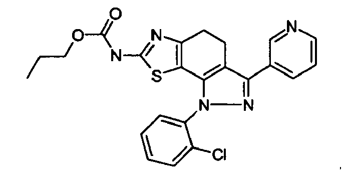 | 466 | 3.61 |

### Beispiele 5 - Umsetzung der Chlorpyridylbausteine

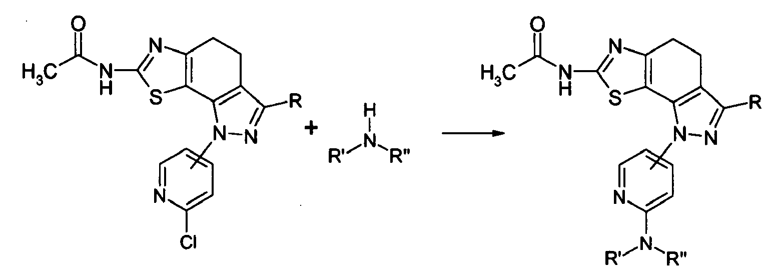

### Synthesemethode H

Liegen die Amine in flüssiger Form vor, so werden 0.2 mmol des Chlorpyridinbaustein in 0.5 mL Amin gelöst und in der Mikrowelle (CEM) 10 min lang auf 120 °C erwärmt. Nach Entfernen des überschüssigen Amins wird der Rückstand chromatographisch gereinigt.

### Synthesemethode I

Eine Lösung von 1.2 mmol Chlorpyridinbaustein, 3 mmol Amin in 3 mL N-Methylpyrrolidinon, DMSO oder DMF in der Mikrowelle (CEM) 10 min lang auf 120 °C erwärmt. Nach Entfernen des Lösungsmittels und des überschüssigen Amins wird der Rückstand chromatographisch gereinigt.

### Beispiele 5.1 - 5.23

| **#** | **Edukt** | **Struktur** | **Masse [M+1]⁺** | **HPLC Rt [min] bzw. Smp. bzw. *DC:R_{f}*** |
|---|---|---|---|---|
| **5.1** | **I-3** | 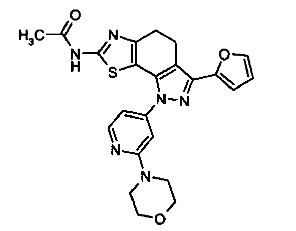 **¹H-NMR DMSO-d6, d [ppm]:** 8.28 (d, 5.4 Hz, 1H), 7.79 (s, 1H), 7.05 (s, 1H), 6.9-6.88 (m, 1H), 6.8 (d, 3.3 Hz, 1H), 6.64-6.62 (m, 1H), 3.72-3.67 (m, 2H), 3.58-3.55 (m, 2H), 3.11-3.06 (m, 2H), 3.04-3.00 (m, 2H), 2.13 (s, 3H). | 463 | 1.67 |
| **5.2** | **I-3** | 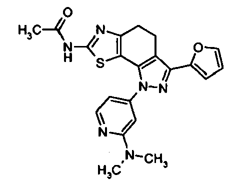 | 421 | 1.44 |
| **5.3** | **I-14** | 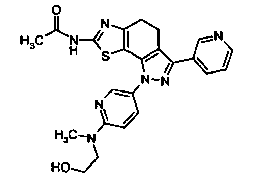 | 462 | 0.3 |
| **5.4** | **I-14** | 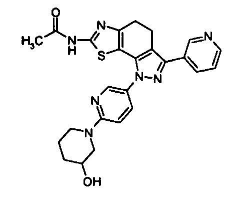 | 488 | 0.28 |
| **5.5** | **I-14** | 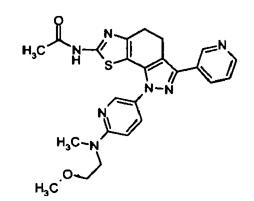 | 476 | 1.5 |
| **5.6** | **I-14** | 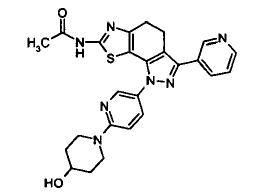 | 488 | 1.34 |
| **5.7** | **I-14** | 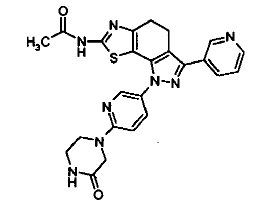 | 487 | 1.35 |
| **5.8** | **I-14** | 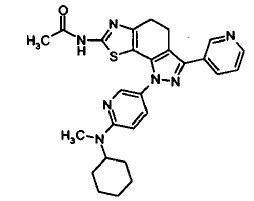 | 500 | 1.89 |
| **5.9*** | **I-14** | 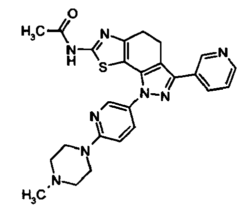 | 487 | 1.16 |
| **5.9b** | **I-14** | 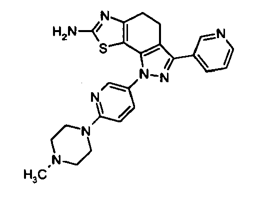 | 445 | R_{f} = 0.02 DCM:MeOH 9:1 |
| **5.10** | **I-14** | 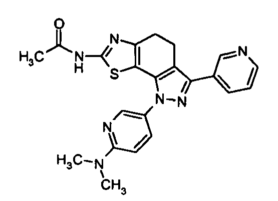 | 432 | 1.26 |
| **5.11** | **I-14** | 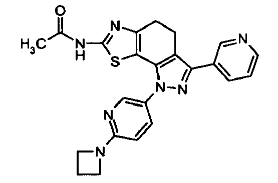 | 444 | 1.31 |
| **5.12** | **I-14** | 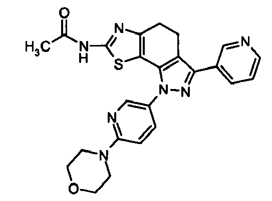 | 474 | 1.52 |
| **5.13** | **I-14** | 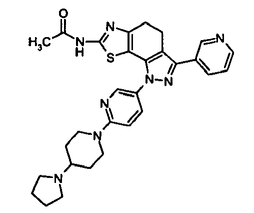 | 541 | 2.93 |
| **5.14** | **I-14** | 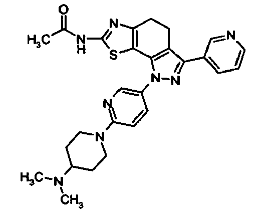 | 515 | 2.78 |
| **5.15** | **I-14** | 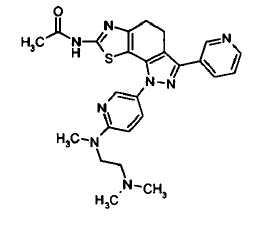 | 489 | 1.26 |
| **5.16*** | **I-15** | 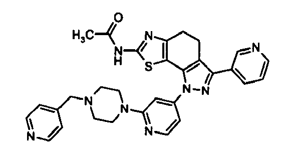 | 564 | R_{f}=0.56 MeOH |
| **5.16b** | **I-15** | 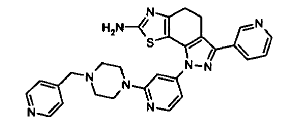 | 522 | 1.13 |
| **5.17** | **I-15** | 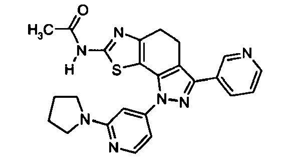 | 458 | R_{f} = 0.41 DCM:MeOH 8:2 |
| **5.18** | **I-15** | 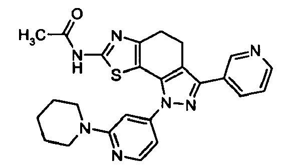 | 472 | 1.36 |
| **5.19** | **I-14** | 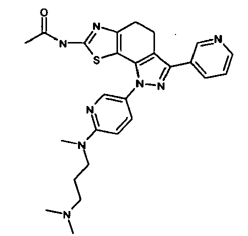 | 503 | R_{f}= 0.06 DCM:MeOH 9:1 |
| **5.20** | **I-14** | 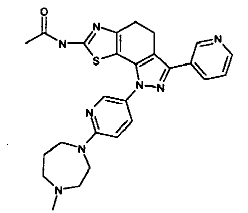 | 501 | R_{f} = 0.08 DCM:MeOH 9:1 |
| **5.21** | **I-14** | 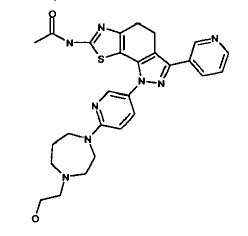 | 531 | 1.17 |
| **5.22** | **I-14** | 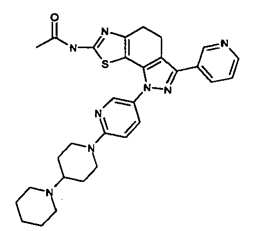 | 555 | 1.28 |
| **5.23*** | **I-14** | 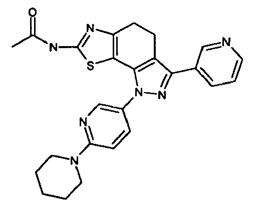 | 472 | 3.35 |
| **5.23b** | **I-14** | 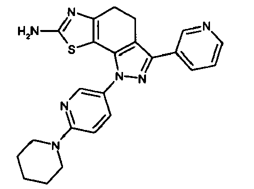 | 430 | 3.12 |

| | | | | |
|---|---|---|---|---|
| *Unter den Reaktionsbedingungen wird eine Abspaltung der Acetylgruppe beobachtet. Das entsprechende isolierte, freie Amin in den Beispielen 5.9b, 5.16b und 5.23b wird mit Essigsäureanhydrid in Dioxan wieder acetyliert. | | | | |

### Beispiele 6 - Reduktive Aminierung

### Methode J

Eine Lösung von 70 mg II-10 (0.15 mmol) und 22 µL *N*-Methylpiperidin-4-on (0.18 mmol) in 5 mL Dichlormethan wird 2 h bei RT gerührt. Nach Zugabe von 40 mg Natriumtriacetoxyborhydrid (0.18 mmol) wird die Reaktionsmischung weitere 15 h gerührt. Der Ansatz wird mit Dichlormethan verdünnt, mit verdünnter Natriumhydrogencarbonat-Lösung gewaschen, die organische Phase getrocknet und eingeengt. Der Rückstand wird in einer sehr geringen Menge Ethylacetat/Methanol angelöst und mit Diethylether kristallisiert. Die ausgefallenen Kristalle werden filtriert und im Vakuum getrocknet.

### Methode K

Eine Lösung von 220 mg II-11 (0.5 mmol) und 0.1 mL Benzylamin (1 mmol) in 5 mL Methanol wird 15 h bei 60 °C gerührt und anschließend mit 155 mg Natriumtriacetoxyborhydrid (0.7 mmol) und 40 mg Natriumacetat (0.5 mmol) versetzt. Nach Hydrolyse mit Natriumhydrogencarbonat und Extraktion mit Dichlormethan wird die organische Phase getrocknet, eingeengt und der Rückstand chromatographisch gereinigt.

### Beispiele 6.1- 6.4

| **#** | **Edukt** | **Methode** | **Struktur** | **Masse [M+1]⁺** | **HPLC:Rt [min] bzw. Smp. bzw. *DC:R_{f}*** |
|---|---|---|---|---|---|
| **6.1** | II-10 | **I** | 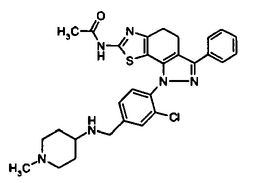 | 547 | 1.27 |
| **6.2** | II-11 | **K** | 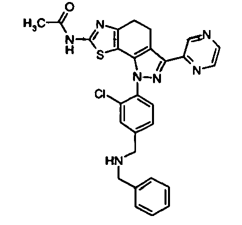 | 542 | 1.55 |
| **6.3** | II- 11 | **K** | 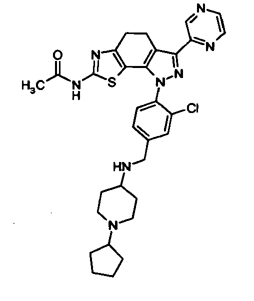 | 603 | 0.30 |
| **6.4** | II-12 | **I** | 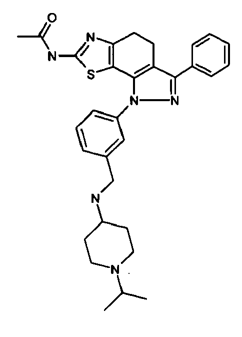 | 541 | Smp: 166 °C |

### Beispiele 7

### Synthesemethode L - Kreuzkupplung mit Arylboronsäuren

Eine Suspension von 100 mg 1-24 (0.2 mmol) in 3 mL Aceton wird mit 0.3 mmol der entsprechenden Boronsäure versetzt. Anschließend gibt man 4.4 mg Palladium(II)acetat (19 µmol), 4 µL Diazabicyclooctan (39 µmol) und 80 mg Kaliumcarbonat zu und erhitzt das Reaktionsgefäß 20 min auf 100 °C in der Mirkowelle, danach nochmals 2 x für jeweils 40 min bei 120 °C und 70 °C. Nach Entfernung des Lösungsmittels wird das Reaktionsgemisch chromatographisch gereinigt.

### Synthesemethode M - Kreuzkupplung mit Alkinen

Zu einer Lösung von 200 mg I-31 (0.3 mmol), 6 mg Kupfer(I)jodid (34 µmol) und 24 mg Triphenylphosphinpalladium(II)chlorid (34 µmol) in 25 mL entgastem THF gibt man unter Rühren und unter Argonatmosphäre 56 mg *N,N*-Dimethylaminoprop-2-in (0.7 mmol) und Diisopropylethylamin zu und rührt die Reaktionsmischung 15 h bei 80 °C. Man gibt 100 µL Alkin sowie 10 mg CuJ und 20 mg Pd-Katalysator zu und lässt weitere 24 h bei 55 °C rühren. Man stellt mit wässriger NH₃-Lösung alkalisch, verdünnt mit Wasser und extrahiert 2 x mit THF. Die vereinigten organischen Phasen werden mit gesättigter NaCl-Lösung ausgeschüttelt, getrocknet, filtriert und eingeengt. Der Rückstand wird chromatographisch gereinigt.

### Synthesemethode N - Palladiumkatalysierte Aminierung

Zu einer Lösung von 200 mg I-31 (0.3 mmol), 38 mg 4-Aminopyridin (0.4 mmol), 47 mg Tris(dibenzylidenaceton)dipalladium (51 µmol), 111 mg Natrium-*tert*-butylat (1 mmol) in 4 mL entgastem DMF wird 30 mg Tri-*tert*-Butylphosphintetrafluorborat (0.1 mmol) gegeben und 4 h bei 90 °C unter Argon gerührt. Nach Hydrolyse mit Phosphatpuffer und Wasser wird mit Dichlormethan extrahiert. Die organische Phase wird getrocknet, filtriert und eingeengt und der Rückstand chromatographisch gereinigt.

### Beispiele 7.1 - 7.8

| **#** | **Edukt** | **Struktur** | **Masse [M+1]⁺** | **HPLC Rt [min]** |
|---|---|---|---|---|
| **7.1** | **I-24** | 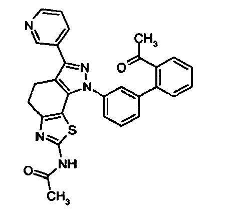 **¹H-NMR DMSO-d6, d [ppm]:** 8.95 (s, 1H), 8.61-8.58 (m, 1H), 8.14-8.11 (m, 1H), 7.67-7.65 (m, 2H), 7.63-7.58 (m, 3H), 7.56-7.48 (m, 2H), 7.45-7.41 (m, 1H), 3.17-3.12 (m, 2H), 3.06-3.01 (m, 2H), 2.26 (s, 3H), 2.10 (s, 3H). | 506 | 1.83 |
| **7.2** | **I-24** | 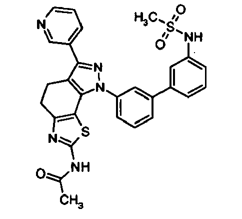 | 557 | 3.63 |
| **7.3** | **I-24** | 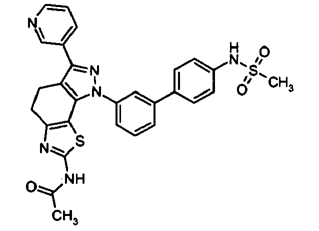 | 557 | 3.24 |
| **7.4** | **I-24** | 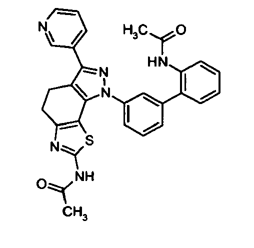 | 521 | 1.66 |
| **7.5** | **I-24** | 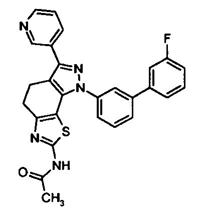 | 482 | 3.57 |
| **7.6** | **I-24** | 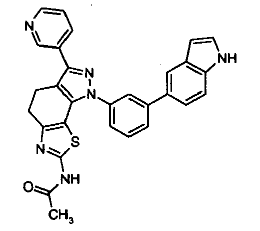 | 503 | 1.89 |
| **7.7** | **I-31** | 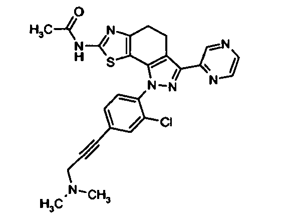 | 504 | 1.47 |
| **7.8** | **I-31** | 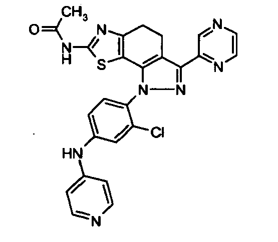 | 515 | 1.47 |

### Beispiel 8

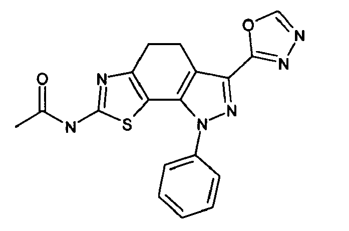

60 mg *N*-(3-Hydrazinocarbonyl-1-phenyl-4,5-dihydro-1*H*-pyrazolo[3',4':3,4]benz[1,2-*d*]thiazol-7-yl)-acetamid (0.16 mmol), welches nach der Synthesemethode B aus 1-6 und Hydrazin dargestellt werden kann, werden mit 3 mL Triethylorthoformiat 30 min bei 180 °C in der Mikrowelle behandelt. Nach Entfernung des überschüssigen Orthoesters im Vakuum wird der Rückstand chromatographisch gereinigt. Ausbeute 4 mg
[M+1]⁺= 379
Rt = 1.75 min.

### Beispiele 9.1 - 9.4

Analog zur Synthese von Beispiel 11-2 werden folgende Verbindungen hergestellt.

| **#** | **Edukt** | **Struktur** | **[M+1]⁺** | **Rt [min]** |
|---|---|---|---|---|
| **9.1** | **I-34** | 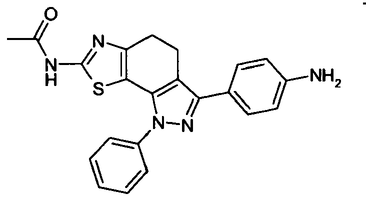 **¹H-NMR DMSO-d6, d [ppm]:** 7.61-7.51 (m, 5H), 7.40 (d, 8.5 Hz, 2H), 6.64 (d, 8.5 Hz, 2H), 3.06-2.95 (m, 4H), 2.09 (s, 3H). | 402 | 1.63 |
| **9.2** | **I-33** | 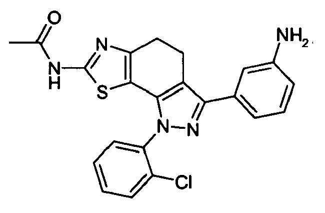 | 436 | 1.67 |
| **9.3** | **I-32** | 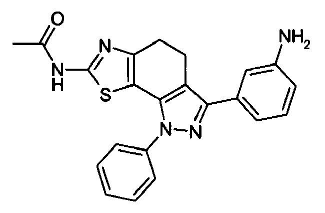 | 402 | 1.56 |
| **9.4** | **I-35** | 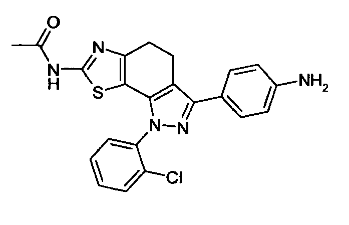 | 436 | 1.66 |

Das nachfolgende Beispiel beschreibt die biologische Wirkung der erfindungsgemäßen Verbindungen ohne die Erfindung auf dieses Beispiel einzuschränken.

### HCT116 Zytotoxizitätstest

Der Test basiert auf der Reduktion von AlamarBlue (Biosource Int., USA) in lebenden (metabolisch aktiven) Zellen zu einem fluorometrisch nachweisbaren Produkt. In Gegenwart von zelltoxischen Substanzen kann das Substrat nicht mehr reduziert werden, so dass kein Anstieg der Fluoreszenz messbar ist.

HCT116 (humane Kolonkarzinomzelllinie) Zellen werden in Mikrotiterplatten ausgesät und über Nacht in Kulturmedium bei 37 °C und 5% CO₂ inkubiert. Die Testsubstanzen werden in Medium schrittweise verdünnt und zu den Zellen gegeben, so dass das Gesamtvolumen 200 µL/well beträgt. Als Kontrollen dienen Zellen, die mit Medium, jedoch nicht mit Substanz, versetzt werden. Nach einer Inkubationszeit von 4 - 6 Tagen gibt man 20 µL/well AlamarBlue zu und inkubiert die Zellen für weitere 6 - 8 h bei 37 °C. Zur Fluoreszenzmessung wird bei einer Wellenlänge von 545 nm angeregt und bei 590 nm die Emission gemessen.
Die Berechnung von EC₅₀ Werten erfolgt mit Hilfe des Programms GraphPad Prism.
Alle angeführten Beispiele weisen einen EC₅₀ (HCT-116) von kleiner 5 µM auf.

Die Substanzen der vorliegenden Erfindung sind PI3-Kinase Inhibitoren. Aufgrund ihrer biologischen Eigenschaften eignen sich die neuen Verbindungen der allgemeinen Formel (1), deren Isomere und deren physiologisch verträgliche Salze zur Behandlung von Erkrankungen, die durch exzessive oder anomale Zellproliferation charakterisiert sind.

Zu solchen Erkrankungen gehören beispielsweise: Virale Infektionen (z.B. HIV und Kaposi Sarkoma); Entzündung und Autoimmun-Erkrankungen (z.B. Colitis, Arthritis, Alzheimer Erkrankung, Glomerulonephritis und Wund-Heilung); bakterielle, fungale und/oder parasitäre Infektionen; Leukämien, Lymphome und solide Tumore; HautErkrankungen (z.B. Psoriasis); Knochen-Erkrankungen; kardiovaskuläre Erkrankungen (z.B. Restenose und Hypertrophie). Ferner sind sie nützlich als Schutz von proliferierenden Zellen (z.B. Haar-, Intestinal-, Blut- und Progenitor-Zellen) gegen DNA-Schädigung durch Strahlung, UV-Behandlung und/oder zytostatischer Behandlung (Davis et al., 2001).

Beispielsweise können folgende Krebserkrankungen mit erfindungsgemäßen Verbindungen behandelt werden, ohne jedoch darauf beschränkt zu sein: Hirntumore wie beispielsweise Akustikusneurinom, Astrozytome wie pilozytisches Astrozytome, fibrilläres Astrozytom, protoplasmatisches Astrozytom, gemistozytäres Astrozytom, anaplastisches Astrozytom und Glioblastome, Hirnlymphome, Himmetastasen, Hypophysentumor wie Prolaktinom, HGH (human growth hormon) produzierender Tumor und ACTH produzierender Tumor (adrenocorticotropes Hormon), Kraniopharyngiome, Medulloblastome, Meningeome und Oligodendrogliome; Nerventumore (Neoplasmen) wie zum Beispiel Tumore des vegetativen Nervensystems wie Neuroblastoma sympathicum, Ganglioneurom, Paragangliom (Phäochromozytom, Chromaffinom) und Glomuscaroticum-Tumor, Tumore am peripheren Nervensystem wie Amputationsneurom, Neurofibrom, Neurinom (Neurilemmom, Schwannom) und malignes Schwannom, sowie Tumore am zentralen Nervensystem als Hirn- und Rückenmarkstumoren; Darmkrebs wie zum Beispiel Rektumkarzinom, Kolonkarzinom, Analkarzinom, Dünndarmtumore und Zwölffingerdarmtumore; Lidtumore wie Basaliom oder Basalzellkarzinom; Bauchspeicheldrüsenkrebs oder Pankreaskarzinom; Blasenkrebs oder Blasenkarzinom; Lungenkrebs (Bronchialkarzinom) wie beispielsweise kleinzellige Bronchialkarzinome (Haferzellkarzinome) und nicht-kleinzellige Bronchialkarzinome wie Plattenepithelkarzinome, Adenokarzinome und großzellige Bronchialkarzinome; Brustkrebs wie zum Beispiel Mammakarzinom wie infiltrierend duktales Karzinom, Kolloidkarzinom, lobulär invasives Karzinom, tubuläres Karzinom, adenozystisches Karzinom und papilläres Karzinom; Non-Hodgkin-Lymphomen (NHL) wie beispielsweise Burkitt-Lymphom, niedrigmaligne Non-Hodgkin-Lymphomen (NHL) und Mucosis fungoides; Gebärmutterkrebs oder Endometriumkarzinom bzw. Corpuskarzinom; CUP-Syndrom (Cancer of Unknown Primary); Eierstockskrebs oder Ovarial-Karzinom wie mucinöse, endometriale oder seröse Krebs; Gallenblasenkrebs; Gallengangskrebs wie beispielsweise Klatskin-Tumor; Hodenkrebs wie zum Beispiel Seminome und Nicht-Seminome; Lymphom (Lymphosarkom) wie zum Beispiel malignes Lymphom, Morbus Hodgkin, Non-Hodgkin-Lymphomen (NHL) wie chronisch lymphatische Leukämie, Haarzell-Leukämie, Immunozytom, Plasmozytom (multiples Myelom), Immunoblastom, Burkitt-Lymphom, T-Zonen-Mycosis fungoides, großzellig-anaplastisches Lymphoblastom und Lymphoblastom; Kehlkopfkrebs wie zum Beispiel Stimmbandtumoren, supraglottisch, glottisch und subglottisch Kehlkopftumore; Knochenkrebs wie zum Beispiel Osteochondrom, Chondrom, Chondroblastom, Chondromyxoidfibrom, Osteom, Osteoid-Osteom, Osteoblastom; eosinophiles Granulom, Riesenzell-Tumor, Chondrosarkom, Osteosarkom, Ewing-Sarkom, Retikulo-Sarkom, Plasmozytom, Riesenzell-Tumor, fibröse Dysplasie, juvenile Knochenzyste und aneurysmatische Knochenzyste; Kopf-Hals-Tumoren wie zum Beispiel Tumoren der Lippen, Zunge, Mundboden, Mundhöhle Zahnfleisch, Gaumen, Speicheldrüsen, Rachen, Nasenhöhlen, Nasennebenhöhlen, Kehlkopf und Mittelohr; Leberkrebs wie zum Beispiel das Leberzellkarzinom oder hepatozelluläres Karzinom (HCC); Leukämien wie zum Beispiel akute Leukämien wie akute lymphatische/lymphoblastische Leukämie (ALL), akute myeloische Leukämie (AML); chronische Leukämien wie chronisch lymphatische Leukämie (CLL), chronisch myeloische Leukämie (CML); Magenkrebs oder Magenkarzinom wie zum Beispiel papilläres, tubuläres und muzinöses Adenokarzinom, Siegelringzellkarzinom adenosquamöses Karzinom, kleinzelliges Karzinom und undifferenziertes Karzinom; Melanome wie zum Beispiel oberflächlich spreitendes, knotiges, lenitgo-maligna und akral-lentiginöse Melanom; Nierenkrebs wie zum Beispiel Nierenzellkarzinom oder Hypernephrom oder Grawitz-Tumor; Speiseröhrenkrebs oder Ösophaguskarzinom; Peniskrebs; Prostatakrebs; Rachenkrebs oder Pharynxkarzinome wie zum Beispiel Nasopharynxkarzinome, Oropharynxkarzinome und Hypopharynxkarzinome; Retinoblastom; Scheidenkrebs oder Vaginalkarzinom; Plattenepithelkarzinome, Adenokarzinome, in situ Karzinome, maligne Melanome und Sarkome; Schilddrüsen-Karzinome wie zum Beispiel papilläre, follikuläre und medulläre Schilddrüsen-Karzinom, sowie anaplastische Karzinome; Spinaliom, Stachelzellkarzinom und Plattenepithelkarzinom der Haut; Thymome, Urethrakrebs und Vulvakrebs.

Die neuen Verbindungen können zur Prävention, Kurz- oder Langzeitbehandlung der vorstehend erwähnten Krankheiten auch gegebenenfalls in Kombination mit anderen "State-of-art" Verbindungen, wie anderen anti-Tumor Substanzen, zytotoxische Substanzen, Zellproliferationshemmer, anti-angiogene Substanzen, Steroide oder Antikörper, verwendet werden.

Die Verbindungen der allgemeinen Formel (1) können allein oder in Kombination mit anderen erfindungsgemäßen Wirkstoffen, gegebenenfalls auch in Kombination mit weiteren pharmakologisch aktiven Wirkstoffen, zur Anwendung gelangen. Chemotherapeutica welche in Kombination mit den erfindungsgemäßen Verbindungen verabreicht werden können, umfassen ohne darauf eingeschränkt zu sein, Hormone, Hormonanaloge und Antihormone (z.B. Tamoxifen, Toremifen, Raloxifen, Fulvestrant, Megestrol Acetat, Flutamid, Nilutamid, Bicalutamid, Aminoglutethimid, Cyproteron Acetat, Finasterid, Buserelin Acetat, Fludrocortinson, Fluoxymesteron, Medroxyprogesteron, Octreotid), Aromatase Inhibitoren (z.B. Anastrozol, Letrozol, Liarozol, Vorozol, Exemestan, Atamestan,), LHRH Agonisten und Antagonists (z.B. Goserelin Acetat, Luprolid), Inhibitoren von Wachstumsfaktoren (growth factors wie zum Beispiel "platelet derived growth factor" und "hepatocyte growth factor", Inhibitoren sind z. B. "growth factor"-Antikörper, "growth factor receptor"-Antikörper und Tyrosinekinase Inhibitoren, wie zum Beispiel Gefitinib, Imatinib, Lapatinib und Trastuzumab); Antimetabolite (z.B. Antifolate wie Methotrexat, Raltitrexed, Pyrimidinanaloge wie 5-Fluorouracil, Capecitabin und Gemcitabin, Purin- und Adenosinanaloge wie Mercaptopurin, Thioguanin, Cladribin und Pentostatin, Cytarabin, Fludarabin); Antitumor-Antibiotica (z.B. Anthracycline wie Doxorubicin, Daunorubicin, Epirubicin und Idarubicin, Mitomycin-C, Bleomycin, Dactinomycin, Plicamycin, Streptozocin); Platinderivative (z.B. Cisplatin, Oxaliplatin, Carboplatin); Alkylierungsagentien (z.B. Estramustin, Meclorethamin, Melphalan, Chlorambucil, Busulphan, Dacarbazin, Cyclophosphamid, Ifosfamid, Temozolomid, Nitrosoharnstoffe wie zum Beispiel Carmustin und Lomustin, Thiotepa); antimitotische Agentien (z.B. Vinca-Alkaloide wie zum Beispiel Vinblastin, Vindesin, Vinorelbin und Vincristin; und Taxane wie Paclitaxel, Docetaxel); Topoisomerase Inhibitoren (z.B. Epipodophyllotoxine wie zum Beispiel Etoposid und Etopophos, Teniposid, Amsacrin, Topotecan, Irinotecan, Mitoxantron) und verschiedene Chemotherapeutica wie Amifostin, Anagrelid, Clodronat, Filgrastin, Interferon alpha, Leucovorin, Rituximab, Procarbazin, Levamisol, Mesna, Mitotan, Pamidronat und Porfimer.

Geeignete Anwendungsformen sind beispielsweise Tabletten, Kapseln, Zäpfchen, Lösungen, - insbesondere Lösungen zur Injektion (s.c., i.v., i.m.) und Infusion - Säfte, Emulsionen oder dispersible Pulver. Hierbei soll der Anteil der pharmazeutisch wirksamen Verbindung(en) jeweils im Bereich von 0,1 - 90 Gew.-%, bevorzugt 0,5 - 50 Gew.-% der Gesamtzusammensetzung liegen, das heißt in Mengen die ausreichend sind, um den unten angegebenen Dosierungsbereich zu erreichen. Die genannten Dosen können, falls erforderlich, mehrmals täglich gegeben werden.

Entsprechende Tabletten können beispielsweise durch Mischen des oder der Wirkstoffe mit bekannten Hilfsstoffen, beispielsweise inerten Verdünnungsmitteln, wie Calciumcarbonat, Calciumphosphat oder Milchzucker, Sprengmitteln, wie Maisstärke oder Alginsäure, Bindemitteln, wie Stärke oder Gelatine, Schmiermitteln, wie Magnesiumstearat oder Talk, und/oder Mitteln zur Erzielung des Depoteffektes, wie Carboxymethylcellulose, Celluloseacetatphthalat, oder Polyvinylacetat erhalten werden. Die Tabletten können auch aus mehreren Schichten bestehen.

Entsprechend können Dragees durch Überziehen von analog den Tabletten hergestellten Kernen mit üblicherweise in Drageeüberzügen verwendeten Mitteln, beispielsweise Kollidon oder Schellack, Gummi arabicum, Talk, Titandioxid oder Zucker, hergestellt werden. Zur Erzielung eines Depoteffektes oder zur Vermeidung von Inkompatibilitäten kann der Kern auch aus mehreren Schichten bestehen. Desgleichen kann auch die Drageehülle zur Erzielung eines Depoteffektes aus mehreren Schichten bestehen, wobei die oben bei den Tabletten erwähnten Hilfsstoffe verwendet werden können.

Säfte der erfindungsgemäßen Wirkstoffe beziehungsweise Wirkstoffkombinationen können zusätzlich noch ein Süßungsmittel, wie Saccharin, Cyclamat, Glycerin oder Zucker sowie ein geschmacksverbesserndes Mittel, z.B. Aromastoffe, wie Vanillin oder Orangenextrakt, enthalten. Sie können außerdem Suspendierhilfsstoffe oder Dickungsmittel, wie Natriumcarboxymethylcellulose, Netzmittel, beispielsweise Kondensationsprodukte von Fettalkoholen mit Ethylenoxid, oder Schutzstoffe, wie p-Hydroxybenzoate, enthalten.

Injektions- und Infusionslösungen werden in üblicher Weise, z.B. unter Zusatz von Isotonantien, Konservierungsmitteln, wie p-Hydroxybenzoate, oder Stabilisatoren, wie Alkalisalzen der Ethylendiamintetraessigsäure, gegebenenfalls unter Verwendung von Emulgiermitteln und /oder Dispergiermitteln, wobei beispielsweise bei der Verwendung von Wasser als Verdünnungsmittel gegebenenfalls organische Lösemittel als Lösevermittler bzw. Hilfslösemittel eingesetzt werden können, hergestellt und in Injektionsflaschen oder Ampullen oder Infusionsflaschen abgefüllt.

Die eine oder mehrere Wirkstoffe beziehungsweise Wirkstoffkombinationen enthaltenden Kapseln können beispielsweise hergestellt werden indem man die Wirkstoffe mit inerten Trägern, wie Milchzucker oder Sorbit, mischt und in Gelatinekapseln einkapselt. Geeignete Zäpfchen lassen sich beispielsweise durch Vermischen mit dafür vorgesehenen Trägermitteln, wie Neutralfetten oder Polyäthylenglykol beziehungsweise dessen Derivaten, herstellen.

Als Hilfsstoffe seien beispielsweise Wasser, pharmazeutisch unbedenkliche organische Lösemittel, wie Paraffine (z.B. Erdölfraktionen), Öle pflanzlichen Ursprungs (z.B. Erdnuss- oder Sesamöl), mono- oder polyfunktionelle Alkohole (z.B. Ethanol oder Glycerin), Trägerstoffe wie z.B. natürliche Gesteinsmehle (z.B. Kaoline, Tonerden, Talkum, Kreide) synthetische Gesteinsmehle (z.B. hochdisperse Kieselsäure und Silikate), Zucker (z.B. Rohr-, Milch- und Traubenzucker) Emulgiermittel (z.B. Lignin, Sulfitablaugen, Methylcellulose, Stärke und Polyvinylpyrrolidon) und Gleitmittel (z.B. Magnesiumstearat, Talkum, Stearinsäure und Natriumlaurylsulfat) erwähnt.

Die Applikation erfolgt in üblicher Weise, vorzugsweise oral oder transdermal, insbesondere bevorzugt oral. Im Falle der oralen Anwendung können die Tabletten selbstverständlich außer den genannten Trägerstoffen auch Zusätze, wie z.B. Natriumcitrat, Calciumcarbonat und Dikalziumphosphat zusammen mit verschiedenen Zuschlagstoffen, wie Stärke, vorzugsweise Kartoffelstärke, Gelatine und dergleichen enthalten. Weiterhin können Gleitmittel, wie Magnesiumstearat, Natriumlaurylsulfat und Talkum zum Tablettieren mitverwendet werden. Im Falle wässriger Suspensionen können die Wirkstoffe außer den oben genannten Hilfsstoffen mit verschiedenen Geschmacksaufbesserem oder Farbstoffen versetzt werden.

Für den Fall der parenteralen Anwendung können Lösungen der Wirkstoffe unter Verwendung geeigneter flüssiger Trägermaterialien eingesetzt werden.
Die Dosierung für die intravenöse Anwendung liegt bei 1 - 1000 mg pro Stunde, vorzugsweise zwischen 5 - 500 mg pro Stunde.

Trotzdem kann es gegebenenfalls notwendig sein, von den genannten Mengen abzuweichen, und zwar in Abhängigkeit vom Körpergewicht bzw. der Art des Applikationsweges, vom individuellen Verhalten gegenüber dem Medikament, der Art von dessen Formulierung und dem Zeitpunkt bzw. Intervall, zu welchen die Verabreichung erfolgt. So kann es in einigen Fällen ausreichend sein, mit weniger als der vorgenannten Mindestmenge auszukommen, während in anderen Fällen die genannte obere Grenze überschritten werden muss. Im Falle der Applikation größerer Mengen kann es empfehlenswert sein, diese in mehreren Einzelgaben über den Tag zu verteilen.

Die nachfolgenden Formulierungsbeispiele illustrieren die vorliegende Erfindung ohne sie jedoch in ihrem Umfang zu beschränken:

### Pharmazeutische Formulierungsbeispiele

| A) | Tabletten | pro Tablette |
|---|---|---|
| | Wirkstoff nach Formel (1) | 100 mg |
| | Milchzucker | 140 mg |
| | Maisstärke , | 240 mg |
| | Polyvinylpyrrolidon | 15 mg |
| | Magnesiumstearat | 5 mg |
| | | 500 mg |

Der fein gemahlene Wirkstoff, Milchzucker und ein Teil der Maisstärke werden miteinander vermischt. Die Mischung wird gesiebt, danach mit einer Lösung von Polyvinylpyrrolidon in Wasser befeuchtet, geknetet, feucht granuliert und getrocknet. Das Granulat, der Rest der Maisstärke und das Magnesiumstearat werden gesiebt und miteinander vermischt. Das Gemisch wird zu Tabletten geeigneter Form und Größe verpresst.

| B) | Tabletten | pro Tablette |
|---|---|---|
| | Wirkstoff nach Formel (1) | 80 mg |
| | Milchzucker | 55 mg |
| | Maisstärke | 190 mg |
| | Mikrokristalline Cellulose | 35 mg |
| | Polyvinylpyrrolidon | 15 mg |
| | Natrium-carboxymethylstärke | 23 mg |
| | Magnesiumstearat | 2 mg |
| | | 400 mg |

Der fein gemahlene Wirkstoff, ein Teil der Maisstärke, Milchzucker, mikrokristalline Cellulose und Polyvinylpyrrolidon werden miteinander vermischt, die Mischung gesiebt und mit dem Rest der Maisstärke und Wasser zu einem Granulat verarbeitet, welches getrocknet und gesiebt wird. Dazu gibt man die Natriumcarboxymethylstärke und das Magnesiumstearat, vermischt und verpresst das Gemisch zu Tabletten geeigneter Größe.

| C) | Ampullenlösung | |
|---|---|---|
| | Wirkstoff nach Formel (1) | 50 mg |
| | Natriumchlorid | 50 mg |
| | Aqua pro inj. | 5 ml |

Der Wirkstoff wird bei Eigen-pH oder gegebenenfalls bei pH 5,5 - 6,5 in Wasser gelöst und mit Natriumchlorid als Isotonans versetzt. Die erhaltene Lösung wird pyrogenfrei filtriert und das Filtrat unter aseptischen Bedingungen in Ampullen abgefüllt, die anschließend sterilisiert und zugeschmolzen werden. Die Ampullen enthalten 5 mg, 25 mg und 50 mg Wirkstoff.

## Patentansprüche

1. Verbindungen der allgemeinen Formel (1), 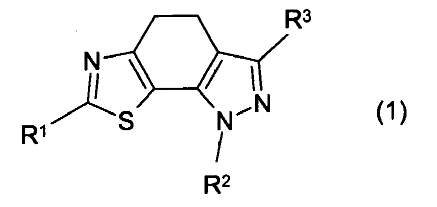 worin
R¹ ausgewählt aus der Gruppe bestehend aus -NHR^{c}, -NHC(O)R^{c}, -NHC(O)OR^{c}, -NHC(O)NR^{c}R^{c} und -NHC(O)SR^{c};
R² ein Rest, gegebenenfalls substituiert mit einem oder mehreren R⁴, ausgewählt aus der Gruppe bestehend aus C₁₋₆Alkyl, C₃₋₈Cycloalkyl, 3-8 gliedriges Heterocycloalkyl, C₆₋₁₀Aryl, C₇₋₁₆Arylalkyl und 5-10 gliedriges Heteroaryl;
R³ ein gegebenenfalls mit einem oder mehreren R^{e} und/oder R^{f} substituiert Rest ausgewählt aus der Gruppe bestehend aus C₆₋₁₀Aryl und 5-10 gliedriges Heteroaryl;
R⁴ ein Rest ausgewählt aus der Gruppe bestehend aus R^{a}, R^{b} und R^{a} substituiert mit einem oder mehreren, gleich oder verschiedenen R^{c} und/oder R^{b};
jedes R^{a} unabhängig voneinander ausgewählt aus der Gruppe bestehend aus C₁₋₆Alkyl, C₃₋₈Cycloalkyl, C₄₋₁₁Cycloalkylalkyl, C₆₋₁₀Aryl, C₇₋₁₆Arylalkyl, 2-6 gliedriges Heteroalkyl, 3-8 gliedriges Heterocycloalkyl, 4-14 gliedriges Heterocycloalkylalkyl, 5-10 gliedriges Heteroaryl und 6-16 gliedriges Heteroarylalkyl;
jedes R^{b} ein geeigneter Rest und jeweils unabhängig voneinander ausgewählt aus der Gruppe bestehend aus =O, -OR^{c}, C₁₋₃Haloalkyloxy, -OCF₃, =S, -SR^{c}, =NR^{c}, =NOR^{c}, -NR^{c}R^{c}, Halogen, -CF3, -CN, -NC, -OCN, -SCN, -NO, -NO₂, =N₂, -N₃, -S(O)R^{c}, -S(O)₂R^{c}, -S(O)₂OR^{c}, -S(O)NR^{c}R^{c}, -S(O)₂NR^{c}R^{c}, -OS(O)R^{c}, -OS(O)₂R^{c}, -OS(O)₂OR^{c}, -OS(O)₂NR^{c}R^{c}, -C(O)R^{c}, -C(O)OR^{c}, -C(O)NR^{c}R^{c}, -C(O)N(R^{g})NR^{c}R^{c}, -C(O)N(R^{g})OR^{c}, -CN(R^{g})NR^{c}R^{c}, -OC(O)R^{c}, -OC(O)OR^{c}, -OC(O)NR^{c}R^{c}, -OCN(R^{g})NR^{c}R^{c}, -N(R^{g})C(O)R^{c}, -N(R^{g})C(O)R^{c}, -N(R^{g})C(S)R^{c}, -N(R^{g})S(O)₂R^{c}, -N(R^{g})S(O)₂NR^{c}R^{c}, -N[S(O)₂]₂R^{c},-N(R^{g})C(O)OR^{c}, -N(R^{g})C(O)NR^{c}R^{c}, und -N(R^{g})CN(R^{g})NR^{c}R^{c};
jedes R^{c} unabhängig voneinander Wasserstoff oder ein gegebenenfalls mit einem oder mehreren, gleich oder verschiedenen R^{d} und/oder R^{e} substituierter Rest ausgewählt aus der Gruppe bestehend aus C₁₋₆Alkyl, C₃₋₈Cycloalkyl, C₄₋₁₁Cycloalkylalkyl, C₆₋₁₀Aryl, C₇₋₁₆Arylalkyl, 2-6 gliedriges Heteroalkyl, 3-8 gliedriges Heterocycloalkyl, 4-14 gliedriges Heterocycloalkylalkyl, 5-10 gliedriges Heteroaryl und 6-16 gliedriges Heteroarylalkyl,
jedes R^{d} unabhängig voneinander Wasserstoff oder ein gegebenenfalls mit einem oder mehreren, gleich oder verschiedenen R^{e} und/oder R^{f} substituierter Rest ausgewählt aus der Gruppe bestehend aus C₁₋₆Alkyl, C₃₋₈Cycloalkyl, C₄₋₁₁Cycloalkylalkyl, C₆₋₁₀Aryl, C₇₋₁₆Arylalkyl, 2-6 gliedriges Heteroalkyl, 3-8 gliedriges Heterocycloalkyl, 4-14 gliedriges Heterocycloalkylalkyl, 5-10 gliedriges Heteroaryl und 6-16 gliedriges Heteroarylalkyl,
jedes R^{e} ein geeigneter Rest und jeweils unabhängig voneinander ausgewählt aus der Gruppe bestehend aus =O, -OR^{f}, C₁₋₃Haloalkyloxy, -OCF₃, =S, -SR^{f}, =NR^{f}, =NOR^{f}, -NR^{f}R^{f}, Halogen, -CF3, -CN, -NC, -OCN, -SCN, -NO, -NO₂, =N₂, -N₃, -S(O)R^{f}, -S(O)₂R^{f}, -S(O)₂OR^{f}, -S(O)NR^{f}R^{f}, -S(O)₂NR^{f}R^{f}, -OS(O)R^{f}, -OS(O)₂R^{f}, -OS(O)₂OR^{f}, -OS(O)₂NR^{f}R^{f}, -C(O)R^{f}, -C(O)OR^{f}, -C(O)NR^{f}R^{f}, -CN(R^{g})NR^{f}R^{f}, -OC(O)R^{f}, -OC(O)OR^{f}, -OC(O)NR^{f}R^{f}, -OCN(R^{g})NR^{f}R^{f}, -N(R^{g})C(O)R^{f}, -N(R^{g})C(S)R^{f}, -N(R^{g})S(O)₂R^{f}, -N(R^{g})C(O)OR^{f}, -N(R^{g})C(O)NR^{f}R^{f}, und -N(R^{g})CN(R^{g})NR^{f}R^{f};
jedes R^{f} unabhängig voneinander Wasserstoff oder ein gegebenenfalls mit einem oder mehreren, gleich oder verschiedenen R^{g} substituierter Rest ausgewählt aus der Gruppe bestehend aus C₁₋₆Alkyl, C₃₋₈Cycloalkyl, C₄₋₁₁Cycloalkylalkyl, C₆₋₁₀Aryl, C₇₋₁₆Arylalkyl, 2-6 gliedriges Heteroalkyl, 3-8 gliedriges Heterocycloalkyl, 4-14 gliedriges Heterocycloalkylalkyl, 5-10 gliedriges Heteroaryl und 6-16 gliedriges Heteroarylalkyl
jedes R^{g} unabhängig voneinander Wasserstoff, C₁₋₆Alkyl, C₃₋₈Cycloalkyl, C₄₋₁₁Cycloalkylalkyl, C₆₋₁₀Aryl, C₇₋₁₆Arylalkyl, 2-6 gliedriges Heteroalkyl, 3-8 gliedriges Heterocycloalkyl, 4-14 gliedriges Heterocycloalkylalkyl, 5-10 gliedriges Heteroaryl und 6-16 gliedriges Heteroarylalkyl
bedeuten, gegebenenfalls in Form ihrer Tautomeren, ihrer Racemate, ihrer Enantiomere, ihrer Diastereomere und ihrer Gemische, sowie gegebenenfalls ihrer pharmakologisch unbedenklichen Säureadditionssalze.

2. Verbindungen nach Anspruch 1, wobei R³ ein Rest ausgewählt aus der Gruppe bestehend aus Phenyl, Furyl, Pyridyl, Pyrimidinyl und Pyrazinyl, gegebenenfalls substituiert mit einem oder mehreren R⁴, bedeutet.

3. Verbindungen nach Anspruch 2, wobei R³ Pyridyl bedeutet.

4. Verbindungen nach Anspruch 1 bis 3, wobei R¹ -NHC(O)R^{c} bedeutet.

5. Verbindungen nach Anspruch 4, wobei R¹ -NHC(O)CH₃ bedeutet.

6. Verbindungen der Formel (A) 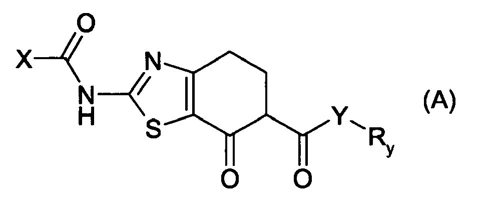 wobei
X -CH₃, -OR⁴ oder -SR⁴ und
Y Phenyl, 5-10 gliedriges Heteroaryl oder die Gruppe -C(O)O und
R^{y} Wasserstoff,-NO₂ oder C₁₋₆Alkyl bedeuten und R⁴ wie vorstehend definiert ist.

7. Verbindungen nach Anspruch 6, wobei R⁴ gleich - C₁₋₆Alkyl bedeutet.

8. Verbindungen der allgemeinen Formel (A) nach Anspruch 6 oder 7 zur Verwendung als Syntheseintermediate.

9. Verbindungen - oder deren pharmazeutisch wirksamen Salze - nach Anspruch 1 bis 5 als Arzneimittel.

10. Verbindungen - oder deren pharmazeutisch wirksamen Salze - nach Anspruch 1 bis 5 zur Herstellung eines Arzneimittels mit antiproliferativer Wirkung.

11. Pharmazeutische Zubereitungen, enthaltend als Wirkstoff eine oder mehrere Verbindungen der allgemeinen Formel (1) gemäß einem der Ansprüche 1 bis 5 oder deren physiologisch verträglichen Salze gegebenenfalls in Kombination mit üblichen Hilfs- und/oder Trägerstoffen.

12. Verwendung von Verbindungen der allgemeinen Formel (1) gemäß einem der Ansprüche 1 bis 5 zur Herstellung eines Arzneimittels zur Behandlung und/oder Prävention von Krebs.

13. Pharmazeutische Präparation umfassend eine Verbindung der allgemeinen Formel (1) gemäß einem der Ansprüche 1 bis 5 und mindestens eine weitere zytostatische oder zytotoxische, von Formel (1) verschiedene Wirksubstanz, gegebenenfalls in Form ihrer Tautomeren, ihrer Racemate, ihrer Enantiomere, ihrer Diastereomere und ihrer Gemische, sowie gegebenenfalls ihrer pharmakologisch unbedenklichen Säureadditionssalze.

## Claims

1. Compounds of the general formula (1), 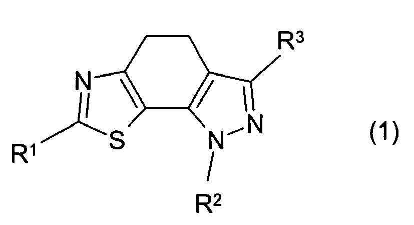 in which
R¹ is selected from the group consisting of -NHR^{c}, -NHC(O)R^{c}, -NHC(O)OR^{c}, -NHC(O)NR^{c}R^{c} and -NHC(O)SR^{c};
R² is a radical which is optionally substituted by one or more R⁴ and which is selected from the group consisting of C₁₋₆alkyl, C₃₋₈cycloalkyl, 3-8-membered heterocycloalkyl, C₆₋₁₀aryl, C₇₋₁₆arylalkyl and 5-10-membered heteroaryl;
R³ is a radical which is optionally substituted by one or more R^{e} and/or R^{f} and is selected from the group consisting of C₆₋₁₀aryl and 5-10-membered heteroaryl;
R⁴ is a radical selected from the group consisting of R^{a}, R^{b} and R^{a} which is substituted by one or more, identical or different, R^{c} and/or R^{b};
each R^{a} is, independently of each other, selected from the group consisting of C₁₋₆alkyl, C₃₋₈cycloalkyl, C₄₋₁₁cycloalkylalkyl, C₆₋₁₀aryl, C₇₋₁₆arylalkyl, 2-6-membered heteroalkyl, 3-8-membered heterocycloalkyl, 4-14-membered heterocycloalkylalkyl, 5-10-membered heteroaryl and 6-16-membered heteroarylalkyl;
each R^{b} is a suitable radical and in each case selected, independently of each other, from the group consisting of =O, -OR^{c}, C₁₋₃haloalkyloxy, -OCF₃, =S, -SR^{c}, =NR^{c}, =NOR^{c}, -NR^{c}R^{c}, halogen, -CF3, -CN, -NC, -OCN, -SCN, -NO, -NO₂, =N₂, -N₃, -S(O)R^{c}, -S(O)₂R^{c}, -S(O)₂OR^{c}, -S(O)NR^{c}R^{c}, -S(O)₂NR^{c}R^{c}, -OS(O)R^{c}, -OS(O)₂R^{c}, -OS(O)₂OR^{c}, -OS(O)₂NR^{c}R^{c}, -C(O)R^{c}, -C(O)OR^{c}, -C(O)NR^{c}R^{c}, -C(O)N(R^{g})NR^{c}R^{c}, -C(O)N(R^{g})OR^{c}, -CN(R^{g})NR^{c}R^{c}, -OC(O)R^{c}, -OC(O)OR^{c}, -OC(O)NR^{c}R^{c}, -OCN(R^{g})NR^{c}R^{c}, -N(R^{g})C(O)R^{c}, -N(R^{g})C(O)R^{c}, -N(R^{g})C(S)R^{c}, -N(R^{g})S(O)₂R^{c}, -N(R^{g})S(O)₂NR^{c}R^{c}, -N[S(O)₂]R^{c}, -N(R^{g})C(O)OR^{c}, -N(R^{g})C(O)NR^{c}R^{c}, and -N(R^{g})CN(R^{g})NR^{c}R^{c};
each R^{c} is, independently of each other, hydrogen or a radical which is optionally substituted by one or more, identical or different, R^{a} and/or R^{e} and which is selected from the group consisting of C₁₋₆alkyl, C₃₋₈cycloalkyl, C₄₋₁cycloalkylalkyl, C₆₋₁₀aryl, C₇₋₁₆arylalkyl, 2-6-membered heteroalkyl, 3-8-membered heterocycloalkyl, 4-14-membered heterocycloalkylalkyl, 5-10-membered heteroaryl and 6-16-membered heteroarylalkyl,
each R^{d} is, independently of each other, hydrogen or a radical which is optionally substituted by one or more, identical or different, R^{e} and/or R^{f} and which is selected from the group consisting of C₁₋₆alkyl, C₃₋₈cycloalkyl, C₄₋₁₁cycloalkylalkyl, C₆₋₁₀aryl, C₇₋₁₆arylalkyl, 2-6-membered heteroalkyl, 3-8-membered heterocycloalkyl, 4-14-membered heterocycloalkylalkyl, 5-10-membered heteroaryl and 6-16-membered heteroarylalkyl,
each R^{e} is a suitable radical and in each case selected, independently of each other, from the group consisting of =O, -OR^{f}, C₁₋₃haloalkyloxy, -OCF₃, =S, -SR^{f}, =NR^{f}, =NOR^{f}, -NR^{f}R^{f}, halogen, -CF3, -CN, -NC, -OCN, -SCN, -NO, -NO₂, =N₂, -N₃, -S(O)R^{f}, -S(O)₂R^{f}, -S(O)₂OR^{f}, -S(O)NR^{f}R^{f}, -S(O)₂NR^{f}R^{f}, -OS(O)R^{f}, -OS(O)₂R^{f}, -OS(O)₂OR^{f}, -OS(O)₂NR^{f}R^{f}, -C(O)R^{f}, -C(O)OR^{f}, -C(O)NR^{f}R^{f}, -CN(R^{g})NR^{f}R^{f}, -OC(O)R^{f}, -OC(O)OR^{f}, -OC(O)NR^{f}R^{f}, -OCN(R^{g})NR^{f}R^{f}, -N(R^{g})C(O)R^{f}, -N(R^{g})C(S)R^{f}, -N(R^{g})S(O)₂R^{f}, -N(R^{g})C(O)OR^{f}, -N(R^{g})C(O)NR^{f}R^{f}, and -N(R^{g})CN(R^{g})NR^{f}R^{f};
each R^{f} is, independently of each other, hydrogen or a radical which is optionally substituted by one or more, identical or different, R^{g} and is selected from the group consisting of C₁₋₆alkyl, C₃₋₈cycloalkyl, C₄₋₁₁cycloalkylalkyl, C₆₋₁₀aryl, C₇₋₁₆arylalkyl, 2-6-membered heteroalkyl, 3-8-membered heterocycloalkyl, 4-14-membered heterocycloalkylalkyl, 5-10-membered heteroaryl and 6-16-membered heteroarylalkyl,
each R^{g} is, independently of each other, hydrogen, C₁₋₆alkyl, C₃₋₈Cycloalkyl, C₄₋₁₁cycloalkylalkyl, C₆₋₁₀aryl, C₇₋₁₆arylalkyl, 2-6-membered heteroalkyl, 3-8-membered heterocycloalkyl, 4-14-membered heterocycloalkylalkyl, 5-10-membered heteroaryl and 6-16-membered heteroarylalkyl,
where appropriate in the form of their tautomers, their racemates, their enantiomers, their diastereomers and their mixtures, as well as, where appropriate, their pharmacologically harmless acid addition salts.

2. Compounds according to Claim 1, wherein R³ is a radical selected from the group consisting of phenyl, furyl, pyridyl, pyrimidinyl and pyrazinyl, optionally substituted by one or more R⁴.

3. Compounds according to Claim 2, wherein R³ is pyridyl.

4. Compounds according to Claims 1 to 3, wherein R¹ is -NHC(O)R^{c}.

5. Compounds according to Claim 4, wherein R¹ is -NHC(O)CH₃.

6. Compounds of the formula (A) 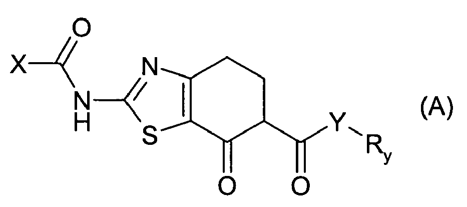 wherein
X is -CH₃, -OR⁴ or -SR⁴ and
Y is phenyl, 5-10-membered heteroaryl or the group -C(O)O, and
R^{y} is hydrogen, -NO₂ or C₁₋₆alkyl and R⁴ is as hereinbefore defined.

7. Compounds according to Claim 6, wherein R⁴ is -C₁₋₆alkyl.

8. Compounds of the general formula (A) according to Claim 6 or 7 for use as synthesis intermediates.

9. Compounds, or their pharmaceutically active salts, according to Claims 1 to 5 as medicaments.

10. Compounds, or their pharmaceutically active salts, according to Claims 1 to 5 for producing a medicament having an antiproliferative effect.

11. Pharmaceutical preparations which comprise, as active compound, one or more compounds of the general formula (1) according to one of Claims 1 to 5, or their physiologically tolerated salts, where appropriate in combination with customary auxiliary substances and/or carrier substances.

12. Use of compounds of the general formula (1) according to one of Claims 1 to 5 for producing a medicament for treating and/or preventing cancer.

13. Pharmaceutical preparation which comprises a compound of the general formula (1) according to one of Claims 1 to 5 and at least one further cytostatic or cytotoxic active substance which differs from formula (1), where appropriate in the form of their tautomers, their racemates, their enantiomers, their diastereomers and their mixtures, as well as, where appropriate, their pharmacologically harmless acid addition salts.

## Revendications

1. Composés de formule générale (1) 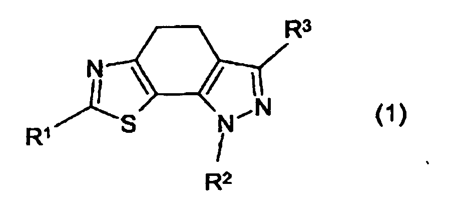 où
R¹ est choisi dans le groupe consistant en -NHR^{c}, -NHC(O)R^{c}, -NHC(O)OR^{c}, -NHC(O)NR^{c}R^{c} et -NHC(O)SR^{c} ;
R² est un groupement, éventuellement substitué avec un ou plusieurs R⁴, choisi dans le groupe consistant en C₁₋₆alkyle, C₃₋₈cycloalkyle, hétérocycloalkyle à 3-8 chaînons, C₆₋₁₀aryle, C₇₋₁₆arylalkyle et hétéroaryle à 5-10 chaînons ;
R³ est un groupement éventuellement substitué avec un ou plusieurs R^{e} et/ou R^{f} choisi dans le groupe consistant en C₆₋₁₀aryle et hétéroaryle à 5-10 chaînons ;
R⁴ est un groupement choisi dans le groupe consistant en R^{a}, R^{b} et R^{a} substitué avec un ou plusieurs R^{c} et/ou R^{b} identiques ou différents ;
chaque R^{a} est choisi indépendamment des autres dans le groupe consistant en C₁₋₆alkyle, C₃₋₈cycloalkyle, C₄₋₁₁cycloalkylalkyle, C₆₋₁₀aryle, C₇₋₁₆arylalkyle, hétéroalkyle à 2-6 chaînons, hétérocycloalkyle à 3-8 chaînons, hétérocycloalkylalkyle à 4-14 chaînons, hétéroaryle à 5-10 chaînons et hétéroarylalkyle à 6-16 chaînons ;
chaque R^{b} est un groupement approprié et choisi dans chaque cas indépendamment des autres dans le groupe consistant en =O, -OR^{c}, C₁₋₃haloalkyloxy, -OCF₃, =S, -SR^{c}, =NR^{c}, =NOR^{c}, -NR^{c}R^{c}, halogène, -CF3, -CN,-NC, -OCN, -SCN, -NO, -NO₂, =N₂, -N₃, -S(O)R^{c}, -S(O)₂R^{c}, -S(O)₂OR^{c},-S(O)NR^{c}R^{c}, -S(O)₂NR^{c}R^{c}, -OS(O)R^{c}, -OS(O)₂R^{c}, -OS(O)₂OR^{c}, -OS(O)₂NR^{c}R^{c},-C(O)R^{c}, -C(O)OR^{c}, -C(O)NR^{c}R^{c}, -C(O)N(R^{g})NR^{c}R^{c}, -C(O)N(R^{g})OR^{c},-CN(R^{g})NR^{c}R^{c}, -OC(O)R^{c}, -OC(O)OR^{c}, -OC(O)NR^{c}R^{c}, -OCN(R^{g})NR^{c}R^{c},-N(R^{g})C(O)R^{c}, -N(R^{g})C(O)R^{c}, -N(R^{g})C(S)R^{c}, -N(R^{g})S(O)₂R^{c}, -N(R^{g})S(O)₂NR^{c}R^{c}, -N[S(O)₂]₂R^{c}, -N(R^{g})C(O)OR^{c}, -N(R^{g})C(O)NR^{c}R^{c} et -N(R^{g})CN(R^{g})NR^{c}R^{c} ;
chaque R^{c} représente indépendamment des autres l'hydrogène ou un groupement éventuellement substitué avec un ou plusieurs R^{d} et/ou R^{e} identiques ou différents choisi dans le groupe consistant en C₁₋₆alkyle, C₃₋₈cycloalkyle, C₄₋₁₁cycloalkylalkyle, C₆₋₁₀aryle, C₇₋₁₆arylalkyle, hétéroalkyle à 2-6 chaînons, hétérocycloalkyle à 3-8 chaînons, hétérocycloalkylalkyle à 4-14 chaînons, hétéroaryle à 5-10 chaînons et hétéroarylalkyle à 6-16 chaînons ;
chaque R^{d} représente indépendamment des autres l'hydrogène ou un groupement éventuellement substitué avec un ou plusieurs R^{e} et/ou R^{f} identiques ou différents choisi dans le groupe consistant en C₁₋₆alkyle, C₃₋₈cycloalkyle, C₄₋₁₁cycloalkylalkyle, C₆₋₁₀aryle, C₇₋₁₆arylalkyle, hétéroalkyle à 2-6 chaînons, hétérocycloalkyle à 3-8 chaînons, hétérocycloalkylalkyle à 4-14 chaînons, hétéroaryle à 5-10 chaînons et hétéroarylalkyle à 6-16 chaînons ;
chaque R^{e} est un groupement approprié et choisi dans chaque cas indépendamment des autres dans le groupe consistant en =O, -OR^{f}, C₁₋₃haloalkyloxy, -OCF₃, =S, -SR^{f}, =NR^{f}, =NOR^{f}, -NR^{f}R^{f}, halogène, -CF3, -CN,-NC, -OCN, -SCN, -NO, -NO₂, =N₂, -N₃, -S(O)R^{f}, -S(O)₂R^{f}, -S(O)₂OR^{f},-S(O)NR^{f}R^{f}, -S(O)₂NR^{f}R^{f}, -OS(O)R^{f}, -OS(O)₂R^{f}, -OS(O)₂OR^{f}, -OS(O)₂NR^{f}R^{f},-C(O)R^{f}, -C(O)OR^{f}, -C(O)NR^{f}R^{f}, -CN(R^{g})NR^{f}R^{f}, -OC(O)R^{f}, -OC(O)OR^{f},-OC(O)NR^{f}R^{f}, -OCN(R^{g})NR^{f}R^{f}, -N(R^{g})C(O)R^{f}, -N(R^{g})C(S)R^{f}, -N(R^{g})S(O)₂R^{f},-N(R^{g})C(O)OR^{f}, -N(R^{g})C(O)NR^{f}R^{f} et -N(R^{g})CN(R^{g})NR^{f}R^{f};
chaque R^{f} représente indépendamment des autres l'hydrogène ou un groupement éventuellement substitué avec un ou plusieurs R^{g} identiques ou différents choisi dans le groupe consistant en C₁₋₆alkyle, C₃₋₈cycloalkyle, C₄₋₁₁cycloalkylalkyle, C₆₋₁₀aryle, C₇₋₁₆arylalkyle, hétéroalkyle à 2-6 chaînons, hétérocycloalkyle à 3-8 chaînons, hétérocycloalkylalkyle à 4-14 chaînons, hétéroaryle à 5-10 chaînons et hétéroarylalkyle à 6-16 chaînons ;
chaque R^{g} est choisi indépendamment des autres parmi l'hydrogène, C₁₋₆alkyle, C₃₋₈cycloalkyle, C₄₋₁₁cycloalkylalkyle, C₆₋₁₀aryle, C₇₋₁₆arylalkyle, hétéroalkyle à 2-6 chaînons, hétérocycloalkyle à 3-8 chaînons, hétérocycloalkylalkyle à 4-14 chaînons, hétéroaryle à 5-10 chaînons et hétéroarylalkyle à 6-16 chaînons ;
éventuellement sous forme de leurs tautomères, de leurs racémates, de leurs énantiomères, de leurs diastéréoisomères et de leurs mélanges, et éventuellement de leurs sels d'addition d'acide pharmacologiquement acceptables.

2. Composés selon la revendication 1 où R³ représente un groupement choisi dans le groupe consistant en phényle, furyle, pyridyle, pyrimidyle et pyrazinyle, éventuellement substitué avec un ou plusieurs R⁴.

3. Composés selon la revendication 2 où R³ représente pyridyle.

4. Composés selon les revendications 1 à 3 où R¹ représente -NHC(O)R^{c}.

5. Composés selon la revendication 4 où R¹ représente -NHC(O)CH₃.

6. Composés de formule (A) 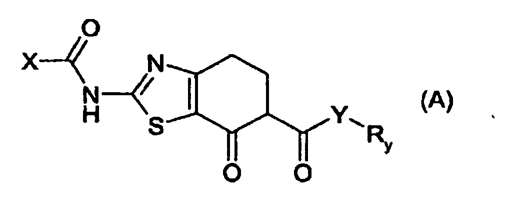 où
X représente -CH₃, -OR⁴ ou -SR⁴ et
Y représente phényle, hétéroaryle à 5-10 chaînons ou le groupe -C(O)O et
R^{y} représente l'hydrogène, -NO₂ ou C₁₋₆alkyle et R⁴ est défini comme précédemment.

7. Composés selon la revendication 6 où R⁴ représente C₁₋₆alkyle.

8. Composés de formule générale (A) selon la revendication 6 ou 7 destinés à être utilisés comme intermédiaires de synthèse.

9. Composés, ou leurs sels pharmaceutiquement actifs, selon les revendications 1 à 5 comme médicament.

10. Composés, ou leurs sels pharmaceutiquement actifs, selon les revendications 1 à 5 pour la fabrication d'un médicament à action antiproliférative.

11. Préparations pharmaceutiques contenant comme principe actif un ou plusieurs composés de formule générale (1) selon l'une des revendications 1 à 5 ou leurs sels physiologiquement acceptables éventuellement en combinaison avec des adjuvants et/ou vecteurs habituels.

12. Utilisation de composés de formule générale (1) selon l'une des revendications 1 à 5 pour la fabrication d'un médicament pour le traitement et/ou la prévention du cancer.

13. Préparation pharmaceutique comprenant un composé de formule générale (1) selon l'une des revendications 1 à 5 et au moins une autre substance active cytostatique ou cytotoxique, différente de la formule (1), éventuellement sous forme de leurs tautomères, de leurs racémates, de leurs énantiomères, de leurs diastéréoisomères et de leurs mélanges, et éventuellement de leurs sels d'addition d'acide pharmacologiquement acceptables.
